# EUROPEAN PATENT APPLICATION

(11) **EP 4 053 287 A1**
(43) Date of publication of application: **07.09.2022**
(21) Application number: 20881305.5
(22) Date of filing: 27.10.2020
(51) Int. Cl.: C12P 7/24, C12N 15/53

(54) **BENZALDEHYDE PRODUCTION METHOD**

(30) Priority: 28.10.2019 JP 2019195625
(71) Applicant: AJINOMOTO CO., INC., Chuo-ku Tokyo 104-8315 (JP)
(72) Inventor: TAKAKURA, Yasuaki, Kawasaki-shi, Kanagawa 210-8681 (JP); ONO, Takuto, Kawasaki-shi, Kanagawa 210-8681 (JP); IGONINA, Olga, Kawasaki-shi, Kanagawa 210-8681 (JP); KOENUMA, Keiko, Kawasaki-shi, Kanagawa 210-8681 (JP); NOZAKI, Hiroyuki, Kawasaki-shi, Kanagawa 210-8681 (JP)
(74) Representative: Hoffmann Eitle
(86) International application number: PCT/JP2020/040189
(87) International publication number: WO 2021/085405

(57) **Abstract**

A method for producing benzaldehyde is provided. Benzaldehyde is produced by a method for producing benzaldehyde, comprising a step of producing benzaldehyde by using amino acid deaminase (AAD), 4-hydroxymandelate synthase (HMAS), (S)-mandelate dehydrogenase (SMDH), and benzoylformate decarboxylase (BFDC), wherein at least a part of the step is carried out in the presence of catalase.

## Description

### Technical Field

The present invention relates to a method for producing benzaldehyde.

### Background Art

Benzaldehyde is an ingredient of the smell of almond and apricot kernel, and is used as an aromatic by being blended in foods, drinks, perfumes, and so forth. Benzaldehyde is mainly produced by chemical synthesis.

Furthermore, there are known some enzymes involved in biosynthesis of benzaldehyde. Examples of such enzymes include amino acid deaminase (AAD), 4-hydroxymandelate synthase (HMAS), (S)-mandelate dehydrogenase (SMDH), and Benzoylformate decarboxylase (BFDC) (Patent document 1). There has been reported a method for producing benzaldehyde from L-phenylalanine by using microorganism(s) having these enzymes (Patent document 1).

Furthermore, there has been reported a method for producing benzaldehyde from D-phenylalanine by using D-amino acid oxidase and peroxidase (Non-patent document 1). Non-patent document 1 discloses that the activity of D-amino acid oxidase is inhibited by hydrogen peroxide generated by catalytic reaction of this enzyme and hydrogen peroxide is removed by using catalase.

### Prior art references

### Patent documents

Patent document 1: WO2017/122747

### Non-patent documents

Non-patent document 1: Krzysztof Okrasaa et al. In vitro bi-enzymatic synthesis of benzaldehyde from phenylalanine: practical and mechanistic studies. Journal of Molecular Catalysis B: Enzymatic 31 (2004) 97-101.

### Summary of Invention

### Object to be Achieved by the Invention

An object of the present invention is to provide a method for efficiently producing benzaldehyde.

### [Means for Achieving the Object]

The inventors of the present invention conducted various researches in order to achieve the aforementioned object. As a result, the inventors found that, upon production of benzaldehyde using amino acid deaminase, 4-hydroxymandelate synthase, (S)-mandelate dehydrogenase, and benzoylformate decarboxylase, benzaldehyde can be efficiently produced by carrying out at least a part of the production of benzaldehyde in the presence of catalase and accomplished the present invention.

The present invention can be thus embodied as follows.
[1] A method for producing benzaldehyde, the method comprising the step (A) mentioned below:
   (A) a step of producing benzaldehyde by using four enzymes: amino acid deaminase (AAD), 4-hydroxymandelate synthase (HMAS), (S)-mandelate dehydrogenase (SMDH), and benzoylformate decarboxylase (BFDC),
      wherein at least a part of the step (A) is carried out in the presence of catalase.
[2] The method mentioned above, wherein the part is a part using HMAS.
[3] The method mentioned above,
   wherein the four enzymes are used in the form of at least one microorganism having these enzymes, and
   wherein the at least one microorganism is a single microorganism alone having the four enzymes or a combination of a plurality of microorganisms having the four enzymes as a whole.
[4] The method mentioned above, wherein the AAD is AAD not generating hydrogen peroxide.
[5] The method mentioned above, wherein benzaldehyde is produced from L-phenylalanine or a carbon source.
[6] The method mentioned above, wherein the step (A) comprises the steps (A1) and (A2) mentioned below:
   (A1) a step of producing benzoylformate by using the AAD, HMAS, and SMDH;
   (A2) a step of converting benzoylformate generated in the step (A1) into benzaldehyde by using the BFDC.
[7] The method mentioned above, wherein the step (A1) comprise the step (1a), (1b), or (1c) mentioned below:
   (1a) a step of cultivating at least one microorganism in a culture medium containing the carbon source to generate and accumulate benzoylformate in the culture medium, wherein the at least one microorganism is a single microorganism alone having the AAD, HMAS, and SMDH as well as L-phenylalanine-producing ability or a combination of a plurality of microorganisms as a whole having the AAD, HMAS, and SMDH as well as L-phenylalanine-producing ability;
   (1b) a step of cultivating at least one microorganism in a culture medium containing L-phenylalanine to generate and accumulate benzoylformate in the culture medium, wherein the at least one microorganism is a single microorganism alone having the AAD, HMAS, and SMDH or a combination of a plurality of microorganisms as a whole having the AAD, HMAS, and SMDH
   (1c) a step of allowing the AAD, HMAS, and SMDH to coexist with L-phenylalanine in a reaction mixture to generate and accumulate benzoylformate in the reaction mixture.
[8] The method mentioned above,
   wherein the AAD, HMAS, and SMDH in the step (1c) are used in the form of cells of at least one microorganism having the AAD, HMAS, and SMDH, and
   wherein the at least one microorganism is a single microorganism alone having the AAD, HMAS, and SMDH or a combination of a plurality of microorganisms having the AAD, HMAS, and SMDH as a whole.
[9] The method mentioned above, wherein the step (A2) comprises the step (2a) or (2b) mentioned below:
   (2a) a step of cultivating a microorganism having the BFDC in a culture medium containing benzoylformate generated in the step (A1) to generate and accumulate benzaldehyde in the culture medium;
   (2b) a step of allowing the BFDC to coexist with benzoylformate generated in the step (A1) in a reaction mixture to generate and accumulate benzaldehyde in the reaction mixture.
[10] The method mentioned above, wherein the BFDC in the step (2b) is used in the form of cells of a microorganism having the BFDC.
[11] The method mentioned above, wherein the cells are used in the form of a culture broth of the at least one microorganism, cells collected from the culture broth, a processed product thereof, or a combination thereof.
[12] The method mentioned above, wherein the catalase is a protein defined in (a), (b), or (c) mentioned below:
   (a) a protein comprising the amino acid sequence of SEQ ID NO: 4, 6, 8, 10, 12, 14, 16, 18, 20, 22, 24, 26, 28, 30, or 32;
   (b) a protein comprising the amino acid sequence of SEQ ID NO: 4, 6, 8, 10, 12, 14, 16, 18, 20, 22, 24, 26, 28, 30, or 32 but including substitution, deletion, insertion, and/or addition of 1 to 10 amino acid residues, and having catalase activity;
   (c) a protein comprising an amino acid sequence showing an identity of 90% or higher to the amino acid sequence of SEQ ID NO: 4, 6, 8, 10, 12, 14, 16, 18, 20, 22, 24, 26, 28, 30, or 32, and having catalase activity.
[13] The method mentioned above, wherein the microorganism(s) each is/are a bacterium or yeast.
[14] The method mentioned above, wherein the microorganism(s) each is/are a bacterium belonging to the family *Enterobacteriaceae* or a coryneform bacterium.
[15] The method mentioned above, wherein the microorganism(s) each is/are a bacterium belonging to the genus *Escherichia.*
[16] The method mentioned above, wherein the microorganism(s) each is/are *Escherichia coli.*
[17] The method mentioned above, the method further comprising collecting benzaldehyde.

### Modes for Carrying out the Invention

Hereinafter, the present invention will be explained in detail.

The method of the present invention is a method for producing benzaldehyde comprising a step of producing benzaldehyde by using amino acid deaminase (AAD), 4-hydroxymandelate synthase (HMAS), (S)-mandelate dehydrogenase (SMDH), and benzoylformate decarboxylase (BFDC), wherein at least a part of the step is carried out in the presence of catalase. These four enzymes, i.e. AAD, HMAS, SMDH, and BFDC, are also collectively referred to as "benzaldehyde generation enzymes". The benzaldehyde generation enzymes and catalase are also collectively referred to as "objective enzymes". Genes encoding the objective enzymes are also collectively referred to as "objective genes".

### <1> Benzaldehyde generation enzymes (AAD, HMAS, SMDH, and BFDC)

Amino acid deaminase (AAD) is known as an enzyme that catalyzes the reaction of oxidatively deaminating an amino acid (e.g. EC 1.4.3.2 or EC 1.4.99.B3). AAD used in the present invention uses at least L-phenylalanine as a substrate. AAD used in the present invention may or may not use any other amino acid as a substrate, so long as it uses L-phenylalanine as a substrate. That is, in the present invention, the term "AAD" refers to a protein that has the activity of catalyzing the reaction of oxidatively deaminating L-phenylalanine to generate phenylpyruvate. This activity is also referred to as "AAD activity". The term "AAD activity" may specifically refer to the activity of catalyzing the reaction of deaminating L-phenylalanine to generate phenylpyruvate in the presence of an electron acceptor. Examples of the electron acceptor include oxygen and cytochrome b. When oxygen is used as the electron acceptor, hydrogen peroxide may be generated by oxidative deamination. When cytochrome b is used as the electron acceptor, a reduced form of cytochrome b may be generated by oxidative deamination. That is, in an embodiment, the term "AAD activity" may refer to, for example, the activity of catalyzing the reaction of generating phenylpyruvate, ammonia, and hydrogen peroxide from L-phenylalanine, water, and oxygen. Also, in another embodiment, the term "AAD activity" may refer to, for example, the activity of catalyzing the reaction of generating phenylpyruvate, ammonia, and a reduced form of cytochrome b from L-phenylalanine, water, and cytochrome b. A gene encoding AAD is also referred to as "AAD gene". AAD is also referred to as "amino acid oxidase" or "L-phenylalanine oxidase". Examples of AAD include AADs of various organisms such as microorganisms. Specific examples of AAD include AADs of *Providencia* bacteria such as *Providencia rettgeri* (WO2009/028338), AADs of *Proteus* bacteria such as *Proteus mirabilis* (Massad G et al., Proteus mirabilis amino acid deaminase: cloning, nucleotide sequence, and characterization of aad. J Bacteriol. 1995 Oct;177(20):5878-83.), and AADs of other various organisms. Examples of *Providencia rettgeri* include the *Providencia rettgeri* AJ2770 strain (FERM BP-941) and IFO13501 strain. The nucleotide sequence of the AAD gene of the *Providencia rettgeri* AJ2770 strain (FERM BP-941) (the modified nucleotide sequence used in Examples section) is shown as SEQ ID NO: 37, and the amino acid sequence of AAD encoded by this gene is shown as SEQ ID NO: 38. As AAD, in particular, AAD not generating hydrogen peroxide may be used. For example, AAD of *Providencia rettgeri* AJ2770 strain (FERM BP-941) can be AAD not generating hydrogen peroxide. As AAD, one kind of AAD may be used, or two or more kinds of AADs may be used.

The AAD activity can be measured by, for example, incubating the enzyme with the substrate (i.e. L-phenylalanine) in the presence of oxygen, and measuring the enzyme- and substrate-dependent generation of the product, i.e. phenylpyruvate (Massad G et al., Proteus mirabilis amino acid deaminase: cloning, nucleotide sequence, and characterization of aad. J Bacteriol. 1995 Oct;177(20):5878-83.). The generation of phenylpyruvate can be measured by, for example, measuring coloration due to complex formation between phenylpyruvate and ferric ion as an increase in absorbance at 614 nm (Ibid.).

4-hydroxymandelate synthase (HMAS) is known as an enzyme that catalyzes the reaction of oxidatively decarboxylating an alpha-keto acid such as 4-hydroxyphenylpyruvate (e.g. EC 1.13.11.46). HMAS used in the present invention uses at least phenylpyruvate as a substrate. HMAS used in the present invention may or may not use any other alpha-keto acid such as 4-hydroxyphenylpyruvate as a substrate, so long as it uses phenylpyruvate as a substrate. That is, in the present invention, the term "HMAS" refers to a protein that has the activity of catalyzing the reaction of oxidatively decarboxylating phenylpyruvate to generate (S)-mandelate. This activity is also referred to as "HMAS activity". The term "HMAS activity" may specifically refer to the activity of catalyzing the reaction of decarboxylating phenylpyruvate to generate (S)-mandelate in the presence of an electron acceptor. Examples of the electron acceptor include oxygen. That is, the term "HMAS activity" may refer to, for example, the activity of catalyzing the reaction of generating (S)-mandelate and CO₂ from phenylpyruvate and oxygen. A gene encoding HMAS is also referred to as "HMAS gene". Examples of HMAS include HMASs of various organisms such as microorganisms. Specific examples of HMAS include HMASs of *Amycolatopsis* bacteria such as *Amycolatopsis orientalis* and *Amycolatopsis balhimycina,* HMASs of *Streptomyces* bacteria such as *Streptomyces coelicolor, Streptomyces toyocaensis,* and *Streptomyces rimosus,* HMASs of *Rhodococcus* bacteria such as *Rhodococcus rhodnii,* HMASs of *Actinoplanes* bacteria such as *Actinoplanes teichomyceticus, Actinoplanes rectilineatus, and Actinoplanes subtropicus,* HMASs of *Kibdelosporangium* bacteria such as *Kibdelosporangium aridum*, HMASs of *Nonomuraea* bacteria such as *Nonomuraea coxensis,* HMASs of *Herpetosiphon* bacteria such as *Herpetosiphon aurantiacus*, and HMASs of other various organisms. The nucleotide sequence of the HMAS gene of *Actinoplanes teichomyceticus* (codon-optimized for expression in *E. coli)* is shown as SEQ ID NO: 39, and the amino acid sequence of HMAS encoded by this gene is shown as SEQ ID NO: 40. As HMAS, one kind of HMAS may be used, or two or more kinds of HMASs may be used. In an embodiment, HMASs of Kibdelosporangium aridum and Actinoplanes rectilineatus may be excluded from HMAS. In another embodiment, HMASs of Amycolatopsis orientalis, Streptomyces coelicolor, Kibdelosporangium aridum, and Actinoplanes rectilineatus may be excluded from HMAS.

The HMAS activity can be measured by, for example, incubating the enzyme with the substrate (i.e. phenylpyruvate) in the presence of oxygen, and measuring the enzyme- and substrate-dependent generation of the product, i.e. (S)-mandelate (Sun Z et al., Metabolic engineering of the L-phenylalanine pathway in Escherichia coli for the production of S- or R-mandelic acid. Microb Cell Fact. 2011 Sep 13;10:71.).

The term "(S)-mandelate dehydrogenase (SMDH)" refers to a protein that has the activity of catalyzing the reaction of oxidizing (S)-mandelate to generate benzoylformate (e.g. EC 1.1.99.31). This activity is also referred to as "SMDH activity". The term "SMDH activity" may specifically refer to the activity of catalyzing the reaction of oxidizing (S)-mandelate to generate benzoylformate in the presence of an electron acceptor. Examples of the electron acceptor include NAD. Examples of the electron acceptor also include artificial electron acceptors such as phenazinemethosulfate (PMS) and dichloroindophenol (DCIP). A gene encoding SMDH is also referred to as "SMDH gene". Examples of SMDH include SMDHs of various organisms such as microorganisms. Specific examples of SMDH include MdlB proteins encoded by mdlB genes of *Pseudomonas* bacteria such as *Pseudomonas putida,* and SMDHs of other various organisms. The nucleotide sequence of the mdlB gene (SMDH gene) of *Pseudomonas putida* is shown as SEQ ID NO: 41, and the amino acid sequence of MdlB protein (SMDH) encoded by this gene is shown as SEQ ID NO: 42. As SMDH, one kind of SMDH may be used, or two or more kinds of SMDHs may be used.

The SMDH activity can be measured by, for example, incubating the enzyme with the substrate (i.e. (S)-mandelate) in the presence of NAD, and measuring the enzyme- and substrate-dependent reduction of NAD (B.S. Al-Baharna and R.Y Hamzah, Aerobic metabolism of mandelates by Burkholderia cepacia ATTC 29351. Arab J. Biotech., Vol. 6, No.(1) Jan. (2003): 13-28.). The SMDH activity can also be measured by, for example, incubating the enzyme with the substrate (i.e. (S)-mandelate) in the presence of phenazine methosulfate (PMS) and dichloroindophenol (DCIP), and measuring the enzyme- and substrate-dependent reduction of DCIP (Ibid.). The SMDH activity can also be measured by, for example, incubating the enzyme with the substrate (i.e. (S)-mandelate) in sodium phosphate-citrate buffer in the presence of potassium ferricyanide, and measuring the enzyme- and substrate-dependent reduction of potassium ferricyanide (Peng Wang et al., Immobilization of (S)-mandelate dehydrogenase and its catalytic performance on stereoselective transformation of mandelic acid. Journal of the Taiwan Institute of Chemical Engineers Volume 45, Issue 3, May 2014, Pages 744-748.).

The term "benzoylformate decarboxylase (BFDC)" refers to a protein that has the activity of catalyzing the reaction of decarboxylating benzoylformate to generate benzaldehyde (e.g. EC 4.1.1.7). This activity is also referred to as "BFDC activity". A gene encoding BFDC is also referred to as "BFDC gene". Examples of BFDC include BFDCs of various organisms such as microorganisms. Specific examples of BFDC include MdlC proteins encoded by mdlC genes of *Pseudomonas* bacteria such as *Pseudomonas putida*, and BFDCs of other various organisms. The nucleotide sequence of the mdlC gene (BFDC gene) of *Pseudomonas putida* is shown as SEQ ID NO: 43, and the amino acid sequence of MdlC protein (BFDC) encoded by this gene is shown as SEQ ID NO: 44. As BFDC, one kind of BFDC may be used, or two or more kinds of BFDCs may be used.

The BFDC activity can be measured by, for example, incubating the enzyme with the substrate (i.e. benzoylformate), and measuring the enzyme- and substrate-dependent generation of the product, i.e. benzaldehyde (Park, J.K. and Jung, J.Y, Production of benzaldehyde by encapsulated whole-cell benzoylformate decarboxylase, Enzyme Microb Technol, 30, 726-733, 2002.).

That is, the benzaldehyde generation enzyme genes each may be, for example, a gene having any of known nucleotide sequences such as the nucleotide sequences exemplified above. Also, the benzaldehyde generation enzymes each may be, for example, a protein having any of known amino acid sequences such as the amino acid sequences exemplified above. The expression "a gene or protein has a nucleotide or amino acid sequence" means that a gene or protein comprises the nucleotide or amino acid sequence unless otherwise stated, and also include cases where a gene or protein consists of the nucleotide or amino acid sequence.

The benzaldehyde generation enzyme genes each may be a variant of any of the benzaldehyde generation enzyme genes exemplified above, so long as the original function thereof is maintained. Similarly, the benzaldehyde generation enzymes each may be a variant of any of the benzaldehyde generation enzymes exemplified above, so long as the original function thereof is maintained. A variant that maintains the original function thereof is also referred to as "conservative variant". Examples of the conservative variants include, for example, homologues and artificially modified versions of the benzaldehyde generation enzyme genes and benzaldehyde generation enzymes exemplified above.

The expression "the original function is maintained" means that a variant of a gene or protein has a function (such as activity or property) corresponding to the function (such as activity or property) of the original gene or protein. The expression "the original function is maintained" used for a gene means that a variant of the gene encodes a protein that maintains the original function. That is, the expression "the original function is maintained" used for the AAD gene, HMAS gene, SMDH gene, and BFDC gene means that the variant of the genes encodes a protein having AAD activity, HMAS activity, SMDH activity, and BFDC activity, respectively. The expression "the original function is maintained" used for AAD, HMAS, SMDH, and BFDC means that the variant of the proteins has AAD activity, HMAS activity, SMDH activity, and BFDC activity, respectively.

Hereinafter, examples of the conservative variants will be explained.

Homologues of the benzaldehyde generation enzyme genes or homologues of benzaldehyde generation enzymes can be easily obtained from public databases by, for example, BLAST search or FASTA search using any of the nucleotide sequences of the benzaldehyde generation enzyme genes exemplified above or any of the amino acid sequences of benzaldehyde generation enzymes exemplified above as a query sequence. Furthermore, homologues of the benzaldehyde generation enzyme genes can be obtained by, for example, PCR using a chromosome of an organism such as bacteria and yeast as the template, and oligonucleotides prepared on the basis of any of the nucleotide sequences of the benzaldehyde generation enzyme genes exemplified above as primers.

The benzaldehyde generation enzyme genes each may be a gene encoding a protein having any of the aforementioned amino acid sequences (e.g. the amino acid sequence shown as SEQ ID NO: 38 for AAD; the amino acid sequence shown as SEQ ID NO: 40 for HMAS; the amino acid sequence shown as SEQ ID NO: 42 for SMDH; and the amino acid sequence shown as SEQ ID NO: 44 for BFDC) including substitution, deletion, insertion, and/or addition of one or several amino acid residues at one or several positions, so long as the original function is maintained. For example, the encoded protein may have an extended or deleted N-terminus and/or C-terminus. Although the number meant by the term "one or several" used above may differ depending on the positions of amino acid residues in the three-dimensional structure of the protein or the types of amino acid residues, specifically, it is, for example, 1 to 50, 1 to 40, or 1 to 30, preferably 1 to 20, more preferably 1 to 10, still more preferably 1 to 5, particularly preferably 1 to 3.

The aforementioned substitution, deletion, insertion, or addition of one or several amino acid residues each are a conservative mutation that maintains the original function of the protein. Typical examples of the conservative mutation are conservative substitutions. The conservative substitution is a mutation wherein substitution takes place mutually among Phe, Trp, and Tyr, if the substitution site is an aromatic amino acid; among Leu, Ile, and Val, if it is a hydrophobic amino acid; between Gln and Asn, if it is a polar amino acid; among Lys, Arg, and His, if it is a basic amino acid; between Asp and Glu, if it is an acidic amino acid; and between Ser and Thr, if it is an amino acid having a hydroxyl group. Examples of substitutions considered as conservative substitutions include, specifically, substitution of Ser or Thr for Ala, substitution of Gln, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of Gly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr, and substitution of Met, Ile, or Leu for Val. Furthermore, such substitution, deletion, insertion, or addition of amino acid residues as mentioned above includes a naturally occurring mutation due to an individual difference, or a difference of species of the organism from which the gene is derived (mutant or variant).

Furthermore, the benzaldehyde generation enzyme genes each may be a gene encoding a protein having an amino acid sequence showing an identity of, for example, 50% or more, 65% or more, or 80% or more, preferably 90% or more, more preferably 95% or more, still more preferably 97% or more, particularly preferably 99% or more, to the total amino acid sequence of any of the aforementioned amino acid sequences, so long as the original function is maintained.

Furthermore, the benzaldehyde generation enzyme genes each may be a gene, such as a DNA, that is able to hybridize under stringent conditions with a probe that can be prepared from any of the aforementioned nucleotide sequences (e.g. the nucleotide sequence shown as SEQ ID NO: 37 for AAD gene; the nucleotide sequence shown as SEQ ID NO: 39 for HMAS gene; the nucleotide sequence shown as SEQ ID NO: 41 for SMDH gene; and the nucleotide sequence shown as SEQ ID NO: 43 for BFDC gene), such as a sequence complementary to the whole sequence or a partial sequence of any of the aforementioned nucleotide sequences, so long as the original function is maintained. The "stringent conditions" refer to conditions under which a so-called specific hybrid is formed, and a non-specific hybrid is not formed. Examples of the stringent conditions include those under which highly identical DNAs hybridize to each other, for example, DNAs not less than 50%, 65%, or 80% identical, preferably not less than 90% identical, more preferably not less than 95% identical, still more preferably not less than 97% identical, particularly preferably not less than 99% identical, hybridize to each other, and DNAs less identical than the above do not hybridize to each other, or conditions of washing of typical Southern hybridization, i.e., conditions of washing once, preferably 2 or 3 times, at a salt concentration and temperature corresponding to 1 x SSC, 0.1% SDS at 60°C, preferably 0.1 x SSC, 0.1% SDS at 60°C, more preferably 0.1 x SSC, 0.1% SDS at 68°C.

The probe used for the aforementioned hybridization may be a part of a sequence that is complementary to the gene as described above. Such a probe can be prepared by PCR using oligonucleotides prepared on the basis of a known gene sequence as primers and a DNA fragment containing any of the aforementioned genes as a template. As the probe, for example, a DNA fragment having a length of about 300 bp can be used. When a DNA fragment having a length of about 300 bp is used as the probe, in particular, the washing conditions of the hybridization may be, for example, 50°C, 2 x SSC and 0.1% SDS.

Furthermore, since properties concerning degeneracy of codons changes depending on the host, the benzaldehyde generation enzyme genes each may include substitution of respective equivalent codons for any codons. That is, the benzaldehyde generation enzyme genes each may be a variant of any of the benzaldehyde generation enzyme genes exemplified above due to the degeneracy of the genetic code. For example, the benzaldehyde generation enzyme genes each may be a gene modified so that it has optimal codons according to codon frequencies in a host to be used.

The term "identity" between amino acid sequences means an identity between the amino acid sequences calculated by blastp with default scoring parameters (i.e. Matrix, BLOSUM62; Gap Costs, Existence = 11, Extension = 1; Compositional Adjustments, Conditional compositional score matrix adjustment). The term "identity" between nucleotide sequences means an identity between the nucleotide sequences calculated by blastn with default scoring parameters (i.e. Match/Mismatch Scores = 1, - 2; Gap Costs = Linear).

Examples of HMAS further include HMASs having a "specific mutation" (WO2017/122747). Also, examples of the HMAS gene further include HMAS genes encoding HMASs having the "specific mutation". HMAS having the "specific mutation" is also referred to as "mutant HMAS". A gene encoding a mutant HMAS is also referred to as "mutant HMAS gene".

HMAS not having the "specific mutation" is also referred to as "wild-type HMAS". A gene encoding a wild-type HMAS is also referred to as "wild-type HMAS gene". The term "wild-type" referred to herein is used for convenience to distinguish the "wild-type" HMAS from the "mutant" HMAS, and the "wild-type" HMAS is not limited to those obtained as natural substances, and includes any HMAS not having the "specific mutation". Examples of the wild-type HMAS include, for example, HMASs exemplified above. In addition, all conservative variants of HMASs exemplified above should be included in wild-type HMASs, provided that such conservative variants do not have the "specific mutation".

A mutant HMAS may be identical to a wild-type HMAS, such as HMASs exemplified above and conservative variants thereof, provided that the mutant HMAS has the "specific mutation". That is, a mutant HMAS may be a protein having any of the amino acid sequences of wild-type HMASs, but having the "specific mutation". Specifically, a mutant HMAS may be, for example, a protein having the amino acid sequence shown as SEQ ID NO: 40, provided that the mutant HMAS has the "specific mutation". Also, specifically, a mutant HMAS may be, for example, a protein having the amino acid sequence shown as SEQ ID NO: 40, but including substitution, deletion, insertion, and/or addition of one or several amino acid residues, provided that the mutant HMAS has the "specific mutation". Also, specifically, a mutant HMAS may be, for example, a protein having an amino acid sequence showing an identity of 50% or more, 65% or more, or 80% or more, 90% or more, 95% or more, 97% or more, or 99% or more to the amino acid sequence of SEQ ID NO: 40, provided that the mutant HMAS has the "specific mutation".

In a conservative variant to be used as a wild-type HMAS, conservative mutation(s) may occur at position(s) other than the position(s) of the "specific mutation". That is, in other words, a mutant HMAS may be a protein having any of the amino acid sequences of HMASs exemplified above, but having the "specific mutation" and further including conservative mutation(s), such as substitution, deletion, insertion, and/or addition of one or several amino acid residues, at position(s) other than the position(s) of the "specific mutation".

The "specific mutation" may be a mutation effective for production of benzaldehyde, such as a mutation resulting in an increased production of benzaldehyde in the method of the present invention. Such an increased production of benzaldehyde may be due to, for example, an increased generation of (S)-mandelate by HMAS. Hence, the "specific mutation" may also be a mutation resulting in an increased generation of (S)-mandelate by HMAS.

Examples of the "specific mutation" include mutations at amino acid residues corresponding to T2, M3, G5, Y18, A27, D35, E46, E180, A187, E191, V194, A199, D201, Q206, 1217, D220, T222, G255, F319, G327, 1336, K337, V343, and Q347. The "specific mutation" may consist of a mutation at one amino acid residue, or may consist of a combination of mutations at two or more amino acid residues. That is, the "specific mutation" may comprise or consist of, for example, mutation(s) at amino acid residue(s) corresponding to one or more amino acid residues selected from the group consisting of T2, M3, G5, Y18, A27, D35, E46, E180, A187, E191, V194, A199, D201, Q206, 1217, D220, T222, G255, F319, G327, 1336, K337, V343, and Q347.

In the aforementioned notation used for defining amino acid residues, the numbers represent the positions in the amino acid sequence shown as SEQ ID NO: 40, and the letters at the left side of the numbers represent the amino acid residues at the respective positions in the amino acid sequence shown as SEQ ID NO: 40 (i.e. the amino acid residues before modification at the respective positions). That is, for example, "T2" represents T (Thr) residue at position 2 in the amino acid sequence shown as SEQ ID NO: 40.

In each of the aforementioned mutations, the amino acid residue after modification may be any amino acid residue other than the amino acid residue before modification. Examples of the amino acid residue after modification include K (Lys), R (Arg), H (His), A (Ala), V (Val), L (Leu), I (Ile), G (Gly), S (Ser), T (Thr), P (Pro), F (Phe), W (Trp), Y (Tyr), C (Cys), M (Met), D (Asp), E (Glu), N (Asn), and Q (Gln), provided that the amino acid residues after modification is other than the amino acid residue before modification. As the amino acid residues after modification, there may be selected those effective for production of benzaldehyde.

Specific examples of the "specific mutation" include mutations corresponding to T2N, M3I, G5R, Y18F, A27V, D35G, E46Q, E180K, A187V, E191K, V194G, A199(S, V), D201N, Q206R, I217(L, V), D220(A, N), T222S, G255D, F319Y, G327(D, S), I336V, K337Q, V343M, and Q347L. That is, the mutations at amino acid residues corresponding to T2, M3, G5, Y18, A27, D35, E46, E180, A187, E191, V194, A199, D201, Q206, 1217, D220, T222, G255, F319, G327, 1336, K337, V343, and Q347 may be, for example, the mutations corresponding to T2N, M3I, G5R, Y18F, A27V, D35G, E46Q, E180K, A187V, E191K, V194G, A199(S, V), D201N, Q206R, I217(L, V), D220(A, N), T222S, G255D, F319Y, G327(D, S), I336V, K337Q, V343M, and Q347L, respectively. The "specific mutation" may comprise or consist of, for example, mutation(s) corresponding to one or more kinds of mutations selected from the group consisting of T2N, M3I, G5R, Y18F, A27V, D35G, E46Q, E180K, A187V, E191K, V194G, A199(S, V), D201N, Q206R, I217(L, V), D220(A, N), T222S, G255D, F319Y, G327(D, S), 1336V, K337Q, V343M, and Q347L.

In the aforementioned notation used for defining mutations, the numbers and the letters at the left side of the numbers represent the same as described above. In the aforementioned notation used for defining mutations, the letters at the right side of the numbers represent the amino acid residues after modification at the respective positions. That is, for example, "T2N" represents a mutation for replacing T (Thr) residue at position 2 in the amino acid sequence shown as SEQ ID NO: 40 with N (Asn) residue. Also, for example, A199(S, V) represents a mutation for replacing A (Ala) residue at position 199 in the amino acid sequence shown as SEQ ID NO: 40 with S (Ser) or V (Val) residue.

Combination of mutations is not particularly limited. Specific examples of combination of mutations include M3I/A199S/G255D, Y18F/D220N, A27V/E191K, D35G/E46Q/T222S/I336V, E180K/I217V/D220N, A187V/I217V, A199V/I217V/K337Q, D201N/I217V, I217V/F319Y, D220A/Q347L, and A199V/Q206R/I217V/K337Q. Particular examples of combination of mutations include A199V/Q206R/I217V/K337Q. That is, the "specific mutation" may comprise or consist of, for example, mutations corresponding to any of these combinations.

In the aforementioned notation used for defining combinations, the numbers and the letters at the left and right sides of the numbers represent the same as described above. In the aforementioned notation used for defining combinations, two or more mutations noted together and inserted with "/" represent a double or more multiple mutation. That is, for example, "M3I/A199S/G255D" represents a triple mutation of M3I, A199S, and G255D.

A "mutation corresponding to a mutation at the amino acid residue at position X in the amino acid sequence shown in SEQ ID NO: 40" should be read as a mutation at an amino acid residue corresponding to the amino acid residue at position X in the amino acid sequence shown in SEQ ID NO: 40". That is, for example, a "mutation corresponding to T2N" represents a mutation for replacing an amino acid residue corresponding to T2 with N (Asn) residue.

The "position X" in an amino acid sequence refers to the X-th position counted from the N-terminus of the amino acid sequence, and the amino acid residue of the N-terminus is the amino acid residue at position 1. The positions defined in the aforementioned mutations represent the relative positions, and the absolute positions thereof may shift due to deletion, insertion, addition, and so forth of amino acid residue(s). For example, if one amino acid residue is deleted or inserted at a position on the N-terminus side of position X in the amino acid sequence shown as SEQ ID NO: 40, the amino acid residue originally at position X is relocated at position X-1 or X+l, however, it is still regarded as the "amino acid residue corresponding to the amino acid residue at position X of the amino acid sequence shown as SEQ ID NO: 40".

The amino acid residues before modifications defined in the above-exemplified mutations are typical ones, but may not necessarily be limited thereto. For example, the "amino acid residue corresponding to T2" may typically be T (Thr) residue, however, it may not necessarily be T (Thr) residue. That is, when a wild-type HMAS has an amino acid sequence other than that shown in SEQ ID NO: 40, the "amino acid residue corresponding to T2" may be an amino acid residue other than T (Thr) residue. Therefore, the "mutation corresponding to T2N" includes not only a mutation, when the "amino acid residue corresponding to T2" is T (Thr) residue, for replacing this T (Thr) residue with N (Asn) residue, but also includes a mutation, when the "amino acid residue corresponding to T2" is K (Lys), R (Arg), H (His), A (Ala), V (Val), L (Leu), I (Ile), G (Gly), S (Ser), P (Pro), F (Phe), W (Trp), C (Cys), M (Met), D (Asp), E (Glu), or Q (Gln) residue, for replacing this residue with N (Asn) residue. The same can be similarly applied to the other mutations.

In the amino acid sequence of a certain HMAS, which amino acid residue is an "amino acid residue corresponding to the amino acid residue at position X in the amino acid sequence shown in SEQ ID NO: 40" can be determined by aligning the amino acid sequence of the certain HMAS and the amino acid sequence of SEQ ID NO: 40. The alignment can be performed by, for example, using known gene analysis software. Specific examples of such software include DNASIS produced by Hitachi Solutions, GENETYX produced by Genetyx, and so forth (Elizabeth C. Tyler et al., Computers and Biomedical Research, 24 (1) 72-96, 1991; Barton GJ et al., Journal of Molecular Biology, 198 (2), 327-37, 1987).

The aforementioned descriptions concerning the conservative variants of benzaldehyde generation enzyme genes and benzaldehyde generation enzymes can be similarly applied to any genes and proteins such as catalase gene and catalase.

### <2> Catalase

The term "catalase" refers to a protein that has the activity of catalyzing the reaction of degrading hydrogen peroxide to generate water and oxygen (e.g. EC 1.11.1.6). This activity is also referred to as "catalase activity". A gene encoding catalase is also referred to as "catalase gene". Examples of catalase include catalases of various organisms such as microorganisms. Specific examples of catalase include catalases of *Escherichia* bacteria such as *E. coli,* catalases of *Erwinia* bacteria such as *Erwinia mallotivora* and *Erwinia tracheiphila*, catalases of *Pseudomonas* bacteria such as *Pseudomonas putida*, Pseudomonas entomophi*la*, *Pseudomonas parafulva*, and *Pseudomonas protegens*, catalases of *Shewanella* bacteria such as *Shewanella oneidensis,* catalases of *Bacillus* bacteria such as *Bacillus subtilis,* catalases of *Thermus* bacteria such as *Thermus thermophilus,* catalases of *Rhodothermus* bacteria such as *Rhodothermus marinus,* catalases of *Corynebacterium* bacteria such as *C. glutamicum,* catalases of *Micrococcus* bacteria such as *Micrococcus lysodeikticus.* Specific examples of catalases of *E. coli* include HPI and HPII. The nucleotide sequence of DNA fragments comprising catalase genes of these organisms are shown as SEQ ID NOS: 3, 5, 7, 9, 11, 13, 15, 17, 19, 21, 23, 25, 27, 29, and 31, and the amino acid sequences of catalases encoded by these genes are shown as SEQ ID NOS: 4, 6, 8, 10, 12, 14, 16, 18, 20, 22, 24, 26, 28, 30, and 32. As catalase, one kind of catalase may be used, or two or more kinds of catalases may be used. In an embodiment, catalases comprising the amino acid sequence shown as SEQ ID NO: 16 (e.g. catalase consisting of the amino acid sequence shown as SEQ ID NO: 16) may be excluded from catalase.

The catalase gene and catalase may be, for example, a gene having any of known nucleotide sequences such as the nucleotide sequences exemplified above and a protein having any of known amino acid sequences such as the amino acid sequences exemplified above, respectively. The catalase gene and catalase may also be, for example, a conservative variant of any of the genes exemplified above (e.g. a conservative variant of a gene having any of known nucleotide sequences such as the nucleotide sequences exemplified above) and a conservative variant of any of the proteins exemplified above (e.g. a conservative variant of a protein having any of known amino acid sequences such as the amino acid sequences exemplified above), respectively. Specifically, for example, the catalase gene may be a gene encoding a protein having any of known amino acid sequences such as the amino acid sequences exemplified above including substitution, deletion, insertion, and/or addition of one or several amino acid residues at one or several positions, so long as the original function is maintained. Also, for example, the catalase gene may be a gene encoding a protein having an amino acid sequence showing an identity of, for example, 50% or more, 65% or more, 80% or more, 90% or more, 95% or more, 97% or more, or 99% or more, to the total amino acid sequence of any of known amino acid sequences such as the amino acid sequences exemplified above, so long as the original function is maintained. The expression "the original function is maintained" used for the catalase gene means that a variant of the gene encodes a protein having catalase activity. The expression "the original function is maintained" used for catalase means that the variant of the proteins has catalase activity. As for such conservative variants of genes and proteins, the aforementioned descriptions concerning the conservative variants of benzaldehyde generation enzyme genes and benzaldehyde generation enzymes can be similarly applied.

The catalase activity can be measured by, for example, incubating the enzyme with hydrogen peroxide, and measuring an enzyme-dependent decrease in hydrogen peroxide. The decrease in hydrogen peroxide can be measured, for example, as a decrease in absorbance at 240 nm. Furthermore, hydrogen peroxide can be quantified by, for example, using peroxidase such as Horseradish peroxidase (HRP) in combination with s coloring substrate.

### <3> Production of benzaldehyde generation enzymes and catalase

As each of the objective enzymes (i.e. benzaldehyde generation enzymes and catalase), a commercially available product may be used, or one appropriately produced and obtained may be used. For example, examples of the commercially available product of catalase include commercially available catalase preparations. Examples of the commercially available catalase preparations include those used in the Examples section.

Each of the objective enzymes can be produced by, for example, allowing a host having the objective enzyme gene encoding the objective enzyme (i.e. any of the benzaldehyde generation enzyme genes and catalase gene) to express the gene.

Each of the objective enzymes can also be produced by, for example, expressing the objective enzyme gene encoding the objective enzyme in a cell-free protein synthesis system.

Hereafter, production of the objective enzymes using a host having the objective enzyme gene is explained in detail.

### <3-1> Host

The host has the objective enzyme gene. Specifically, the host has the objective enzyme gene in such a manner that the gene can be expressed. The host may be or may not be a host inherently having the objective enzyme gene. The host may be, for example, a host not inherently having the objective enzyme gene but modified so as to have the objective enzyme gene. A host modified so as to have the objective enzyme gene can be obtained by introducing the objective enzyme gene into a host not having the objective enzyme gene. Means for introducing a gene will be described below. The phrase "having the objective enzyme gene" is also referred to as "having the objective enzyme".

The host may have been modified so that the activity of the objective enzyme is increased. Specifically, the host may have been modified so that the activity of the objective enzyme is increased as compared with a non-modified strain. For example, a host not inherently having the objective enzyme gene may be modified so that the activity of the objective enzyme is increased. That is, by introducing the objective enzyme gene into a host not inherently having the objective enzyme gene, the activity of the objective enzyme of the host can be increased (i.e. the activity of the objective enzyme can be imparted to the host). Also, for example, a host inherently having the objective enzyme gene may be modified so that the activity of the objective enzyme is increased. Means for increasing the activity of a protein (e.g. an enzyme) will be described below.

As the host, such a host as described below can be used as it is, or after appropriate modification (e.g. introduction of the objective enzyme gene or enhancement of the activity of the objective enzyme). That is, the host may be a modified strain derived from such a host as described below.

The host is not particularly limited, so long as it is able to express the functional objective enzyme. Examples of the host include microorganisms, plant cells, insect cells, and animal cells. Particular examples of the host include microorganisms. Examples of the microorganisms include bacteria and yeast. Particular examples of the microorganisms include bacteria.

Examples of the bacteria include bacteria belonging to the family *Enterobacteriaceae,* coryneform bacteria, and *Bacillus* bacteria.

Examples of bacteria belonging to the family *Enterobacteriaceae* include bacteria belonging to the genus *Escherichia, Enterobacter, Pantoea, Klebsiella, Serratia, Erwinia, Photorhabdus*, *Providencia, Salmonella, Morganella*, or the like. Specifically, bacteria classified into the family *Enterobacteriaceae* according to the taxonomy used in the NCBI (National Center for Biotechnology Information) database (http://www.ncbi.nlm.nih.gov/Taxonomy/Browser/wwwtax.cgi?id=91347) can be used. The *Escherichia* bacteria are not particularly limited, and examples thereof include those classified into the genus *Escherichia* according to the taxonomy known to those skilled in the field of microbiology. Examples of the *Escherichia* bacteria include, for example, those described in the work of Neidhardt et al. (Backmann B.J., 1996, Derivations and Genotypes of some mutant derivatives of Escherichia coli K-12, pp.2460-2488, Table 1, In F.D. Neidhardt (ed.), Escherichia coli and Salmonella Cellular and Molecular Biology/Second Edition, American Society for Microbiology Press, Washington, D.C.). Examples of the *Escherichia* bacteria include, for example, *Escherichia coli.* Specific examples of *Escherichia coli* include, for example, *Escherichia coli* K-12 strains such as W3110 strain (ATCC 27325) and MG1655 strain (ATCC 47076); *Escherichia coli* K5 strain (ATCC 23506); *Escherichia coli* B strains such as BL21 (DE3) strain; and derivative strains thereof. Examples the *Enterobacter* bacterium include, for example, *Enterobacter agglomerans* and *Enterobacter aerogenes.* Examples the *Pantoea* bacteria include, for example, *Pantoea ananatis*, *Pantoea stewartii, Pantoea agglomerans,* and *Pantoea citrea.* Examples of the *Erwinia* bacteria include *Erwinia amylovora* and *Erwinia carotovora.* Examples of the *Klebsiella* bacteria include *Klebsiella planticola.* The bacteria belonging to the family *Enterobacteriaceae* has been recently reclassified into a plurality of families on the basis of comprehensive comparative genome analysis (Adelou M. et al., Genome-based phylogeny and taxonomy of the 'Enterobacteriales': proposal for Enterobacterales ord. nov. divided into the families Enterobacteriaceae, Erwiniaceae fam. nov., Pectobacteriaceae fam. nov., Yersiniaceae fam. nov., Hafniaceae fam. nov., Morganellaceae fam. nov., and Budviciaceae fam. nov., Int. J. Syst. Evol. Microbiol., 2016, 66:5575-5599). However, in the present invention, bacteria previously the family *Enterobacteriaceae* shall be treated as bacteria belonging to the family *Enterobacteriaceae.*

Examples of the coryneform bacteria include bacteria belonging to the genus *Corynebacterium, Brevibacterium, Microbacterium,* or the like.

Specific examples of such coryneform bacteria include the following species.
*Corynebacterium acetoacidophilum*
*Corynebacterium acetoglutamicum*
*Corynebacterium alkanolyticum*
*Corynebacterium callunae*
*Corynebacterium crenatum*
*Corynebacterium glutamicum*
*Corynebacterium lilium*
*Corynebacterium melassecola*
*Corynebacterium thermoaminogenes (Corynebacterium efficiens)*
*Corynebacterium herculis*
*Brevibacterium divaricatum (Corynebacterium glutamicum)*
*Brevibacterium flavum (Corynebacterium glutamicum)*
*Brevibacterium immariophilum*
*Brevibacterium lactofermentum (Corynebacterium glutamicum)*
*Brevibacterium roseum*
*Brevibacterium saccharolyticum*
*Brevibacterium thiogenitalis*
*Corynebacterium ammoniagenes (Corynebacterium stationis)*
*Brevibacterium album*
*Brevibacterium cerinum*
*Microbacterium ammoniaphilum*

Specific examples of the coryneform bacteria include the following strains.
*Corynebacterium acetoacidophilum* ATCC 13870
*Corynebacterium acetoglutamicum* ATCC 15806
*Corynebacterium alkanolyticum* ATCC 21511
*Corynebacterium callunae* ATCC 15991
*Corynebacterium crenatum* AS1.542
*Corynebacterium glutamicum* ATCC 13020, ATCC 13032, ATCC 13060, ATCC 13869, FERM BP-734
*Corynebacterium lilium* ATCC 15990
*Corynebacterium melassecola* ATCC 17965
*Corynebacterium efficiens (Corynebacterium thermoaminogenes)* AJ12340 (FERM BP-1539)
*Corynebacterium herculis* ATCC 13868
*Brevibacterium divaricatum (Corynebacterium glutamicum)* ATCC 14020
*Brevibacterium flavum (Corynebacterium glutamicum)* ATCC 13826, ATCC 14067, AJ12418 (FERM BP-2205)
*Brevibacterium immariophilum* ATCC 14068
*Brevibacterium lactofermentum (Corynebacterium glutamicum)* ATCC 13869
*Brevibacterium roseum* ATCC 13825
*Brevibacterium saccharolyticum* ATCC 14066
*Brevibacterium thiogenitalis* ATCC 19240
*Corynebacterium ammoniagenes (Corynebacterium stationis)* ATCC 6871, ATCC 6872
*Brevibacterium album* ATCC 15111
*Brevibacterium cerinum* ATCC 15112
*Microbacterium ammoniaphilum* ATCC 15354

The coryneform bacteria include bacteria that had previously been classified into the genus *Brevibacterium,* but are presently united into the genus *Corynebacterium* (Int. J. Syst. Bacteriol., 41, 255 (1991)). Moreover, *Corynebacterium stationis* includes bacteria that had previously been classified as *Corynebacterium ammoniagenes,* but is presently re-classified into *Corynebacterium stationis* on the basis of nucleotide sequence analysis of 16S rRNA etc. (Int. J. Syst. Evol. Microbiol., 60, 874-879 (2010)).

Examples of the *Bacillus* bacteria include, for example, *Bacillus subtilis, Bacillus amyloliquefaciens, Bacillus pumilus, Bacillus licheniformis, Bacillus megaterium, Bacillus brevis, Bacillus polymixa*, and *Bacillus stearothermophilus.* Specific examples of *Bacillus subtilis* include, for example, the *Bacillus subtilis* 168 Marburg strain (ATCC 6051) and PY79 strain (Plasmid, 1984, 12, 1-9). Specific examples of *Bacillus amyloliquefaciens* include, for example, the *Bacillus amyloliquefaciens* T strain (ATCC 23842) and N strain (ATCC 23845).

Examples of the yeast include yeast belonging to the genus *Saccharomyces* such as *Saccharomyces cerevisiae,* the genus *Candida* such as *Candida utilis,* the genus *Pichia* such as *Pichia pastoris,* the genus *Hansenula* such as *Hansenula polymorpha,* and the genus *Schizosaccharomyces* such as *Schizosaccharomyces pombe.*

These strains are available from, for example, the American Type Culture Collection (Address: 12301 Parklawn Drive, Rockville, Maryland 20852, P.O. Box 1549, Manassas, VA20108, United States of America). That is, registration numbers are given to the respective strains, and the strains can be ordered by using these registration numbers (refer to http://www.atcc.org/). The registration numbers of the strains are listed in the catalogue of the American Type Culture Collection. These strains can also be obtained from, for example, the depositories at which the strains were deposited.

The objective enzyme gene can be obtained by, for example, cloning from an organism having the objective enzyme gene. For cloning, nucleotides containing the gene, such as genomic DNA and cDNA, can be used. Alternatively, the objective enzyme gene can be obtained by, for example, chemical synthesis (Gene, 60(1), 115-127 (1987)).

The obtained objective enzyme gene may be used as it is, or may be modified before use. That is, for example, the obtained objective enzyme gene can be modified, to thereby obtain a variant thereof. Genes can be modified by using a known method. For example, an objective mutation can be introduced into a target site of DNA by the site-specific mutagenesis method. That is, for example, the coding region of a gene can be modified by the site-specific mutation method so that a specific site of the encoded protein include substitution, deletion, insertion, and/or addition of amino acid residue(s). Examples of the site-specific mutagenesis method include a method of using PCR (Higuchi, R., 61, in PCR Technology, Erlich, H.A. Eds., Stockton Press, 1989; Carter P., Meth., in Enzymol., 154, 382, 1987), and a method of using a phage (Kramer, W. and Frits, H.J., Meth. in Enzymol., 154, 350, 1987; Kunkel, T.A. et al., Meth. in Enzymol., 154, 367, 1987).

In addition, a mutant HMAS gene can be obtained by, for example, modifying a wild-type HMAS gene so that HMAS encoded thereby has the "specific mutation". Such modification can be performed by using a known method such as the site-specific mutagenesis method. Alternatively, a mutant HMAS gene can also be obtained without using a wild-type HMAS gene. For example, a mutant HMAS gene may be directly obtained by chemical synthesis. The obtained mutant HMAS gene may be used as it is, or may be further modified before use.

The method for introducing the objective enzyme gene into a host is not particularly limited. It is sufficient that the objective enzyme gene is harbored by a host in such a manner that the gene can be expressed. The objective enzyme gene can be introduced into a host by the same way as that for introduction of a gene described below in the "Methods for increasing activity of protein".

In addition, when a host already has an HMAS gene on the chromosome thereof or the like, the host can also be modified to have a mutant HMAS gene by introducing the "specific mutation" into the HMAS gene on the chromosome or the like. A mutation can be introduced into a gene on a chromosome or the like by, for example, natural mutation, mutagenesis treatment, or genetic engineering.

The host may have L-phenylalanine-producing ability. The term "host having L-phenylalanine-producing ability" may refer to a host that is able to biosynthesize L-phenylalanine upon being cultured in a culture medium, such as a culture medium containing a carbon source. Hence, specifically, the term "host having L-phenylalanine-producing ability" may refer to a host that is able to biosynthesize L-phenylalanine from a carbon source. The biosynthesized L-phenylalanine may be used as a raw material for production of benzaldehyde. Hence, specifically, the term "host having L-phenylalanine-producing ability" may also refer to a host that is able to biosynthesize L-phenylalanine in an amount required as a raw material for production of benzaldehyde. The biosynthesized L-phenylalanine may be or may not be accumulated as a product, for example, in cells and/or the culture medium. That is, the biosynthesized L-phenylalanine may be immediately consumed. For example, the biosynthesized L-phenylalanine may be immediately converted into benzaldehyde or an intermediate thereof. Therefore, in an embodiment, L-phenylalanine-producing ability may be measured on the basis of production of benzaldehyde or an intermediate thereof.

Furthermore, the host of the present invention may be a host inherently having L-phenylalanine-producing ability, or may be a host modified so that it has L-phenylalanine-producing ability. The host having L-phenylalanine-producing ability can be obtained by imparting L-phenylalanine-producing ability to such a host as mentioned above, or enhancing L-phenylalanine-producing ability of such a host as mentioned above.

Hereafter, specific examples of the method for imparting or enhancing L-phenylalanine-producing ability will be explained. Such modifications as exemplified below for imparting or enhancing L-phenylalanine-producing ability may be independently used, or may be used in an appropriate combination.

Examples of the method for imparting or enhancing L-phenylalanine-producing ability include a method of increasing the activity of an L-phenylalanine biosynthesis enzyme. That is, the host may have been modified so that the activity of an L-phenylalanine biosynthesis enzyme is increased. The activity of one kind of L-phenylalanine biosynthesis enzyme may be increased, or the activities of two or more kinds of L-phenylalanine biosynthesis enzymes may be increased. The method for increasing the activity of a protein (enzyme etc.) will be described later. The activity of a protein (enzyme etc.) can be increased by, for example, increasing the expression of a gene encoding the protein.

Examples of the L-phenylalanine biosynthesis enzymes include common biosynthesis enzymes of aromatic amino acids, such as 3-deoxy-D-arabinoheptulosonate-7-phosphate synthase (*aroF*, *aroG*, *aroH*), 3-dehydroquinate synthase (*aroB*), 3-dehydroquinate dehydratase (*aroD*), shikimate dehydrogenase (*aroE*), shikimate kinase (*aroK*, *aroL*), 5-enolpyruvylshikimate-3-phosphate synthase (*aroA*), and chorismate synthase (*aroC*); as well as chorismate mutase *(pheA),* prephenate dehydratase *(pheA),* and tyrosine amino transferase *(tyrB).* Shown in the parentheses after the names of the enzymes are examples of the names of the genes encoding the enzymes (the same shall apply to the same occasions hereafter). Chorismate mutase and prephenate dehydratase may be encoded by *pheA* gene as a bifunctional enzyme. The expression of genes encoding some L-phenylalanine biosynthesis enzymes, such as DAHP synthase, 3-dehydroquinate synthase, and 3-dehydroquinate dehydratase, can be repressed by a tyrosine repressor TyrR, which is encoded by *tyrR* gene. Therefore, the activity of an L-phenylalanine biosynthesis enzyme can be increased by, for example, reducing the activity of the tyrosine repressor TyrR. In addition, some L-phenylalanine biosynthesis enzymes can be subject to feedback inhibition by aromatic amino acid(s) such as L-phenylalanine. For example, the bifunctional chorismate mutase-prephenate dehydratase can be subject to feedback inhibition by L-phenylalanine. Therefore, the activity of an L-phenylalanine biosynthesis enzyme can be increased by, for example, using a gene encoding a mutant L-phenylalanine biosynthesis enzyme desensitized to such feedback inhibition.

Examples of the method for imparting or enhancing L-phenylalanine-producing ability further include a method of reducing the activity of an enzyme that is involved in the by-production of a substance other than L-phenylalanine. Such a substance other than L-phenylalanine is also referred to as "byproduct". Examples of the byproduct include other aromatic amino acids such as L-tyrosine and L-tryptophan. Examples of the enzyme that is involved in the by-production of L-tyrosine include a bifunctional enzyme chorismate mutase-prephenate dehydrogenase *(tyrA).*

Specific examples of L-phenylalanine-producing bacteria and parent strains for deriving them include, for example, *E. coli* AJ12739 (tyrA::Tn10, tyrR) (VKPM B-8197), which is deficient in the chorismate mutase-prephenate dehydrogenase and the tyrosine repressor (WO03/044191), *E. coli* AJ12741, which is deficient in the chorismate mutase-prephenate dehydrogenase and the tyrosine repressor, and contains a mutant *aroG* gene encoding 3-deoxy-D-arabinoheptulosonate-7-phosphate synthase desensitized to feedback inhibition, a mutant *pheA* gene encoding chorismate mutase-prephenate dehydratase desensitized to feedback inhibition, and an *aroL* gene encoding shikimate kinase (JP H05-344881 A), *E. coli* HW1089 (ATCC 55371), which contains *pheA34* gene encoding a chorismate mutase-prephenate dehydratase desensitized to feedback inhibition (U.S. Patent No. 5,354,672), *E. coli* MWEC101-b (KR8903681), *E*. *coli* NRRL B-12141, NRRL B-12145, NRRL B-12146, and NRRL B-12147 (U.S. Patent No. 4,407,952). Specific examples of L-phenylalanine-producing bacteria and parent strains for deriving them also include, for example, *E. coli* K-12 <W3110(tyrA)/pPHAB> (FERM BP-3566), *E. coli* K-12 <W3110(tyrA)/pPHAD> (FERM BP-12659), *E. coli* K-12 <W3110(tyrA)/pPHATerm> (FERM BP-12662), and *E. coli* K-12 AJ12604 <W3110(tyrA)/pBR-aroG4, pACMAB> (FERM BP-3579), which contains a gene encoding a chorismate mutase-prephenate dehydratase desensitized to feedback inhibition (EP 488424 B1). Specific examples of L-phenylalanine-producing bacteria and parent strains for deriving them further include, for example, strains belonging to the genus *Escherichia* having an increased activity of the protein encoded by the *yedA* gene or the *yddG* gene (U.S. Patent Published Applications Nos. 2003/0148473 and 2003/0157667, WO03/044192). Specific examples of L-phenylalanine-producing bacteria and parent strains for deriving them also include, for example, *Corynebacterium glutamicum* strains BPS-13 (FERM BP-1777), K77 (FERM BP-2062), and K78 (FERM BP-2063) (EP 331145 A, JP H02-303495 A), of which phosphoenolpyruvate carboxylase or pyruvate kinase activity is reduced, and tyrosine-auxotrophic strains of coryneform bacteria (JP H05-049489 A).

The genes and proteins used for breeding a host having L-phenylalanine-producing ability may be, for example, a gene having any of known nucleotide sequences and a protein having any of amino acid sequences, respectively. Also, the genes and proteins used for breeding a host having L-phenylalanine-producing ability may be a conservative variant of a gene having any of known nucleotide sequences and a conservative variant of a protein having any of any of known amino acid sequences, respectively. Specifically, for example, the genes used for breeding a host having L-phenylalanine-producing ability may each be a gene encoding a protein having a known amino acid sequence of a protein, but including substitution, deletion, insertion, and/or addition of one or several some amino acid residues at one or several positions, so long as the original function thereof, i.e. enzymatic activity etc., is maintained. As for such conservative variants of genes and proteins, the aforementioned descriptions concerning the conservative variants of the benzaldehyde generation enzyme genes and benzaldehyde generation enzymes can be similarly applied.

The host may have one objective enzyme gene, or may have two or more objective enzyme genes. The host may have, for example, one, two, three, or all four enzymes selected from the four benzaldehyde generation enzyme genes (i.e. AAD, HMAS, SMDH, and BFDC genes). The host may have, for example, the catalase gene in addition to one, two, three, or all four enzymes selected from the four benzaldehyde generation enzyme genes (i.e. AAD, HMAS, SMDH, and BFDC genes).

The host may have any other characteristics, so long as it can produce the objective enzyme.

The order of modifications for constructing the host is not particularly limited.

### <3-2> Microorganism of the present invention

The host may be configured as, in particular, a microorganism (specifically, at least one microorganism) having the four benzaldehyde generation enzymes (i.e. AAD, HMAS, SMDH, and BFDC). The microorganism (specifically, at least one microorganism) having the four benzaldehyde generation enzymes (i.e. AAD, HMAS, SMDH, and BFDC) is also referred to as "the microorganism of the present invention"

The expression "a microorganism has a benzaldehyde generation enzyme" can mean that the microorganism expresses and retains the functional benzaldehyde generation enzyme. The expression "a microorganism has a benzaldehyde generation enzyme" can mean that, specifically, the microorganism expresses and retains the functional benzaldehyde generation enzyme in cells.

The microorganism of the present invention may be a single microorganism, or may be a combination of a plurality of microorganisms. That is, in the present invention, the term "microorganism" as a singular form, such as "a microorganism" and "the microorganism", may be read as at least one microorganism, i.e. either a single microorganism or a combination of a plurality of microorganisms, according to the context.

In cases where the microorganism of the present invention is a single microorganism, this single microorganism alone has all the four benzaldehyde generation enzymes.

In cases where the microorganism of the present invention is a combination of a plurality of microorganisms, this combination has all the four benzaldehyde generation enzymes as a whole. In other words, in cases where the microorganism of the present invention is a combination of a plurality of microorganisms, the four benzaldehyde generation enzymes each are harbored by at least any one of the plurality of microorganisms. The benzaldehyde generation enzymes each may be harbored by any one of the plurality of microorganisms, or by two or more of the plurality of microorganisms. The plurality of microorganisms each may have any one of the benzaldehyde generation enzymes, or two or more of the benzaldehyde generation enzymes. The kind(s) of the benzaldehyde generation enzyme(s) harbored by each of the plurality of microorganisms is/are not particularly limited, so long as production of benzaldehyde is attained. The kind(s) of the benzaldehyde generation enzyme(s) harbored by each of the plurality of microorganisms can be appropriately chosen depending on the various conditions such as the mode for carrying out the production step, e.g. configuration of substeps in the production step. For example, the microorganism of the present invention may be a combination of four microorganisms having the four respective benzaldehyde generation enzymes, i.e. a combination of a microorganism having AAD, a microorganism having HMAS, a microorganism having SMDH, and a microorganism having BFDC. The plurality of microorganisms may be or may not be the same with each other, except for the kind(s) of the benzaldehyde generation enzyme(s) harbored by each of the plurality of microorganisms. For example, the plurality of microorganisms may be or may not be microorganisms derived from the same genus, species, or strain.

The microorganism of the present invention may have catalase.

In cases where the microorganism of the present invention is a single microorganism, this single microorganism may have catalase.

In cases where the microorganism of the present invention is a combination of a plurality of microorganisms, any one or more microorganisms of the plurality of microorganisms may have catalase. Which microorganism(s) of the plurality of microorganisms has/have catalase is not particularly limited, so long as production of benzaldehyde is improved. For example, a microorganism at least having HMAS may have catalase. Alternatively, for example, a microorganism to be used in coexistence with a microorganism at least having HMAS may have catalase.

The microorganism of the present invention may have L-phenylalanine-producing ability.

In cases where the microorganism of the present invention is a single microorganism, this single microorganism may have L-phenylalanine-producing ability.

In cases where the microorganism of the present invention is a combination of a plurality of microorganisms, any one or more microorganisms of the plurality of microorganisms may have L-phenylalanine-producing ability. Which microorganism(s) of the plurality of microorganisms has/have L-phenylalanine-producing ability is not particularly limited, so long as production of benzaldehyde is attained. For example, a microorganism at least having AAD may have L-phenylalanine-producing ability. Alternatively, for example, a microorganism to be used in coexistence with a microorganism at least having AAD may have L-phenylalanine-producing ability.

Incidentally, the microorganism of the present invention has benzaldehyde-producing ability. The term "microorganism having benzaldehyde-producing ability" refers to a microorganism that is able to produce benzaldehyde. The term "microorganism having benzaldehyde-producing ability" may refer to a microorganism that is able to produce benzaldehyde by fermentation, substance conversion, or a combination thereof. That is, the term "microorganism having benzaldehyde-producing ability" may refer to a microorganism that is able to produce benzaldehyde from a carbon source or L-phenylalanine. Specifically, the term "microorganism having benzaldehyde-producing ability" may refer to a microorganism that is able to, upon being cultured in a culture medium containing a carbon source, produce and accumulate benzaldehyde in the culture medium, which intermediate can further be converted into benzaldehyde. Also, specifically, the term "microorganism having benzaldehyde-producing ability" may refer to a microorganism that is able to, upon being cultured in a culture medium containing L-phenylalanine or upon being allowed to coexist with or act on L-phenylalanine in a reaction mixture, produce and accumulate benzaldehyde in the culture medium or reaction mixture, which intermediate can further be converted into benzaldehyde.

Benzaldehyde can be produced from L-phenylalanine by the action of the four benzaldehyde generation enzymes. Hence, the microorganism of the present invention, which has the four benzaldehyde generation enzymes, may be able to produce benzaldehyde from L-phenylalanine. For example, the microorganism of the present invention may be able to produce benzaldehyde from L-phenylalanine via a step of producing benzaldehyde by substance conversion, or via a combination of a substep of producing an intermediate of benzaldehyde by substance conversion and subsequent substep(s) of converting the intermediate into benzaldehyde.

When the microorganism of the present invention has L-phenylalanine-producing ability, the microorganism of the present invention may also be able to produce benzaldehyde from a carbon source. For example, the microorganism of the present invention may be able to produce benzaldehyde from a carbon source via a step of producing benzaldehyde by fermentation, or via a combination of a substep of producing an intermediate of benzaldehyde by fermentation and subsequent substep(s) of converting the intermediate into benzaldehyde.

The microorganism of the present invention may be able to accumulate benzaldehyde in the culture medium or reaction mixture in such a degree that benzaldehyde can be collected therefrom. The microorganism of the present invention may be able to accumulate benzaldehyde in the culture medium or reaction mixture in an amount of, for example, 0.01 mM or more, 0.1 mM more, or 1 mM more.

In cases where the microorganism of the present invention is a single microorganism, this single microorganism has benzaldehyde-producing ability.

In cases where the microorganism of the present invention is a combination of a plurality of microorganisms, this combination has benzaldehyde-producing ability as a whole.

The microorganism of the present invention may further have any other modification(s), so long as production of benzaldehyde is attained.

### <3-3> Methods for increasing activity of protein>

Hereafter, the methods for increasing the activity of a protein (including methods for introducing a gene) will be explained.

The expression "the activity of a protein is increased" can mean that the activity of the protein is increased as compared with a non-modified strain. Specifically, the expression "the activity of a protein is increased" may mean that the activity of the protein per cell is increased as compared with that of a non-modified strain. The term "the activity of a protein per cell" may mean an average value of the activity of the protein per cell. The non-modified strain is also referred to as "non-modified host" or "strain of non-modified host". The term "non-modified strain" referred to herein can refer to a control strain that has not been modified so that the activity of an objective protein is increased. Examples of the non-modified strain can include a wild-type strain and parent strain. Specific examples of the non-modified strain can include the type strains of the species to which the host belongs. Specific examples of the non-modified strain can also include strains exemplified above in relation to the description of the host. That is, in an embodiment, the activity of a protein may be increased as compared with a type strain, i.e. a type strain of the species to which the host belongs. In another embodiment, the activity of a protein may also be increased as compared with the C. *glutamicum* ATCC 13869 strain. In another embodiment, the activity of a protein may also be increased as compared with the C. *glutamicum* ATCC 13032 strain. In another embodiment, the activity of a protein may also be increased as compared with the *E. coli* K-12 MG1655 strain. The state that "the activity of a protein is increased" may also be expressed as "the activity of a protein is enhanced". More specifically, the expression "the activity of a protein is increased" may mean that the number of molecules of the protein per cell is increased, and/or the function of each molecule of the protein is increased as compared with those of a non-modified strain. That is, the term "activity" in the expression "the activity of a protein is increased" is not limited to the catalytic activity of the protein, but may also mean the transcription amount of a gene (i.e. the amount of mRNA) encoding the protein, or the translation amount of the gene (i.e. the amount of the protein). The term "the number of molecules of a protein per cell" may mean an average value of the number of molecules of the protein per cell. Furthermore, the state that "the activity of a protein is increased" can include not only a state that the activity of an objective protein is increased in a strain inherently having the activity of the objective protein, but also a state that the activity of an objective protein is imparted to a strain not inherently having the activity of the objective protein. Furthermore, so long as the activity of the protein is eventually increased, the activity of an objective protein inherently contained in a host may be attenuated and/or eliminated, and then an appropriate type of the objective protein may be imparted to the host.

The degree of the increase in the activity of a protein is not particularly limited, so long as the activity of the protein is increased as compared with a non-modified strain. The activity of the protein may be increased to, for example, 1.2 times or more, 1.5 times or more, 2 times or more, or 3 times or more of that of a non-modified strain. Furthermore, when the non-modified strain does not have the activity of the objective protein, it is sufficient that the protein is produced as a result of introduction of the gene encoding the protein, and for example, the protein may be produced to such an extent that the activity thereof can be measured.

The modification for increasing the activity of a protein can be attained by, for example, increasing the expression of a gene encoding the protein. The expression "the expression of a gene is increased" can mean that the expression of the gene is increased as compared with a non-modified strain such as a wild-type strain and parent strain. Specifically, the expression "the expression of a gene is increased" may mean that the expression amount of the gene per cell is increased as compared with that of a non-modified strain. The term "the expression amount of a gene per cell" may mean an average value of the expression amount of the gene per cell. More specifically, the expression "the expression of a gene is increased" may mean that the transcription amount of the gene (i.e. the amount of mRNA) is increased, and/or the translation amount of the gene (i.e. the amount of the protein expressed from the gene) is increased. The state that "the expression of a gene is increased" can also be referred to as "the expression of a gene is enhanced". The expression of a gene may be increased to, for example, 1.2 times or more, 1.5 times or more, 2 times or more, or 3 times or more of that of a non-modified strain. Furthermore, the state that "the expression of a gene is increased" can include not only a state that the expression amount of an objective gene is increased in a strain that inherently expresses the objective gene, but also a state that the gene is introduced into a strain that does not inherently express the objective gene, and expressed therein. That is, the phrase "the expression of a gene is increased" may also mean, for example, that an objective gene is introduced into a strain that does not possess the gene, and is expressed therein.

The expression of a gene can be increased by, for example, increasing the copy number of the gene.

The copy number of a gene can be increased by introducing the gene into the chromosome of a host. A gene can be introduced into a chromosome by, for example, using homologous recombination (Miller, J.H., Experiments in Molecular Genetics, 1972, Cold Spring Harbor Laboratory). Examples of the gene transfer method utilizing homologous recombination can include, for example, a method of using a linear DNA such as Red-driven integration (Datsenko, K.A., and Wanner, B.L., Proc. Natl. Acad. Sci. USA, 97:6640-6645 (2000)), a method of using a plasmid containing a temperature sensitive replication origin, a method of using a plasmid capable of conjugative transfer, a method of using a suicide vector not having a replication origin that functions in a host, and a transduction method using a phage. Only one copy, or two or more copies of a gene may be introduced. For example, by performing homologous recombination using a sequence which is present in multiple copies on a chromosome as a target, multiple copies of a gene can be introduced into the chromosome. Examples of such a sequence which is present in multiple copies on a chromosome can include repetitive DNAs, and inverted repeats located at the both ends of a transposon. Alternatively, homologous recombination may be performed by using an appropriate sequence on a chromosome such as a gene unnecessary for the production of the objective substance as a target. Furthermore, a gene can also be randomly introduced into a chromosome by using a transposon or Mini-Mu (Japanese Patent Laid-open (Kokai) No. 2-109985, U.S. Patent No. 5,882,888, EP 805867 B1).

Introduction of a target gene into a chromosome can be confirmed by Southern hybridization using a probe having a sequence complementary to the whole gene or a part thereof, PCR using primers prepared on the basis of the sequence of the gene, or the like.

Furthermore, the copy number of a gene can also be increased by introducing a vector containing the gene into a host. For example, the copy number of a target gene can be increased by ligating a DNA fragment containing the target gene with a vector that functions in a host to construct an expression vector of the gene, and transforming the host with the expression vector. The DNA fragment containing the target gene can be obtained by, for example, PCR using the genomic DNA of a microorganism having the target gene as the template. As the vector, a vector autonomously replicable in the cell of the host can be used. The vector can be a multi-copy vector. Furthermore, the vector may have a marker such as an antibiotic resistance gene for selection of transformant. Furthermore, the vector may have a promoter and/or terminator for expressing the introduced gene. The vector may be, for example, a vector derived from a bacterial plasmid, a vector derived from a yeast plasmid, a vector derived from a bacteriophage, cosmid, phagemid, or the like. Specific examples of vector autonomously replicable in *Enterobacteriaceae* bacteria such as *Escherichia coli* can include, for example, pUC19, pUC18, pHSG299, pHSG399, pHSG398, pBR322, pSTV29 (all of these are available from Takara), pACYC184, pMW219 (NIPPON GENE), pTrc99A (Pharmacia), pPROK series vectors (Clontech), pKK233-2 (Clontech), pET series vectors (Novagen), pQE series vectors (QIAGEN), pCold TF DNA (Takara Bio), pACYC series vectors, and the broad host spectrum vector RSF1010. Specific examples of vector autonomously replicable in coryneform bacteria can include, for example, pHM1519 (Agric. Biol. Chem., 48, 2901-2903 (1984)); pAM330 (Agric. Biol. Chem., 48, 2901-2903 (1984)); plasmids obtained by improving these and having a drug resistance gene; plasmid pCRY30 described in Japanese Patent Laid-open (Kokai) No. 3-210184; plasmids pCRY21, pCRY2KE, pCRY2KX, pCRY31, pCRY3KE, and pCRY3KX described in Japanese Patent Laid-open (Kokai) No. 2-72876 and U.S. Patent No. 5,185,262; plasmids pCRY2 and pCRY3 described in Japanese Patent Laid-open (Kokai) No. 1-191686; pAJ655, pAJ611, and pAJ1844 described in Japanese Patent Laid-open (Kokai) No. 58-192900; pCG1 described in Japanese Patent Laid-open (Kokai) No. 57-134500; pCG2 described in Japanese Patent Laid-open (Kokai) No. 58-35197; pCG4 and pCG11 described in Japanese Patent Laid-open (Kokai) No. 57-183799; pPK4 described in U.S. Patent No. 6,090,597; pVK4 described in Japanese Patent Laid-open (Kokai) No. 9-322774; pVK7 described in Japanese Patent Laid-open (Kokai) No. 10-215883; pVK9 described in WO2007/046389; pVS7 described in WO2013/069634; and pVC7 described in Japanese Patent Laid-open (Kokai) No. 9-070291. Specific examples of vector autonomously replicable in coryneform bacteria can also include, for example, variants of pVC7 such as pVC7H2 (WO2018/179834).

When a gene is introduced, it is sufficient that the gene is able to be expressed by a host. Specifically, it is sufficient that the gene is harbored by a host so that it is expressed under control of a promoter that functions in the host. The term "a promoter that functions in a host" can refer to a promoter that shows a promoter activity in the host. The promoter may be a promoter derived from the host, or a heterogenous promoter. The promoter may be the native promoter of the gene to be introduced, or a promoter of another gene. As the promoter, for example, such a stronger promoter as described herein may also be used.

A terminator for termination of gene transcription may be located downstream of the gene. The terminator is not particularly limited so long as it functions in a host. The terminator may be a terminator derived from the host, or a heterogenous terminator. The terminator may be the native terminator of the gene to be introduced, or a terminator of another gene. Specific examples of the terminator can include, for example, T7 terminator, T4 terminator, fd phage terminator, *tet* terminator, and *trpA* terminator.

Vectors, promoters, and terminators available in various microorganisms are disclosed in detail in "Fundamental Microbiology Vol. 8, Genetic Engineering, KYORITSU SHUPPAN CO., LTD, 1987", and those can be used.

Furthermore, when two or more of genes are introduced, it is sufficient that the genes each are harbored by a host in such a manner that the genes are able to be expressed. For example, all the genes may be carried by a single expression vector or a chromosome. Furthermore, the genes may be separately carried by two or more expression vectors, or separately carried by a single or two or more expression vectors and a chromosome. An operon made up of two or more genes may also be introduced. When "introducing two or more genes", for example, respective genes encoding two or more kinds of proteins (such as enzymes), respective genes encoding two or more subunits constituting a single protein complex (such as enzyme complex), or a combination of these genes may be introduced.

The gene to be introduced is not particularly limited so long as it encodes a protein that functions in the host. The gene to be introduced may be a gene derived from the host, or may be a heterogenous gene. The gene to be introduced can be obtained by, for example, PCR using primers designed on the basis of the nucleotide sequence of the gene, and using the genomic DNA of an organism having the gene, a plasmid carrying the gene, or the like as a template. The gene to be introduced may also be totally synthesized, for example, on the basis of the nucleotide sequence of the gene (Gene, 60(1), 115-127 (1987)). The obtained gene can be used as it is, or after being modified as required. That is, a variant of a gene may be obtained by modifying the gene. A gene can be modified by a known technique. For example, an objective mutation can be introduced into an objective site of DNA by the site-specific mutation method. That is, the coding region of a gene can be modified by the site-specific mutation method so that a specific site of the encoded protein includes substitution, deletion, insertion, and/or addition of amino acid residues. Examples of the site-specific mutation method can include the method utilizing PCR (Higuchi, R., 61, in PCR Technology, Erlich, H.A. Eds., Stockton Press (1989); Carter, P., Meth. in Enzymol., 154, 382 (1987)), and the method utilizing phage (Kramer, W. and Frits, H.J., Meth. in Enzymol., 154, 350 (1987); Kunkel, T.A. et al., Meth. in Enzymol., 154, 367 (1987)). Alternatively, a variant of a gene may be totally synthesized.

Incidentally, when a protein functions as a complex consisting of a plurality of subunits, a part or all of these subunits may be modified, so long as the activity of the protein is eventually increased. That is, for example, when the activity of a protein is increased by increasing the expression of a gene, the expression of a part or all of these genes that encode the respective subunits may be enhanced. It is usually preferable to enhance the expression of all of those genes encoding the subunits. Furthermore, the subunits constituting the complex may be derived from one kind of organism or two or more kinds of organisms, so long as the complex has a function of the objective protein. That is, for example, genes of the same organism encoding a plurality of subunits may be introduced into a host, or genes of different organisms encoding a plurality of subunits may be introduced into a host.

Furthermore, the expression of a gene can be increased by improving the transcription efficiency of the gene. In addition, the expression of a gene can also be increased by improving the translation efficiency of the gene. The transcription efficiency of the gene and the translation efficiency of the gene can be improved by, for example, modifying an expression control sequence of the gene. The term "expression control sequence" collectively can refer to sites that affect the expression of a gene. Examples of the expression control sequence can include, for example, promoter, Shine-Dalgarno (SD) sequence (also referred to as ribosome binding site (RBS)), and spacer region between RBS and the start codon. Expression control sequences can be identified by using a promoter search vector or gene analysis software such as GENETYX. These expression control sequences can be modified by, for example, a method of using a temperature sensitive vector, or the Red driven integration method (WO2005/010175).

The transcription efficiency of a gene can be improved by, for example, replacing the promoter of the gene on a chromosome with a stronger promoter. The term "stronger promoter" can mean a promoter providing an improved transcription of a gene compared with an inherently existing wild-type promoter of the gene. Examples of stronger promoters can include, for example, the known high expression promoters such as T7 promoter, *trp* promoter, *lac* promoter, *thr* promoter, *tac* promoter, *trc* promoter, *tet* promoter, *araBAD* promoter, *rpoH* promoter, *msrA* promoter, Pm1 promoter (derived from the genus *Bifidobacterium),* PR promoter, and PL promoter. Examples of stronger promoters usable in coryneform bacteria can include, for example, the artificially modified P54-6 promoter (Appl. Microbiol. Biotechnol., 53, 674-679 (2000)), *pta*, *aceA*, *aceB*, *adh*, and *amyE* promoters inducible in coryneform bacteria with acetic acid, ethanol, pyruvic acid, or the like, and *cspB, SOD,* and *tuf* (EF-Tu) promoters, which are potent promoters capable of providing a large expression amount in coryneform bacteria (Journal of Biotechnology, 104 (2003) 311-323; Appl. Environ. Microbiol., 2005 Dec; 71 (12):8587-96), as well as P2 promoter (WO2018/079684), P3 promoter (WO2018/079684), F1 promoter (WO2018/179834), *lac* promoter, *tac* promoter, *trc* promoter, and F1 promoter. Furthermore, as the stronger promoter, a highly-active type of an inherent promoter may also be obtained by using various reporter genes. For example, by making the -35 and -10 regions in a promoter region closer to the consensus sequence, the activity of the promoter can be enhanced (WO00/18935). Examples of highly active-type promoter can include various tac-like promoters (Katashkina JI et al., Russian Federation Patent Application No. 2006134574). Methods for evaluating the strength of promoters and examples of strong promoters are described in the paper of Goldstein et al. (Prokaryotic Promoters in Biotechnology, Biotechnol. Annu. Rev., 1, 105-128 (1995)), and so forth.

The translation efficiency of a gene can be improved by, for example, replacing the Shine-Dalgarno (SD) sequence (also referred to as ribosome binding site (RBS)) for the gene on a chromosome with a stronger SD sequence. The "stronger SD sequence" can mean a SD sequence that provides an improved translation of mRNA compared with the inherent wild-type SD sequence of the gene. Examples of stronger SD sequences can include, for example, RBS of the gene 10 derived from phage T7 (Olins P.O. et al, Gene, 1988, 73, 227-235). Furthermore, it is known that substitution, insertion, or deletion of several nucleotides in a spacer region between RBS and the start codon, especially in a sequence immediately upstream of the start codon (5'-UTR), significantly affects the stability and translation efficiency of mRNA, and hence, the translation efficiency of a gene can also be improved by modifying them.

The translation efficiency of a gene can also be improved by, for example, modifying codons. For example, the translation efficiency of the gene can be improved by replacing a rare codon present in the gene with a synonymous codon that is more frequently used. That is, the gene to be introduced may be modified, for example, so as to contain optimal codons according to the frequencies of codons observed in a host to be used. Codons can be replaced by, for example, the site-specific mutation method for introducing an objective mutation into an objective site of DNA. Alternatively, a gene fragment in which objective codons are replaced may be totally synthesized. Frequencies of codons in various organisms are disclosed in the "Codon Usage Database" (www.kazusa.or.jp/codon; Nakamura, Y. et al, Nucl. Acids Res., 28, 292 (2000)).

Furthermore, the expression of a gene can also be increased by amplifying a regulator that increases the expression of the gene, or deleting or attenuating a regulator that reduces the expression of the gene.

Such methods for increasing the gene expression as mentioned above may be used independently or in any combination.

Furthermore, the modification that increases the activity of a protein can also be attained by, for example, enhancing the specific activity of the enzyme. Enhancement of the specific activity can also include desensitization to feedback inhibition. That is, when a protein is subject to feedback inhibition by a metabolite, the activity of the protein can be increased by mutating a gene or protein in a host so as to desensitize the feedback inhibition. The expression "desensitization to feedback inhibition" can include complete elimination of the feedback inhibition, and attenuation of the feedback inhibition, unless otherwise stated. Also, the state of "being desensitized to feedback inhibition", i.e. the state that feedback inhibition is eliminated or attenuated, can also be referred to as "tolerant to feedback inhibition". A protein showing an enhanced specific activity can be obtained by, for example, searching various organisms. Furthermore, a highly-active type of an inherent protein may also be obtained by introducing a mutation into the existing protein. The mutation to be introduced may be, for example, substitution, deletion, insertion, and/or addition of one or several amino acid residues at one or several positions in the protein. The mutation can be introduced by, for example, such a site-specific mutation method as mentioned above. The mutation may also be introduced by, for example, a mutagenesis treatment. Examples of the mutagenesis treatment can include irradiation of X-ray, irradiation of ultraviolet, and a treatment with a mutation agent such as N-methyl-N'-nitro-N-nitrosoguanidine (MNNG), ethyl methanesulfonate (EMS), and methyl methanesulfonate (MMS). Furthermore, a random mutation may be induced by directly treating DNA in vitro with hydroxylamine. Enhancement of the specific activity may be independently used, or may be used in any combination with such methods for enhancing gene expression as mentioned above.

The method for the transformation is not particularly limited, and conventionally known methods can be used. There can be used, for example, a method of treating recipient cells with calcium chloride so as to increase the permeability thereof for DNA, which has been reported for the *Escherichia coli* K-12 strain (Mandel, M. and Higa, A., J. Mol. Biol., 1970, 53, 159-162), and a method of preparing competent cells from cells which are in the growth phase, followed by transformation with DNA, which has been reported for *Bacillus subtilis* (Duncan, C.H., Wilson, G.A. and Young, F.E., Gene, 1997, 1:153-167). Alternatively, there can also be used a method of making DNA-recipient cells into protoplasts or spheroplasts, which can easily take up recombinant DNA, followed by introducing a recombinant DNA into the DNA-recipient cells, which is known to be applicable to *Bacillus subtilis,* actinomycetes, and yeasts (Chang, S. and Choen, S.N., 1979, Mol. Gen. Genet., 168:111-115; Bibb, M.J., Ward, J.M. and Hopwood, O.A., 1978, Nature, 274:398-400; Hinnen, A., Hicks, J.B. and Fink, G.R., 1978, Proc. Natl. Acad. Sci. USA, 75:1929-1933). Furthermore, the electric pulse method reported for coryneform bacteria (Japanese Patent Laid-open (Kokai) No. 2-207791) can also be used.

An increase in the activity of a protein can be confirmed by measuring the activity of the protein.

An increase in the activity of a protein can also be confirmed by confirming an increase in the expression of a gene encoding the protein. An increase in the expression of a gene can be confirmed by confirming an increase in the transcription amount of the gene, or by confirming an increase in the amount of a protein expressed from the gene.

An increase of the transcription amount of a gene can be confirmed by comparing the amount of mRNA transcribed from the gene with that of a non-modified strain such as a wild-type strain or parent strain. Examples of the method for evaluating the amount of mRNA can include Northern hybridization, RT-PCR, microarray, RNA-seq, and so forth (Sambrook, J., et al., Molecular Cloning A Laboratory Manual/Third Edition, Cold Spring Harbor Laboratory Press, Cold Spring Harbor (USA), 2001). The amount of mRNA may increase to, for example, 1.2 times or more, 1.5 times or more, 2 times or more, or 3 times or more of that of a non-modified strain.

An increase in the amount of a protein can be confirmed by Western blotting using antibodies (Molecular Cloning, Cold Spring Harbor Laboratory Press, Cold Spring Harbor (USA), 2001). The amount of the protein (such as the number of molecules of the protein per cell) may increase to, for example, 1.2 times or more, 1.5 times or more, 2 times or more, or 3 times or more of that of a non-modified strain.

The aforementioned methods for increasing the activity of a protein can be applied to enhancement of the activities of any proteins and enhancement of the expression of any genes.

### <3-4> Method for reducing activity of protein>

Hereafter, the methods for reducing the activity of a protein will be explained.

The expression "the activity of a protein is reduced" can mean that the activity of the protein is reduced as compared with a non-modified strain. Specifically, the expression "the activity of a protein is reduced" may mean that the activity of the protein per cell is reduced as compared with that of a non-modified strain. The term "the activity of a protein per cell" may mean an average value of the activity of the protein per cell. The non-modified strain is also referred to as "non-modified host" or "strain of non-modified host". The term "non-modified strain" referred to herein can refer to a control strain that has not been modified so that the activity of an objective protein is reduced. Examples of the non-modified strain can include a wild-type strain and parent strain. Specific examples of the non-modified strain can include the respective type strains of the species to which the host belongs. Specific examples of the non-modified strain can also include strains exemplified above in relation to the description of the host. That is, in an embodiment, the activity of a protein may be reduced as compared with a type strain, i.e. a type strain of the species to which the host belongs. In another embodiment, the activity of a protein may also be reduced as compared with the C. *glutamicum* ATCC 13869 strain. In another embodiment, the activity of a protein may also be reduced as compared with the C. *glutamicum* ATCC 13032 strain. In another embodiment, the activity of a protein may also be reduced as compared with the *E. coli* K-12 MG1655 strain. The state that "the activity of a protein is reduced" can also include a state that the activity of the protein has completely disappeared. More specifically, the expression "the activity of a protein is reduced" may mean that the number of molecules of the protein per cell is reduced, and/or the function of each molecule of the protein is reduced as compared with those of a non-modified strain. That is, the term "activity" in the expression "the activity of a protein is reduced" is not limited to the catalytic activity of the protein, but may also mean the transcription amount of a gene (i.e. the amount of mRNA) encoding the protein or the translation amount of the gene (i.e. the amount of the protein). The term "the number of molecules of a protein per cell" may mean an average value of the number of molecules of the protein per cell. The state that "the number of molecules of the protein per cell is reduced" can also include a state that the protein does not exist at all. The state that "the function of each molecule of the protein is reduced" can also include a state that the function of each protein molecule has completely disappeared. The degree of the reduction in the activity of a protein is not particularly limited, so long as the activity is reduced as compared with that of a non-modified strain. The activity of a protein may be reduced to, for example, 50% or less, 20% or less, 10% or less, 5% or less, or 0% of that of a non-modified strain.

The modification for reducing the activity of a protein can be attained by, for example, reducing the expression of a gene encoding the protein. The expression "the expression of a gene is reduced" can mean that the expression of the gene is reduced as compared with a non-modified strain such as a wild-type strain and parent strain. Specifically, the expression "the expression of a gene is reduced" may mean that the expression of the gene per cell is reduced as compared with that of a non-modified strain. The term "the expression amount of a gene per cell" may mean an average value of the expression amount of the gene per cell. More specifically, the expression "the expression of a gene is reduced" may mean that the transcription amount of the gene (i.e. the amount of mRNA) is reduced, and/or the translation amount of the gene (i.e. the amount of the protein expressed from the gene) is reduced. The state that "the expression of a gene is reduced" can also include a state that the gene is not expressed at all. The state that "the expression of a gene is reduced" can also be referred to as "the expression of a gene is attenuated". The expression of a gene may be reduced to, for example, 50% or less, 20% or less, 10% or less, 5% or less, or 0% of that of a non-modified strain.

The reduction in gene expression may be due to, for example, a reduction in the transcription efficiency, a reduction in the translation efficiency, or a combination of them. The expression of a gene can be reduced by modifying an expression control sequence of the gene such as a promoter, the Shine-Dalgarno (SD) sequence (also referred to as ribosome-binding site (RBS)), and a spacer region between RBS and the start codon of the gene. When an expression control sequence is modified, one or more nucleotides, two or more nucleotides, or three or more nucleotides, of the expression control sequence are modified. For example, the transcription efficiency of a gene can be reduced by, for example, replacing the promoter of the gene on a chromosome with a weaker promoter. The term "weaker promoter" can mean a promoter providing an attenuated transcription of a gene compared with an inherent wild-type promoter of the gene. Examples of weaker promoters can include, for example, inducible promoters. Examples of weaker promoters can also include, for example, P4 promoter (WO2018/079684) and P8 promoter (WO2018/079684). That is, an inducible promoter may function as a weaker promoter under a non-induced condition, such as in the absence of the corresponding inducer. Furthermore, a part of or the entire expression control sequence may be deleted. The expression of a gene can also be reduced by, for example, manipulating a factor responsible for expression control. Examples of the factor responsible for expression control can include low molecules responsible for transcription or translation control (inducers, inhibitors, etc.), proteins responsible for transcription or translation control (transcription factors etc.), nucleic acids responsible for transcription or translation control (siRNA etc.), and so forth. Furthermore, the expression of a gene can also be reduced by, for example, introducing a mutation that reduces the expression of the gene into the coding region of the gene. For example, the expression of a gene can be reduced by replacing a codon in the coding region of the gene with a synonymous codon used less frequently in a host. Furthermore, for example, the gene expression may be reduced due to disruption of a gene as described herein.

The modification for reducing the activity of a protein can also be attained by, for example, disrupting a gene encoding the protein. The expression "a gene is disrupted" can mean that a gene is modified so that a protein that can normally function is not produced. The state that "a protein that normally functions is not produced" can include a state that the protein is not produced at all from the gene, and a state that the protein of which the function (such as activity or property) per molecule is reduced or eliminated is produced from the gene.

Disruption of a gene can be attained by, for example, deleting the gene on a chromosome. The term "deletion of a gene" can refer to deletion of a partial or entire region of the coding region of the gene. Furthermore, the entire gene including sequences upstream and downstream from the coding region of the gene on a chromosome may be deleted. The region to be deleted may be any region such as an N-terminal region (i.e. a region encoding an N-terminal region of a protein), an internal region, or a C-terminal region (i.e. a region encoding a C-terminal region of a protein), so long as the activity of the protein can be reduced. Deletion of a longer region can usually more surely inactivate the gene. The region to be deleted may be, for example, a region having a length of 10% or more, 20% or more, 30% or more, 40% or more, 50% or more, 60% or more, 70% or more, 80% or more, 90% or more, or 95% or more of the total length of the coding region of the gene. Furthermore, it is preferred that reading frames of the sequences upstream and downstream from the region to be deleted are not the same. Inconsistency of reading frames may cause a frameshift downstream of the region to be deleted.

Disruption of a gene can also be attained by, for example, introducing a mutation for an amino acid substitution (missense mutation), a stop codon (nonsense mutation), addition or deletion of one or two nucleotide residues (frame shift mutation), or the like into the coding region of the gene on a chromosome (Journal of Biological Chemistry, 272:8611-8617 (1997); Proceedings of the National Academy of Sciences, USA, 95 5511-5515 (1998); Journal of Biological Chemistry, 26 116, 20833-20839 (1991)).

Disruption of a gene can also be attained by, for example, inserting another nucleotide sequence into a coding region of the gene on a chromosome. Site of the insertion may be in any region of the gene, and insertion of a longer nucleotide sequence can usually more surely inactivate the gene. It is preferred that reading frames of the sequences upstream and downstream from the insertion site are not the same. Inconsistency of reading frames may cause a frameshift downstream of the region to be deleted. The other nucleotide sequence is not particularly limited so long as a sequence that reduces or eliminates the activity of the encoded protein is chosen, and examples thereof can include, for example, a marker gene such as antibiotic resistance genes, and a gene useful for production of the objective substance.

Particularly, disruption of a gene may be carried out so that the amino acid sequence of the encoded protein is deleted. In other words, the modification for reducing the activity of a protein can be attained by, for example, deleting the amino acid sequence of the protein, specifically, modifying a gene so as to encode a protein of which the amino acid sequence is deleted. The term "deletion of the amino acid sequence of a protein" can refer to deletion of a partial or entire region of the amino acid sequence of the protein. In addition, the term "deletion of the amino acid sequence of a protein" means that the original amino acid sequence disappears in the protein, and can also include cases where the original amino acid sequence is changed to another amino acid sequence. That is, for example, a region that was changed to another amino acid sequence by frameshift may be regarded as a deleted region. When the amino acid sequence of a protein is deleted, the total length of the protein is typically shortened, but there can also be cases where the total length of the protein is not changed or is extended. For example, by deletion of a partial or entire region of the coding region of a gene, a region encoded by the deleted region can be deleted in the encoded protein. In addition, for example, by introduction of a stop codon into the coding region of a gene, a region encoded by the downstream region of the introduction site can be deleted in the encoded protein. In addition, for example, by frameshift in the coding region of a gene, a region encoded by the frameshift region can be deleted in the encoded protein. The aforementioned descriptions concerning the position and length of the region to be deleted in deletion of a gene can be similarly applied to the position and length of the region to be deleted in deletion of the amino acid sequence of a protein.

Such modification of a gene on a chromosome as described above can be attained by, for example, preparing a disruption-type gene modified so that it is unable to produce a protein that functions normally, and transforming a host with a recombinant DNA containing the disruption-type gene to cause homologous recombination between the disruption-type gene and the wild-type gene on a chromosome and thereby substitute the disruption-type gene for the wild-type gene on the chromosome. In this procedure, if a marker gene selected according to the characteristics of the host such as auxotrophy is included in the recombinant DNA, the operation becomes easier. Examples of the disruption-type gene can include a gene of which a partial or entire region of the coding region is deleted, gene including a missense mutation, gene including a nonsense mutation, gene including a frame shift mutation, and gene including insertion of a transposon or marker gene. The protein encoded by the disruption-type gene has a conformation different from that of the wild-type protein, even if it is produced, and thus the function thereof is reduced or eliminated. Such gene disruption based on gene substitution utilizing homologous recombination has already been established, and there are methods of using a linear DNA such as a method called "Red driven integration" (Datsenko, K.A, and Wanner, B.L., Proc. Natl. Acad. Sci. USA, 97:6640-6645 (2000)), and a method utilizing the Red driven integration in combination with an excision system derived from λ phage (Cho, E.H., Gumport, R.I., Gardner, J.F., J. Bacteriol., 184:5200-5203 (2002)) (refer to WO2005/010175), a method of using a plasmid having a temperature sensitive replication origin, a method of using a plasmid capable of conjugative transfer, a method of utilizing a suicide vector not having a replication origin that functions in a host (U.S. Patent No. 6,303,383, Japanese Patent Laid-open (Kokai) No. 05-007491), and so forth.

The modification for reducing activity of a protein can also be attained by, for example, a mutagenesis treatment. Examples of the mutagenesis treatment can include irradiation of X-ray or ultraviolet and treatment with a mutation agent such as N-methyl-N'-nitro-N-nitrosoguanidine (MNNG), ethyl methanesulfonate (EMS), and methyl methanesulfonate (MMS).

When a protein functions as a complex consisting of a plurality of subunits, a part or all of the plurality of subunits may be modified, so long as the activity of the protein is eventually reduced. That is, for example, a part or all of genes that encode the respective subunits may be disrupted or the like. Furthermore, when there is a plurality of isozymes of a protein, a part or all of the activities of the plurality of isozymes may be reduced, so long as the activity of the protein is eventually reduced. That is, for example, a part or all of genes that encode the respective isozymes may be disrupted or the like.

Such methods for reducing the activity of a protein as mentioned above may be used independently or in any combination.

A reduction in the activity of a protein can be confirmed by measuring the activity of the protein.

A reduction in the activity of a protein can also be confirmed by confirming a reduction in the expression of a gene encoding the protein. A reduction in the expression of a gene can be confirmed by confirming a reduction in the transcription amount of the gene or a reduction in the amount of the protein expressed from the gene.

A reduction in the transcription amount of a gene can be confirmed by comparing the amount of mRNA transcribed from the gene with that observed in a non-modified strain. Examples of the method for evaluating the amount of mRNA can include Northern hybridization, RT-PCR, microarray, RNA-seq, and so forth (Molecular Cloning, Cold Spring Harbor Laboratory Press, Cold Spring Harbor (USA), 2001). The amount of mRNA can be reduced to, for example, 50% or less, 20% or less, 10% or less, 5% or less, or 0%, of that observed in a non-modified strain.

A reduction in the amount of a protein can be confirmed by Western blotting using antibodies (Molecular Cloning, Cold Spring Harbor Laboratory Press, Cold Spring Harbor (USA) 2001). The amount of the protein (such as the number of molecules of the protein per cell) can be reduced to, for example, 50% or less, 20% or less, 10% or less, 5% or less, or 0%, of that observed in a non-modified strain.

Disruption of a gene can be confirmed by determining nucleotide sequence of a part or the whole of the gene, restriction enzyme map, full length, or the like of the gene depending on the means used for the disruption.

Such methods for reducing the activity of a protein as mentioned above can be applied to reduction in the activities of any proteins and reduction in the expression of any genes.

<3-5> Cultivation of host

By culturing the host having the objective enzyme gene, the objective enzyme can be expressed.

The culture medium to be used is not particularly limited, so long as the host can proliferate in it and express the functional objective enzyme. As the culture medium, for example, a usual culture medium used for culture of microorganisms such as bacteria and yeast can be used. The culture medium may contain carbon source, nitrogen source, phosphate source, and sulfur source, as well as other medium components such as various organic components and inorganic components as required. The types and concentrations of the medium components can be appropriately set according to various conditions such as the type of the host to be used.

Specific examples of the carbon source include, for example, saccharides such as glucose, fructose, sucrose, lactose, galactose, xylose, arabinose, blackstrap molasses, hydrolysates of starches, and hydrolysates of biomass; organic acids such as acetic acid, citric acid, succinic acid, and gluconic acid; alcohols such as ethanol, glycerol, and crude glycerol; and fatty acids. As the carbon source, plant-derived materials can be preferably used. Examples of the plant include, for example, corn, rice, wheat, soybean, sugarcane, beet, and cotton. Examples of the plant-derived materials include, for example, organs such as root, stem, trunk, branch, leaf, flower, and seed, plant bodies including them, and decomposition products of these plant organs. The forms of the plant-derived materials at the time of use thereof are not particularly limited, and they can be used in any form such as unprocessed product, juice, ground product, and purified product. Pentoses such as xylose, hexoses such as glucose, or mixtures of them can be obtained from, for example, plant biomass, and used. Specifically, these saccharides can be obtained by subjecting a plant biomass to such a treatment as steam treatment, hydrolysis with concentrated acid, hydrolysis with diluted acid, hydrolysis with an enzyme such as cellulase, and alkaline treatment. Since hemicellulose is generally more easily hydrolyzed compared with cellulose, hemicellulose in a plant biomass may be hydrolyzed beforehand to liberate pentoses, and then cellulose may be hydrolyzed to generate hexoses. Further, xylose may be supplied by conversion from hexoses by, for example, imparting a pathway for converting hexose such as glucose to xylose to the host. As the carbon source, one kind of carbon source may be used, or two or more kinds of carbon sources may be used in combination.

The concentration of the carbon source in the medium is not particularly limited, so long as the host can proliferate in it and express the functional objective enzyme. The concentration of the carbon source in the medium may be as high as possible within such a range that production of the objective enzyme is not inhibited. Initial concentration of the carbon source in the medium may be, for example, usually 5 to 30% (w/v), preferably 10 to 20% (w/v). Further, the carbon source may be additionally supplied to the medium as required. For example, the carbon source may be additionally supplied to the medium in proportion to decrease or depletion of the carbon source accompanying progress of the cultivation. While the carbon source may be temporarily depleted so long as the objective enzyme can be eventually produced, it may be preferable to perform the culture so that the carbon source is not depleted or the carbon source does not continue to be depleted.

Specific examples of the nitrogen source include, for example, ammonium salts such as ammonium sulfate, ammonium chloride, and ammonium phosphate, organic nitrogen sources such as peptone, yeast extract, meat extract, and soybean protein decomposition products, ammonia, and urea. Ammonia gas and aqueous ammonia used for pH adjustment may also be used as a nitrogen source. As the nitrogen source, one kind of nitrogen source may be used, or two or more kinds of nitrogen sources may be used in combination.

Specific examples of the phosphate source include, for example, phosphate salts such as potassium dihydrogenphosphate and dipotassium hydrogenphosphate, and phosphoric acid polymers such as pyrophosphoric acid. As the phosphate source, one kind of phosphate source may be used, or two or more kinds of phosphate sources may be used in combination.

Specific examples of the sulfur source include, for example, inorganic sulfur compounds such as sulfates, thiosulfates, and sulfites, and sulfur-containing amino acids such as cysteine, cystine, and glutathione. As the sulfur source, one kind of sulfur source may be used, or two or more kinds of sulfur sources may be used in combination.

Specific examples of other various organic and inorganic components include, for example, inorganic salts such as sodium chloride and potassium chloride; trace metals such as iron, manganese, magnesium, and calcium; vitamins such as vitamin B1, vitamin B2, vitamin B6, nicotinic acid, nicotinamide, and vitamin B12; amino acids; nucleic acids; and organic components containing these such as peptone, casamino acid, yeast extract, and soybean protein decomposition product. As the other various organic and inorganic components, one kind of component may be used, or two or more kinds of components may be used in combination.

Furthermore, when an auxotrophic mutant strain that requires a nutrient such as amino acids for growth thereof is used, it is preferable to supplement such a required nutrient to the culture medium.

Culture conditions are not particularly limited, so long as the host can proliferate in it and express the functional objective enzyme. The culture can be performed with, for example, usual conditions used for culture of microorganisms such as bacteria and yeast. The culture conditions may be appropriately set according to various conditions such as the type of the host to be used. In addition, expression of the objective enzyme gene may be induced, as required.

The culture can be performed by using a liquid medium. At the time of the culture, for example, the host cultured on a solid medium such as agar medium may be directly inoculated into a liquid medium, or the host cultured in a liquid medium as seed culture may be inoculated into a liquid medium for main culture. That is, the culture may be performed separately as seed culture and main culture. In such a case, the culture conditions of the seed culture and the main culture may be or may not be the same. It is sufficient that the objective enzyme gene is expressed at least during the main culture. The amount of the host contained in the culture medium at the start of the culture is not particularly limited. For example, a seed culture broth showing an OD660 of 4 to 100 may be added to a culture medium for main culture in an amount of 0.1 to 100 mass %, preferably 1 to 50 mass %, at the start of the culture.

The culture can be performed as batch culture, fed-batch culture, continuous culture, or a combination of these. The culture medium used at the start of the culture is also referred to as "starting medium". The culture medium supplied to the culture system (e.g. fermentation tank) in the fed-batch culture or the continuous culture is also referred to as "feed medium". To supply a feed medium to the culture system in the fed-batch culture or the continuous culture is also referred to as "feed". Furthermore, when the culture is performed separately as seed culture and main culture, the culture schemes of the seed culture and the main culture may be or may not be the same. For example, both the seed culture and the main culture may be performed as batch culture. Alternatively, for example, the seed culture may be performed as batch culture, and the main culture may be performed as fed-batch culture or continuous culture.

In the present invention, the various components such as the carbon source may be contained in the starting medium, feed medium, or both. That is, the various components such as the carbon source may be additionally supplied to the culture medium independently or in any combination during the culture. These components may be supplied once or a plurality of times, or may be continuously supplied. The types of the components contained in the starting medium may be or may not be the same as those of the components contained in the feed medium. Furthermore, the concentrations of the components contained in the starting medium may be or may not be the same as the concentrations of the components contained in the feed medium. Furthermore, two or more kinds of feed media containing components of different types and/or different concentrations may be used. For example, when feeding is intermittently performed two or more times, the types and/or concentrations of components contained in the feed medium may be or may not be the same for each feeding.

The culture can be performed, for example, under an aerobic condition. The term "aerobic condition" may refer to a condition where the dissolved oxygen concentration in the culture medium is 0.33 ppm or higher, or preferably 1.5 ppm or higher. The oxygen concentration can be controlled to be, for example, 1 to 50%, preferably about 5%, of the saturated oxygen concentration. The culture can be performed, for example, with aeration or shaking. The pH of the culture medium may be, for example, 3 to 10, preferably 4.0 to 9.5. The pH of the culture medium can be adjusted during the culture as required. The pH of the culture medium can be adjusted by using various alkaline and acidic substances such as ammonia gas, aqueous ammonia, sodium carbonate, sodium bicarbonate, potassium carbonate, potassium bicarbonate, magnesium carbonate, sodium hydroxide, potassium hydroxide, calcium hydroxide, and magnesium hydroxide. The culture temperature may be, for example, 20 to 45°C, preferably 25 to 37°C. The culture time may be, for example, 10 to 120 hours. The culture may be continued, for example, until the carbon source contained in the culture medium is consumed, or until the activity of the host is lost.

By culturing the host as described above, a culture broth containing the objective enzyme is obtained. The objective enzyme can be accumulated in, for example, microbial cells of the host. The term "microbial cell" may be appropriately read as "cell" depending on the type of the host. Depending on the host to be used and/or design of the objective enzyme gene, it can also be possible to accumulate the objective enzyme in the periplasm, or to produce the objective enzyme outside cells by secretory production.

The objective enzyme may be used for the method of the present invention as it is contained in the culture broth (specifically, the culture medium or cells), or after being purified from the culture broth (specifically, the culture medium or cells). The purification can be carried out to a desired extent. That is, examples of the objective enzyme include a purified objective enzyme and a fraction containing the objective enzyme. In other words, the objective enzyme may be used in the form of a purified enzyme, may be used in the form of such a fraction (i.e. in the form contained in such a fraction), or may be used in the form of a combination thereof. Such a fraction is not particularly limited, so long as it contains the objective enzyme in such a manner that the objective enzyme can act on the substrate thereof. Examples of such a fraction include a culture broth of the host having the objective enzyme gene (i.e. the host having the objective enzyme), cells collected from the culture broth, a supernatant collected from the culture broth, a processed product thereof (e.g. a processed product of cells such as cell disrupt, cell lysate, cell extract, and others described below), and combinations thereof. The term "purified objective enzyme" may include a roughly-purified product. These fractions may be independently used, or may be used in an appropriate combination.

The objective enzyme may be used, in particular, in the form contained in cells. The cells may be used for the method of the present invention (e.g. the conversion reaction) as they are contained in the culture broth (specifically, the culture medium), or after being collected from the culture broth (specifically, the culture medium). The cells may also be used for the method of the present invention (e.g. the conversion reaction) after being subjected to a treatment as required. That is, examples of the cells include a culture broth of the host having the objective enzyme gene (i.e. the host having the objective enzyme), cells collected from the culture broth, or a processed product thereof. In other words, the cells may be used in the form of a culture broth of the host having the objective enzyme gene (i.e. the host having the objective enzyme), cells collected from the culture broth, a processed product thereof, or a combination thereof. Examples of the processed product include products obtained by subjecting the cells (such as cells contained in a culture broth, or cells collected from the culture broth) to a treatment. The cells in these forms may be independently used, or may be used in an appropriate combination.

The method for collecting the cells from the culture broth is not particularly limited, and for example, known methods can be used. Examples of such methods include, for example, spontaneous precipitation, centrifugation, and filtration. A flocculant may also be used. These methods may be independently used, or may be used in an appropriate combination. The collected cells can be washed as required by using an appropriate medium. The collected cells can be re-suspended as required by using an appropriate medium. Examples of the medium usable for washing or suspending the cells include, for example, aqueous media (aqueous solvents) such as water and aqueous buffer.

Examples of the treatment of the cells include, for example, immobilization on a carrier such as acrylamide and carrageenan, freezing and thawing treatment, and treatment for increasing permeability of cell membranes. Permeability of cell membranes can be increased by, for example, using a surfactant or organic solvent. These treatments may be independently used, or may be used in an appropriate combination.

The objective enzyme may be used for the method of the present invention after being diluted, condensed, or the like as required.

Each of the objective enzymes may be produced independently, or two or more of the objective enzymes may be produced in combination. For example, by allowing the host having two or more objective enzyme genes to express the genes, two or more objective enzymes may be produced in combination.

Furthermore, by culturing the host having the objective enzyme gene, the host may be used for the method of the present invention as the objective enzyme.

### <4> Method of the present invention

The method of the present invention is a method for producing benzaldehyde by using the benzaldehyde generation enzymes and catalase. In other words, the method of the present invention is a method for producing benzaldehyde comprising a step of producing benzaldehyde by using the benzaldehyde generation enzymes, wherein at least a part of the step is carried out in the presence of catalase. This step is also referred to as "production step".

The benzaldehyde generation enzymes may each be used in any form such as those described above. That is, examples of the use of the benzaldehyde generation enzymes include use of them in any form such as those described above. The use form of the benzaldehyde generation enzymes can be set independently for each of the benzaldehyde generation enzymes. The benzaldehyde generation enzymes each may be used, for example, in the form contained in host cells such as microbial cells. Also, the benzaldehyde generation enzymes each may be used, for example, in the form not contained in host cells, e.g. in the form of a purified enzyme. Also, for example, a part of the benzaldehyde generation enzymes may be used in the form contained in host cells such as microbial cells, and the remaining part of the benzaldehyde generation enzymes may be used in the form not contained in host cells, e.g. in the form of a purified enzyme. The benzaldehyde generation enzymes may be used, in particular, in the form of the microorganism of the present invention. That is, particular examples of the use of the benzaldehyde generation enzymes include use of the microorganism of the present invention. Examples of the use of the microorganism of the present invention include cultivation of the microorganism of the present invention and use of cells of the microorganism of the present invention. That is, for example, the microorganism of the present invention may be used as the benzaldehyde generation enzymes by being cultured. Also, for example, cells of the microorganism of the present invention may be used as the benzaldehyde generation enzymes.

Benzaldehyde can be produced by, for example, fermentation, substance conversion, or a combination thereof. That is, the production step may be carried out by, for example, fermentation, substance conversion, or a combination thereof. The embodiment for carrying out the production step can be appropriately set according to various conditions such as the use form of the benzaldehyde generation enzymes. Specifically, the production step may be carried out by, for example, cultivating the microorganism of the present invention, using cells of the microorganism of the present invention, or a combination thereof. The combination may specifically be a combination of cultivating a part of a plurality of microorganisms constituting the microorganism of the present invention and using cells of the remaining part of the plurality of microorganisms. Benzaldehyde can be produced from, for example, a carbon source or L-phenylalanine.

### <4-1> Fermentation method

Benzaldehyde can be produced by, for example, fermentation using the microorganism of the present invention having L-phenylalanine-producing ability. That is, an embodiment of the method of the present invention may be a method for producing benzaldehyde by fermentation using the microorganism of the present invention having L-phenylalanine-producing ability. This embodiment is also referred to as "fermentation method". Also, the step of producing benzaldehyde by fermentation using the microorganism of the present invention having L-phenylalanine-producing ability is also referred to as "fermentation step".

The fermentation step can be performed by cultivating the microorganism of the present invention. Specifically, in the fermentation method, benzaldehyde can be produced from a carbon source. That is, the fermentation step may be, for example, a step of cultivating the microorganism of the present invention in a culture medium, such as a culture medium containing a carbon source, to produce and accumulate benzaldehyde in the culture medium. That is, the fermentation method may be a method for producing benzaldehyde comprising cultivating the microorganism of the present invention in a culture medium, such as a culture medium containing a carbon source, to produce and accumulate benzaldehyde in the culture medium. Also, in other words, the fermentation step may be, for example, a step of producing benzaldehyde from a carbon source by using the microorganism of the present invention.

In cases where the microorganism of the present invention is a combination of a plurality of microorganisms, the plurality of microorganisms may be or may not be cultivated simultaneously. For example, the plurality of microorganisms may be inoculated simultaneously and cultivated, or may be inoculated at different timings individually or in any combination and cultivated. The order and timings for cultivating such a plurality of microorganisms are not particularly limited, so long as production of benzaldehyde from the carbon source is attained. For example, a microorganism having AAD, a microorganism having HMAS, a microorganism having SMDH, and a microorganism having BFDC may be inoculated in this order. The phrase "cultivating the microorganism of the present invention in a culture medium containing a carbon source" used for cases where the microorganism of the present invention is a combination of a plurality of microorganisms means that at least one microorganism selected from the plurality of microorganisms is cultured in a culture medium containing the carbon source so that production of benzaldehyde from the carbon source is attained, and does not necessarily mean that all the plurality of microorganisms are cultured in a culture medium containing the carbon source. That is, the phrase "cultivating the microorganism of the present invention in a culture medium containing a carbon source" used for cases where the microorganism of the present invention is a combination of a plurality of microorganisms may mean that, for example, at least a microorganism having L-phenylalanine-producing ability, which may be a microorganism having AAD, is cultured in a culture medium containing the carbon source. That is, for example, after cultivation of a microorganism having AAD in a culture medium containing the carbon source to thereby completely consume the carbon source to generate such an intermediate as described later, cultivation of other microorganism(s) may be initiated. For cultivation of microorganism(s) after consumption of the carbon source, any additional carbon source that may be or may not be used as a raw material for production of benzaldehyde can be used as required.

The culture medium to be used is not particularly limited, so long as the microorganism of the present invention can proliferate in it and benzaldehyde is produced. Culture conditions are not particularly limited, so long as the microorganism of the present invention can proliferate, and benzaldehyde is produced. The culture medium may contain components useful for the objective enzymes to function. Examples of such components include ascorbic acid and oxygen. Ascorbic acid and oxygen can be, for example, components useful for HMAS to function. That is, at least in the part using HMAS, cultivation may be carried out in the presence of ascorbic acid and oxygen. As for carrying out cultivation in the presence of ascorbic acid and oxygen in the part using HMAS, the descriptions below concerning carrying out the part using HMAS in the presence of catalase can be similarly applied. Oxygen may be supplied to the culture medium by carrying out cultivation under an atmosphere containing oxygen such as air. Components that can form a salt may each be used as a free compound, a salt thereof, or a mixture thereof. That is, the term "component" used for a component that can form a salt in the present invention refers to the component in a free form, a salt thereof, or a mixture thereof, unless otherwise stated. As for the salt of a component that can form a salt, the descriptions concerning the salt of L-phenylalanine can be similarly applied. For example, ascorbic acid may be used as a free compound, a salt thereof, or a mixture thereof. That is, the term " ascorbic acid" used in the present invention refers to ascorbic acid in a free form, a salt thereof, or a mixture thereof, unless otherwise stated. Examples of the salt include, for example, ammonium salt, sodium salt, and potassium salt. As the salt, one kind of salt may be employed, or two or more kinds of salts may be employed in combination. The aforementioned descriptions concerning the cultivation for culturing the host (e.g. the descriptions concerning the culture medium and culture conditions) can be similarly applied to the cultivation in the fermentation method, except that benzaldehyde is produced in the fermentation method.

By cultivating the microorganism of the present invention as described above, a culture broth containing benzaldehyde is obtained.

Production of benzaldehyde can be confirmed by known methods used for detection or identification of compounds. Examples of such methods include, for example, HPLC, UPLC, LC/MS, GC/MS, and NMR. These methods may be independently used, or may be used in an appropriate combination. These methods can also be used for determining the concentrations of various components present in the culture medium.

The produced benzaldehyde can be appropriately collected. That is, the method of the present invention may further comprise a step of collecting benzaldehyde. This step is also referred to as "collection step". The collection step may be a step of collecting benzaldehyde from the culture broth, specifically from the culture medium. The produced benzaldehyde can be collected by known methods used for separation and purification of compounds. Examples of such methods include, for example, ionexchange resin method, membrane treatment, precipitation, extraction, distillation, and crystallization. Benzaldehyde can be collected specifically by extraction with an organic solvent such as ethyl acetate or by steam distillation. These methods may be independently used, or may be used in an appropriate combination.

Furthermore, when benzaldehyde deposits in the culture medium, it can be collected by, for example, centrifugation or filtration. Benzaldehyde deposited in the culture medium and benzaldehyde dissolving in the culture medium may also be isolated together after benzaldehyde dissolving in the culture medium is crystallized.

The collected benzaldehyde may contain, for example, microbial cells, medium components, moisture, and by-product metabolites of the microorganism, in addition to benzaldehyde. The purity of the collected benzaldehyde may be, for example, 30% (w/w) or higher, 50% (w/w) or higher, 70% (w/w) or higher, 80% (w/w) or higher, 90% (w/w) or higher, or 95% (w/w) or higher.

### <4-2> Substance conversion method

Benzaldehyde can also be produced by, for example, substance conversion using the benzaldehyde generation enzymes (e.g. the microorganism of the present invention). That is, another embodiment of the method of the present invention may be a method for producing benzaldehyde by substance conversion using the benzaldehyde generation enzymes (e.g. the microorganism of the present invention). This embodiment is also referred to as "substance conversion method". Also, the step of producing benzaldehyde by substance conversion using the benzaldehyde generation enzymes (e.g. the microorganism of the present invention) is also referred to as "substance conversion step".

Specifically, in the substance conversion method, benzaldehyde can be produced from L-phenylalanine. More specifically, in the substance conversion method, benzaldehyde can be produced by converting L-phenylalanine into benzaldehyde by using the benzaldehyde generation enzymes (e.g. the microorganism of the present invention). That is, the substance conversion step may be a step of converting L-phenylalanine into benzaldehyde by using the benzaldehyde generation enzymes (e.g. the microorganism of the present invention). In the substance conversion step, L-phenylalanine, which is the substance before conversion, is also referred to as "substrate", and benzaldehyde, which is the substance after conversion, is also referred to as "product".

L-phenylalanine may be used as a free compound, a salt thereof, or a mixture thereof. That is, the term "L-phenylalanine" used in the present invention refers to L-phenylalanine in a free form, a salt thereof, or a mixture thereof, unless otherwise stated. Examples of the salt include, for example, sulfate salt, hydrochloride salt, carbonate salt, ammonium salt, sodium salt, and potassium salt. As the salt, one kind of salt may be employed, or two or more kinds of salts may be employed in combination.

As L-phenylalanine, a commercially available product may be used, or one appropriately prepared and obtained may be used. The method for producing L-phenylalanine is not particularly limited, and for example, known methods can be used. L-phenylalanine can be produced by, for example, a chemical synthesis method, enzymatic method, substance conversion method, fermentation method, extraction method, or a combination of these. That is, L-phenylalanine can be produced by, for example, cultivating a microorganism having L-phenylalanine-producing ability (L-phenylalanine-producing microorganism), and collecting L-phenylalanine from the culture broth. The produced L-phenylalanine can be used for the method of the present invention as it is, or after being subjected to an appropriate treatment such as concentration, dilution, drying, dissolution, fractionation, extraction, and purification, as required. That is, as L-phenylalanine, for example, a purified product purified to a desired extent may be used, or a material containing L-phenylalanine may be used.

The material containing L-phenylalanine is not particularly limited so long as the AAD generation enzyme (e.g. a microorganism having AAD such as the microorganism of the present invention) can use L-phenylalanine. Specific examples of the material containing L-phenylalanine include culture broth obtained by cultivating a L-phenylalanine-producing microorganism, culture supernatant separated from the culture broth, and processed products thereof such as concentrated products (such as concentrated liquid) thereof and dried products thereof.

In an embodiment, the substance conversion step can be performed by, for example, cultivating the microorganism of the present invention. This embodiment is also referred to as "first embodiment of the substance conversion method". That is, the substance conversion step may be, for example, a step of cultivating the microorganism of the present invention in a culture medium containing L-phenylalanine to convert L-phenylalanine into benzaldehyde. The substance conversion step may be, specifically, a step of cultivating the microorganism of the present invention in a culture medium containing L-phenylalanine to generate and accumulate benzaldehyde in the culture medium.

In cases where the microorganism of the present invention is a combination of a plurality of microorganisms, the plurality of microorganisms may be or may not be cultivated simultaneously. For example, the plurality of microorganisms may be inoculated simultaneously and cultivated, or may be inoculated at different timings individually or in any combination and cultivated. The order and timings for cultivating such a plurality of microorganisms are not particularly limited, so long as conversion of L-phenylalanine into benzaldehyde is attained. For example, a microorganism having AAD, a microorganism having HMAS, a microorganism having SMDH, and a microorganism having BFDC may be inoculated in this order. The phrase "cultivating the microorganism of the present invention in a culture medium containing L-phenylalanine" used for cases where the microorganism of the present invention is a combination of a plurality of microorganisms means that at least one microorganism selected from the plurality of microorganisms is cultured in a culture medium containing L-phenylalanine so that conversion of L-phenylalanine into benzaldehyde is attained, and does not necessarily mean that all the plurality of microorganisms are cultured in a culture medium containing L-phenylalanine. That is, the phrase "cultivating the microorganism of the present invention in a culture medium containing L-phenylalanine" used for cases where the microorganism of the present invention is a combination of a plurality of microorganisms may mean that, for example, at least a microorganism having AAD is cultured in a culture medium containing L-phenylalanine. That is, for example, after cultivation of a microorganism having AAD in a culture medium containing L-phenylalanine to thereby completely convert L-phenylalanine into such an intermediate as described later, cultivation of other microorganism(s) may be initiated.

The culture medium to be used is not particularly limited, so long as the culture medium contains L-phenylalanine, the microorganism of the present invention can proliferate in it, and benzaldehyde is produced. Culture conditions are not particularly limited, so long as the microorganism of the present invention can proliferate, and benzaldehyde is produced. The culture medium may contain components useful for the objective enzymes to function. Examples of such components include ascorbic acid and oxygen. Ascorbic acid and oxygen can be, for example, components useful for HMAS to function. That is, at least in the part using HMAS, cultivation may be carried out in the presence of ascorbic acid and oxygen. As for carrying out cultivation in the presence of ascorbic acid and oxygen in the part using HMAS, the descriptions below concerning carrying out the part using HMAS in the presence of catalase can be similarly applied. The aforementioned descriptions concerning the cultivation for culturing the host (e.g. the descriptions concerning the culture medium and culture conditions) can be similarly applied to the cultivation in the first embodiment of the substance conversion method, except that the culture medium contains L-phenylalanine and benzaldehyde is produced in the first embodiment of the substance conversion method.

L-phenylalanine may be contained in the culture medium over the whole period of the culture, or may be contained in the culture medium during only a partial period of the culture. That is, the phrase "cultivating a microorganism in a culture medium containing L-phenylalanine" does not necessarily mean that L-phenylalanine is contained in the culture medium over the whole period of the culture. For example, L-phenylalanine may be or may not be contained in the culture medium from the start of the culture. When L-phenylalanine is not contained in the culture medium at the start of the culture, L-phenylalanine is supplied to the culture medium after the start of the culture. Timing of the supply can be appropriately set according to various conditions such as the length of the culture period. For example, L-phenylalanine may be supplied to the culture medium after the microorganism of the present invention sufficiently grows. Furthermore, in any case, L-phenylalanine may be additionally supplied to the culture medium as required. For example, L-phenylalanine may be additionally supplied to the culture medium in proportion to decrease or depletion of L-phenylalanine accompanying generation of benzaldehyde. Means for supplying L-phenylalanine to the culture medium is not particularly limited. For example, L-phenylalanine can be supplied to the culture medium by feeding a feed medium containing L-phenylalanine to the culture medium. Furthermore, for example, the microorganism of the present invention and an L-phenylalanine-producing microorganism can be co-cultured to allow the L-phenylalanine-producing microorganism to produce L-phenylalanine in the culture medium, and thereby supply L-phenylalanine to the culture medium. These supply means may be independently used, or may be used in an appropriate combination. The concentration of L-phenylalanine in the culture medium is not particularly limited so long as the microorganism of the present invention can use L-phenylalanine as a raw material of benzaldehyde. The concentration of L-phenylalanine in the culture medium, for example, may be 1 mM or higher, 10 mM or higher, or 30 mM or higher, or may be 5 M or lower, 2 M or lower, or 1 M or lower, or may be within a range defined with a combination thereof. L-phenylalanine may be or may not be contained in the culture medium at a concentration within the range exemplified above over the whole period of the culture. For example, L-phenylalanine may be contained in the culture medium at a concentration within the range exemplified above at the start of the culture, or it may be supplied to the culture medium so that a concentration within the range exemplified above is attained after the start of the culture. In cases where the culture is performed separately as seed culture and main culture, it is sufficient that benzaldehyde is produced at least during the main culture. Hence, it is sufficient that L-phenylalanine is contained in the culture medium at least during the main culture, i.e. over the whole period of the main culture or during a partial period of the main culture, and that is, L-phenylalanine may be or may not be contained in the culture medium during the seed culture. In such cases, terms regarding the culture, such as "culture period (period of culture)" and "start of culture", can be read as those regarding the main culture.

In another embodiment, the substance conversion step can also be performed by, for example, using the benzaldehyde generation enzymes (e.g. cells of the microorganism of the present invention). This embodiment is also referred to as "second embodiment of the substance conversion method". That is, the substance conversion step may be, for example, a step of converting L-phenylalanine in a reaction mixture into benzaldehyde by using the benzaldehyde generation enzymes (e.g. cells of the microorganism of the present invention). The substance conversion step may be, specifically, a step of allowing the benzaldehyde generation enzymes (e.g. cells of the microorganism of the present invention) to coexist with L-phenylalanine in a reaction mixture to generate and accumulate benzaldehyde in the reaction mixture. The substance conversion step may be, more specifically, a step of allowing the benzaldehyde generation enzymes (e.g. cells of the microorganism of the present invention) to act on L-phenylalanine in a reaction mixture to generate and accumulate benzaldehyde in the reaction mixture. The substance conversion step in the second embodiment of the substance conversion method (i.e. a step of converting L-phenylalanine in a reaction mixture into benzaldehyde) is also referred to as "conversion reaction". The conversion reaction may be carried out, in particular, by using the cells of the microorganism of the present invention.

The benzaldehyde generation enzymes may be or may not be supplied to the reaction mixture simultaneously. For example, in cases where the microorganism of the present invention is a combination of a plurality of microorganisms, cells of the plurality of microorganisms may be or may not be supplied to the reaction mixture simultaneously. For example, cells of the plurality of microorganisms may be supplied to the reaction mixture at different timings individually or in any combination. The order and timings for supplying cells of the plurality of microorganisms to the reaction mixture are not particularly limited, so long as conversion of L-phenylalanine into benzaldehyde is attained. For example, AAD, HMAS, SMDH, and BFDC (e.g. cells of a plurality of respective microorganisms having AAD, HMAS, SMDH, and BFDC) may be supplied to the reaction mixture in this order. The phrase "allowing the benzaldehyde generation enzymes to coexist with or act on L-phenylalanine in a reaction mixture" means that at least one enzyme selected from the benzaldehyde generation enzymes is allowed to coexist with or acts on L-phenylalanine in a reaction mixture so that conversion of L-phenylalanine into benzaldehyde is attained, and does not necessarily mean that all of the benzaldehyde generation enzymes are allowed to coexist with or act on L-phenylalanine in a reaction mixture. That is, the phrase "allowing the benzaldehyde generation enzymes to coexist with or act on L-phenylalanine in a reaction mixture" may mean that, for example, at least AAD is allowed to coexist with or act on L-phenylalanine in a reaction mixture. For example, the phrase "allowing cells of the microorganism of the present invention to coexist with or act on L-phenylalanine in a reaction mixture" used for cases where the microorganism of the present invention is a combination of a plurality of microorganisms means that cells of at least one microorganism selected from the plurality of microorganisms are allowed to coexist with or act on L-phenylalanine in a reaction mixture so that conversion of L-phenylalanine into benzaldehyde is attained, and does not necessarily mean that cells of all of the plurality of microorganisms are allowed to coexist with or act on L-phenylalanine in a reaction mixture. That is, the phrase "allowing cells of the microorganism of the present invention to coexist with or act on L-phenylalanine in a reaction mixture" used for cases where the microorganism of the present invention is a combination of a plurality of microorganisms may mean that, for example, cells of at least a microorganism having AAD are allowed to coexist with or act on L-phenylalanine in a reaction mixture. That is, for example, after allowing AAD (e.g. cells of a microorganism having AAD) to coexist with or act on L-phenylalanine in a reaction mixture to thereby completely convert L-phenylalanine into such an intermediate as described later, the other benzaldehyde generation enzymes (e.g. cells of the other microorganism(s)) may be supplied to the reaction mixture.

The cells used for the conversion reaction are not particularly limited so long as the cells having the benzaldehyde-producing ability. The cells may have or may not have proliferation ability.

The conversion reaction can be carried out in an appropriate reaction mixture. Specifically, the conversion reaction can be carried out by allowing the benzaldehyde generation enzymes (e.g. the cells of the microorganism of the present invention) and L-phenylalanine to coexist in an appropriate reaction mixture. The conversion reaction may be carried out by the batch method or may be carried out by the column method. In the case of the batch method, the conversion reaction can be carried out by, for example, mixing the benzaldehyde generation enzymes (e.g. the cells of the microorganism of the present invention) and L-phenylalanine in a reaction mixture contained in a reaction vessel. The conversion reaction may be carried out statically, or may be carried out with stirring or shaking the reaction mixture. In the case of the column method, the conversion reaction can be carried out by, for example, passing a reaction mixture containing L-phenylalanine through a column filled with an immobilized enzyme (e.g. immobilized cells). Examples of the reaction mixture include those based on an aqueous medium (aqueous solvent) such as water and aqueous buffer.

The reaction mixture may contain components other than L-phenylalanine as required, in addition to L-phenylalanine. Examples of the components other than L-phenylalanine include oxygen, metal ions such as ferric ion, ascorbic acid, thiamine pyrophosphate, buffering agents, and other various medium components. Ascorbic acid and oxygen can be, for example, components useful for HMAS to function. That is, at least in the part using HMAS, the conversion reaction may be carried out in the presence of ascorbic acid and oxygen. As for carrying out the conversion reaction in the presence of ascorbic acid and oxygen in the part using HMAS, the descriptions below concerning carrying out the part using HMAS in the presence of catalase can be similarly applied. Oxygen may be supplied to the reaction mixture by carrying out the conversion reaction under an atmosphere containing oxygen such as air. Components that can form a salt may each be used as a free compound, a salt thereof, or a mixture thereof. That is, the term "component" used for a component that can form a salt in the present invention refers to the component in a free form, a salt thereof, or a mixture thereof, unless otherwise stated. As for the salt of a component that can form a salt, the descriptions concerning the salt of L-phenylalanine can be similarly applied. The types and concentrations of the components contained in the reaction mixture may be set according to various conditions such as the characteristics of the benzaldehyde generation enzymes and the type of the host.

Conditions of the conversion reaction (dissolved oxygen concentration, pH of the reaction mixture, reaction temperature, reaction time, concentrations of various components, etc.) are not particularly limited so long as benzaldehyde is generated. The conversion reaction can be performed with, for example, usual conditions used for substance conversion using an enzyme (e.g. substance conversion using a purified enzyme or substance conversion using microbial cells such as resting cells). The conditions of the conversion reaction may be set according to various conditions such as the characteristics of the benzaldehyde generation enzymes and the type of the host. The conversion reaction can be performed, for example, under an aerobic condition. The term "aerobic condition" may refer to a condition where the dissolved oxygen concentration in the culture medium is 0.33 ppm or higher, or preferably 1.5 ppm or higher. The oxygen concentration can be controlled to be, for example, 1 to 50%, preferably about 5%, of the saturated oxygen concentration. The pH of the reaction mixture may be, for example, usually 6.0 to 10.0, preferably 6.5 to 9.0. The reaction temperature may be, for example, usually 15 to 50°C, preferably 15 to 45°C, more preferably 20 to 40°C. The reaction time may be, for example, 5 minutes to 200 hours. In the case of the column method, the loading rate of the reaction mixture may be, for example, such a rate that the reaction time falls within the range of the reaction time exemplified above. Furthermore, the conversion reaction can also be performed with, for example, a culture condition, such as usual conditions used for culture of microorganisms such as bacteria and yeast. In cases where cells such as the cells of the microorganism of the present invention are used, the cells may or may not proliferate during the conversion reaction. That is, the descriptions concerning the cultivation in the first embodiment of the substance conversion method may also be similarly applied to the conversion reaction in the second embodiment of the substance conversion method, except that the cells may or may not proliferate in the second embodiment. In such a case, the culture conditions for obtaining the cells and the conditions of the conversion reaction may be or may not be the same. The concentration of L-phenylalanine in the reaction mixture, for example, may be 1 mM or higher, 10 mM or higher, or 30 mM or higher, or may be 5 M or lower, 2 M or lower, or 1 M or lower, or may be within a range defined with a combination thereof. The density of the cells in the reaction mixture, for example, may be 1 or higher, or may be 300 or lower, or may be within a range defined with a combination thereof, in terms of the optical density (OD) at 600 nm.

During the conversion reaction, the benzaldehyde generation enzymes (e.g. the cells of the microorganism of the present invention), L-phenylalanine, and the other components may be additionally supplied to the reaction mixture independently or in any combination. For example, L-phenylalanine may be additionally supplied to the reaction mixture in proportion to decrease or depletion of L-phenylalanine accompanying generation of benzaldehyde. These components may be supplied once or a plurality of times, or may be continuously supplied.

Means for supplying the various components such as L-phenylalanine to the reaction mixture is not particularly limited. These components each can be supplied to the reaction mixture by, for example, directly adding them to the reaction mixture. Furthermore, for example, the benzaldehyde generation enzymes (e.g. the cells of the microorganism of the present invention) and an L-phenylalanine-producing microorganism can be allowed to coexist (e.g. co-cultured) to allow the L-phenylalanine-producing microorganism to produce L-phenylalanine in the reaction mixture, and thereby supply L-phenylalanine to the reaction mixture.

Furthermore, the reaction conditions may be constant from the start to the end of the conversion reaction, or they may be changed during the conversion reaction.

The expression "the reaction conditions are changed during the conversion reaction" includes not only cases where the reaction conditions are temporally changed, but also includes cases where the reaction conditions are spatially changed. The expression "the reaction conditions are spatially changed" means that, for example, when the conversion reaction is performed by the column method, the reaction conditions such as reaction temperature and enzyme density (e.g. cell density) differ depending on position in the flow.

A culture broth or reaction mixture containing benzaldehyde is obtained by carrying out the substance conversion step as described above. Confirmation of the production of benzaldehyde and collection of benzaldehyde can be carried out in the same manners as those for the fermentation method described above. That is, the substance conversion method may further comprise the collection step, e.g. a step of collecting benzaldehyde from the culture broth or reaction mixture. The collected benzaldehyde may contain, for example, the objective enzymes, microbial cells, medium components, reaction mixture components, moisture, and by-product metabolites of the microorganism, in addition to benzaldehyde. Purity of the collected benzaldehyde may be, for example, 30% (w/w) or higher, 50% (w/w) or higher, 70% (w/w) or higher, 80% (w/w) or higher, 90% (w/w) or higher, or 95% (w/w) or higher.

### <4-3> Combination of substeps

The production step, such as the fermentation step and substance conversion step, may be performed, but is not limited to, via generation of an intermediate. Examples of the intermediate include phenylpyruvate, (S)-mandelate, and benzoylformate, which are the products of reactions catalyzed by AAD, HMAS, and SMDH, respectively. In the array consisting of phenylpyruvate, (S)-mandelate, and benzoylformate, the side of phenylpyruvate is also referred to as "upstream", and the side of benzoylformate is also referred to as "downstream". That is, the production step may comprise a plurality of steps, wherein the plurality of steps is accompanied by generation of one or more intermediates. Each of such plurality of steps comprised in the production step is also referred to as "substep". The production step may comprise, for example, 2, 3, or 4 substeps. Examples of the substeps of the production step include a fermentation substep and a substance conversion substep. Examples of the fermentation substep include a step of producing an intermediate from a carbon source. Examples of the substance conversion substep include a step of converting L-phenylalanine into an intermediate, a step of converting an intermediate into another downstream intermediate, and a step of converting an intermediate into benzaldehyde. The combination of the substeps of the production step is not particularly limited, so long as production of benzaldehyde is attained.

The production step may comprise, for example, a step of producing an intermediate from a carbon source, and a step of converting the intermediate into benzaldehyde. That is, the production step may comprise, for example, a step of producing phenylpyruvate, (S)-mandelate, or benzoylformate from a carbon source, and a step of converting phenylpyruvate, (S)-mandelate, or benzoylformate into benzaldehyde. Specifically, the production step may comprise, for example, a step of producing benzoylformate from a carbon source, and a step of converting benzoylformate into benzaldehyde. Furthermore, the production step may comprise, for example, a step of producing an intermediate A from a carbon source, a step of converting the intermediate A into another downstream intermediate B, and a step of converting the intermediate B into benzaldehyde. Furthermore, the production step may comprise, for example, a step of producing phenylpyruvate from a carbon source, a step of converting phenylpyruvate into (S)-mandelate, a step of converting (S)-mandelate into benzoylformate, and a step of converting benzoylformate into benzaldehyde.

The production step may also comprise, for example, a step of converting L-phenylalanine into an intermediate, and a step of converting the intermediate into benzaldehyde. That is, the production step may comprise, for example, a step of converting L-phenylalanine into phenylpyruvate, (S)-mandelate, or benzoylformate, and a step of converting phenylpyruvate, (S)-mandelate, or benzoylformate into benzaldehyde. Specifically, the production step may comprise, for example, a step of converting L-phenylalanine into benzoylformate, and a step of converting benzoylformate into benzaldehyde. Furthermore, the production step may comprise, for example, a step of converting L-phenylalanine into an intermediate A, a step of converting the intermediate A into another downstream intermediate B, and a step of converting the intermediate B into benzaldehyde. Furthermore, the production step may comprise, for example, a step of converting L-phenylalanine into phenylpyruvate, a step of converting phenylpyruvate into (S)-mandelate, a step of converting (S)-mandelate into benzoylformate, and a step of converting benzoylformate into benzaldehyde.

Furthermore, each substep of the production step may comprise further substeps, as with the production step. The descriptions concerning the production step and substeps thereof can be similarly applied to each substep of the production step and further substeps thereof. That is, for example, a step of converting L-phenylalanine into benzoylformate may comprise a step of converting L-phenylalanine into phenylpyruvate, a step of converting phenylpyruvate into (S)-mandelate, and a step of converting (S)-mandelate into benzoylformate. In each fermentation substep, the intermediate to be produced is also referred to as "product". In each substance conversion substep, the substance before conversion is also referred to as "substrate", and the substance after conversion is also referred to as "product". That is, for example, in a step of converting L-phenylalanine into an intermediate, L-phenylalanine is considered as the substrate, and the intermediate is considered as the product.

Means for carrying out substeps of the production step can be set independently for each of the substeps. The descriptions concerning the means for carrying out the fermentation step can be similarly applied to the means for carrying out each fermentation substep of the production step. The descriptions concerning the means for carrying out the substance conversion step can be similarly applied to the means for carrying out each substance conversion substep of the production step. In this case, the term "benzaldehyde generation enzymes" (e.g. the term "the microorganism of the present invention") used in the production step can be read as the benzaldehyde generation enzyme(s) (e.g. a microorganism having benzaldehyde generation enzyme(s)) corresponding to each substep. Furthermore, "benzaldehyde", which is the product of the fermentation step, can be read as the corresponding product of each fermentation substep. Also, "L-phenylalanine" and "benzaldehyde", which are the substrate and product of the substance conversion step, can be read as the corresponding substrate and product of each substance conversion substep, respectively. In each substance conversion substep, the product generated in the immediately preceding substep is used as the substrate, except for L-phenylalanine. The substrate of each substance conversion substep may be used as a free compound, a salt thereof, or a mixture thereof. That is, the term "substrate" in the present invention refers to the substrate in a free form, a salt thereof, or a mixture thereof, unless otherwise stated. The descriptions concerning the salt of L-phenylalanine can be similarly applied to the salt of substrates.

Each substep of the production step is performed by using the benzaldehyde generation enzyme(s) corresponding to each substep (e.g. a microorganism having the benzaldehyde generation enzyme(s) corresponding to the substep). The term "benzaldehyde generation enzyme(s) corresponding to a substep of the production step" used for each fermentation substep refers to a single enzyme or sequential enzymes that catalyze the conversion of L-phenylalanine into the product of the substep. Also, the term "benzaldehyde generation enzyme(s) corresponding to a substep of the production step" used for each substance conversion substep refers to a single enzyme or sequential enzymes that catalyze the conversion of the substrate of the substep into the product of the substep. That is, for example, a step of producing phenylpyruvate from a carbon source can be carried out by using a microorganism having AAD (e.g. a microorganism having AAD as well as L-phenylalanine-producing ability). Furthermore, for example, a step of producing (S)-mandelate from a carbon source can be carried out by using a microorganism having AAD and HMAS (e.g. a microorganism having AAD and HMAS as well as L-phenylalanine-producing ability). Furthermore, for example, a step of producing benzoylformate from a carbon source can be carried out by using a microorganism having AAD, HMAS, and SMDH (e.g. a microorganism having AAD, HMAS, and SMDH as well as L-phenylalanine-producing ability). Furthermore, for example, a step of converting L-phenylalanine into phenylpyruvate, a step of converting phenylpyruvate into (S)-mandelate, a step of converting (S)-mandelate into benzoylformate, and a step of converting benzoylformate into benzaldehyde can be carried out by using AAD (e.g. a microorganism having AAD), HMAS (e.g. a microorganism having HMAS), SMDH (e.g. a microorganism having SMDH), and BFDC (e.g. a microorganism having BFDC), respectively. Furthermore, for example, a step of converting L-phenylalanine into phenylpyruvate, (S)-mandelate, or benzoylformate can be carried out by using AAD (e.g. a microorganism having AAD), AAD and HMAS (e.g. a microorganism having AAD and HMAS), or AAD, HMAS, and SMDH (e.g. a microorganism having AAD, HMAS, and SMDH), respectively. Furthermore, for example, step of converting phenylpyruvate, (S)-mandelate, or benzoylformate into benzaldehyde can be carried out by using HMAS, SMDH, and BFDC (e.g. a microorganism having HMAS, SMDH, and BFDC), SMDH and BFDC (e.g. a microorganism having SMDH and BFDC), or BFDC (e.g. a microorganism having BFDC), respectively. For any other substep, the benzaldehyde generation enzyme(s) (e.g. microorganism having the benzaldehyde generation enzyme(s)) required for the substep can be appropriately employed.

In cases where a microorganism having a plurality of benzaldehyde generation enzymes is used for a substep of the production step, the microorganism may be a single microorganism, or may be a combination of a plurality of microorganisms. The descriptions concerning the microorganism of the present invention (i.e. the microorganism having the four benzaldehyde generation enzymes) can be similarly applied to the microorganism having a plurality of benzaldehyde generation enzymes. That is, for example, the microorganism having AAD, HMAS, and SMDH may be a single microorganism alone having AAD, HMAS, and SMDH, or may be a combination of a plurality of microorganisms having AAD, HMAS, and SMDH as a whole.

A microorganism used for a certain substep of the production step may be different from or the same as a microorganism used for another substep of the production step. That is, in cases where a single microorganism alone has benzaldehyde generation enzyme(s) corresponding to a certain substep and benzaldehyde generation enzyme(s) corresponding to another substep, the microorganism can be used commonly for these substeps.

Each substep of the production step may be performed by, for example, cultivating the microorganism having benzaldehyde generation enzyme(s) corresponding to the substep, or using cells of the microorganism having benzaldehyde generation enzyme(s) corresponding to the sub step. That is, each fermentation sub step of the production step may also be a step of cultivating the microorganism having benzaldehyde generation enzyme(s) corresponding to the substep and having L-phenylalanine-producing ability in a culture medium containing a carbon source to generate and accumulate the corresponding product in the culture medium. Furthermore, each substance conversion substep of the production step may be a step of cultivating the microorganism having benzaldehyde generation enzyme(s) corresponding to the substep in a culture medium containing the corresponding substrate to generate and accumulate the corresponding product in the culture medium. Each substance conversion substep of the production step may also be a step of allowing the benzaldehyde generation enzyme(s) corresponding to the substep (e.g. cells of the microorganism having benzaldehyde generation enzyme(s) corresponding to the substep) to coexist with the corresponding substrate in a reaction mixture to generate and accumulate the corresponding product in the reaction mixture. All the substeps of the production step may be performed by cultivation or by using the cells. Alternatively, a part of the substeps may be performed by cultivation, while the remaining part of the substeps may be performed by using the cells. Conditions for carrying out each substep of the production step may be appropriately chosen depending on the various conditions such as type(s) of benzaldehyde generation enzyme(s) corresponding to the substep and type(s) of microorganism(s) used in the substep.

The production step may comprise, for example, the steps (A1) and (A2) mentioned below:
(A1) a step of producing benzoylformate by using AAD, HMAS, and SMDH;
(A2) a step of converting benzoylformate generated in the step (A1) into benzaldehyde by using BFDC.

The step (A1) may comprise the step (1a), (1b), or (1c) mentioned below:
(1a) cultivating at least one microorganism in a culture medium containing the carbon source to generate and accumulate benzoylformate in the culture medium, wherein the at least one microorganism is a single microorganism alone having AAD, HMAS, and SMDH as well as L-phenylalanine-producing ability or a combination of a plurality of microorganisms as a whole having AAD, HMAS, and SMDH as well as L-phenylalanine-producing ability;
(1b) cultivating at least one microorganism in a culture medium containing L-phenylalanine to generate and accumulate benzoylformate in the culture medium, wherein the at least one microorganism is a single microorganism alone having AAD, HMAS, and SMDH or a combination of a plurality of microorganisms as a whole having AAD, HMAS, and SMDH
(1c) allowing AAD, HMAS, and SMDH to coexist with L-phenylalanine in a reaction mixture to generate and accumulate benzoylformate in the reaction mixture.

The step (A2) may comprise the step (2a) or (2b) mentioned below:
(2a) cultivating a microorganism having BFDC in a culture medium containing benzoylformate generated in the step (A1) to generate and accumulate benzaldehyde in the culture medium;
(2b) allowing BFDC to coexist with benzoylformate generated in the step (A1) in a reaction mixture to generate and accumulate benzaldehyde in the reaction mixture.

AAD, HMAS, and SMDH in the step (1c) may be used, for example, in the form of cells of at least one microorganism having AAD, HMAS, and SMDH. The at least one microorganism may be a single microorganism alone having AAD, HMAS, and SMDH or a combination of a plurality of microorganisms as a whole having AAD, HMAS, and SMDH.

BFDC in the step (2b) may be used, for example, in the form of cells of a microorganism having BFDC.

The substeps of the production step may be or may not be performed individually. That is, a part or all of the substeps of the production step may be performed simultaneously during a partial period or over the whole period. For example, a substep A that generates a product and a substep B that uses the product as a substrate may be performed individually, or may be performed simultaneously during a partial period or over the whole period. That is, for example, the substeps A and B may be initiated simultaneously, or the substep B may be initiated during progress of or after completion of the substep A. For example, the substeps A and B can be initiated simultaneously by allowing the benzaldehyde generation enzymes corresponding to the substeps A and B (e.g. a microorganism having the benzaldehyde generation enzymes corresponding to the substeps A and B) to coexist with the substrate of the substep A in the reaction system (e.g. reaction mixture or culture medium) at start of the substep A. Alternatively, for example, the substep A can be initiated under conditions where the benzaldehyde generation enzyme(s) corresponding to the substep B (e.g. a microorganism having the benzaldehyde generation enzyme(s) corresponding to the substep B) does not present in the reaction system, and then the substep B can be initiated by allowing the benzaldehyde generation enzyme(s) corresponding to the substep B (e.g. the microorganism having the benzaldehyde generation enzyme(s) corresponding to the substep B) to present in the reaction system during progress of or after completion of the substep A. Furthermore, the product of the substep A may be or may not be collected before use. That is, for example, the product of the substep A may be collected, and the substep B may be performed by using the thus-collected product. The product of the substep A may be used for the substep B as it is, or after being subjected to an appropriate treatment such as concentration, dilution, drying, dissolution, fractionation, extraction, and purification, as required. These descriptions concerning the substeps A and B can be applied to any combination of successive substeps.

### <4-4> Use of catalase

In the method of the present invention, at least a part of the production step is carried out in the presence of catalase. By carrying out at least a part of the production step in the presence of catalase, production of benzaldehyde can be improved. That is, production of benzaldehyde can be improved by using catalase as compared to the case of not using catalase. Examples of the improved production of benzaldehyde include improvement in the production amount of benzaldehyde and improvement in the yield of benzaldehyde.

The improved production of benzaldehyde by using catalase may be due to, for example, degradation of hydrogen peroxide in the culture medium or reaction mixture by catalase. Hydrogen peroxide may be generated from, for example, ascorbic acid and oxygen. Ascorbic acid and oxygen can be used, for example, in combination with HMAS. By degradation of hydrogen peroxide, for example, the activity of HMAS may be increased. Specifically, for example, in cases where the activity of HMAS is inhibited by hydrogen peroxide, degradation of hydrogen peroxide may decrease the inhibition of the activity of HMAS, and thereby the activity of HMAS may be increased. In other words, the improved production of benzaldehyde may be due to, for example, an increase in the activity of HMAS. By the increase in the activity of HMAS, for example, production of (S)-mandelate may be improved. By the improved production of (S)-mandelate, for example, production of benzoylformate may be improved. By the improved production of benzoylformate, for example, production of benzaldehyde may be improved. In other words, the improved production of benzaldehyde may be due to, for example, the improved production of intermediate(s) of benzaldehyde, such as (S)-mandelate and benzoylformate.

That is, examples of at least a part of the production step include a part using HMAS. Examples of the part using HMAS include a period where HMAS is present in the culture medium or reaction mixture. Specific examples of the period where HMAS is present in the culture medium or reaction mixture include a period where a microorganism having HMAS is cultured and a period where HMAS (e.g. a microorganism having HMAS) is present in the reaction mixture. Examples of the part using HMAS also include a substep using HMAS. Particular examples of the part using HMAS also include a period where HMAS is present in the culture medium or reaction mixture within the substep using HMAS.

The use amount of catalase is not particularly limited. so long as production of benzaldehyde can be improved. The use amount of catalase, for example, may be 10 U/mL or more, 20 U/mL or more, 50 U/mL or more, 100 U/mL or more, 200 U/mL or more, 500 U/mL or more, 1000 U/mL or more, 2000 U/mL or more, or 5000 U/mL or more, may be 500000 U/mL or less, 200000 U/mL or less, 100000 U/mL or less, 50000 U/mL or less, 20000 U/mL or less, 10000 U/mL or less, 5000 U/mL or less, 2000 U/mL or less, 1000 U/mL or less, or 500 U/mL or less, or may be within a range defined as a non-contradictory combination thereof, in terms of the catalase activity in the culture medium or reaction mixture. The use amount of catalase may be, specifically, for example, 10 to 100000 U/mL, 100 to 50000 U/mL, or 1000 to 20000 U/mL in terms of the catalase activity in the culture medium or reaction mixture. One unit of catalase activity is defined as an amount of enzyme degrading 1 µmol of hydrogen peroxide per one minute at pH 7.0 and 25°C. The use amount of catalase, for example, may be 1 or higher, or may be 300 or lower, or may be within a range defined as a combination thereof, in terms of the optical density (OD) at 600 nm of host cells having catalase in in the culture medium or reaction mixture.

So long as catalase is used in at least a part of the production step, catalase may be or may not be used in other part(s) of the production step. That is, so long as catalase is used in at least a part of the production step, catalase may be or may not be present in the culture medium or reaction mixture in other part(s) of the production step.

In cases where the part using HMAS is chosen as at least a part of the production step, catalase may be present in the culture medium or reaction mixture over the whole period of the part using HMAS, or may be present in the culture medium or reaction mixture during a partial period of the part using HMAS. The phrase "the part using HMAS is carried out in the presence of catalase" does not necessarily mean that catalase is contained in the culture medium or reaction mixture over the whole period of the part using HMAS. Catalase may be present in the culture medium or reaction mixture, for example, during a period having a length of 50% or longer, 60% or longer, 70% or longer, 80% or longer, 90% or longer, 95% or longer, 97% or longer, 99% or longer, or 100% of the part using HMAS. For example, catalase may be or may not be contained in the culture medium or reaction mixture from the start of the part using HMAS. When catalase is not contained in the culture medium or reaction mixture at the start of the part using HMAS, catalase is supplied to the culture medium or reaction mixture after the start of the part using HMAS. Timing of the supply can be appropriately set according to various conditions such as the length of the culture period and reaction period. For example, in cases where a microorganism having HMAS such as the microorganism of the present invention grows in the part using HMAS, catalase may be supplied to the culture medium or reaction mixture after the microorganism having HMAS such as the microorganism of the present invention sufficiently grows. Furthermore, in any case, catalase may be additionally supplied to the culture medium or reaction mixture as required. Means for supplying catalase to the culture medium or reaction mixture is not particularly limited. Catalase may be or may not be contained in the culture medium or reaction mixture at a concentration within the range exemplified above over the whole period of the part using HMAS. For example, catalase may be contained in the culture medium or reaction mixture at a concentration within the range exemplified above at the start of the part using HMAS, or it may be supplied to the culture medium or reaction mixture so that a concentration within the range exemplified above is attained after the start of the part using HMAS.

Catalase may be used for production of benzaldehyde in any form such as those described above. That is, examples of the use of catalase include use of it in any form such as those described above. Catalase may be used, for example, in the form contained in host cells such as microbial cells. Also, catalase may be used, for example, in the form not contained in host cells, e.g. in the form of a purified enzyme. Catalase may be supplied to the culture medium or reaction mixture separately from the benzaldehyde generation enzymes, or may be supplied to the culture medium or reaction mixture together with the benzaldehyde generation enzymes. For example, by allowing a host having the catalase gene and the benzaldehyde generation enzyme genes to express these genes, catalase and the benzaldehyde generation enzymes can be produced at once, and thereby supplied to the culture medium or reaction mixture at once. Also, in cases where the host having the benzaldehyde generation enzyme(s) such as the microorganism of the present invention has catalase, at least a part of the production step can be carried out in the presence of catalase by carrying out the production step by using the host. For example, in cases where a microorganism at least having HMAS or a microorganism to be used in coexistence therewith has catalase, the part using HMAS can be carried out in the presence of catalase by carrying out the production step by using the host.

Catalase may be or may not be used, for example, in combination with other components that degrade(s) hydrogen peroxide.

### <5> Another embodiment of the method of the present invention

The method of the present invention can also be carried out not only by using catalase but also by using any component that degrades hydrogen peroxide.

That is, another embodiment of the method of the present invention is a method for producing benzaldehyde comprising a step of producing benzaldehyde by using amino acid deaminase (AAD), 4-hydroxymandelate synthase (HMAS), (S)-mandelate dehydrogenase (SMDH), and benzoylformate decarboxylase (BFDC), wherein at least a part of the step is carried out in the presence of a component that degrades hydrogen peroxide.

The aforementioned descriptions concerning the method of the present invention can be similarly applied to such another embodiment, except that a component that degrades hydrogen peroxide is used instead of catalase in such another embodiment. Also, the aforementioned descriptions concerning use of catalase in the method of the present invention can be similarly applied to use of a component that degrades hydrogen peroxide in such another embodiment.

The component that degrades hydrogen peroxide may be a component other than catalase. Examples of the component that degrades hydrogen peroxide include manganese dioxide. Examples of the component that degrades hydrogen peroxide also include peroxidases such as ascorbate peroxidase and peroxiredoxin_{∘}

The component that degrades hydrogen peroxide may be or may not be used, for example, in combination with catalase.

### Examples

Hereinafter, the present invention will be more specifically explained with reference to non-limiting examples.

### Example 1: Expression of amino acid deaminase (AAD)

*E. coli* JM109/pSFN-AAD, which is a strain expressing AAD derived from *Providencia rettgeri* AJ2770 disclosed in the method for producing benzaldehyde (WO2017/122747, Example 1), was cultured overnight at 25°C on LB-amp (100 mg/L) plate. The obtained cells were inoculated into 100 mL of TB-amp (100 mg/L), i.e. TB medium containing 100 mg/L of ampicillin, and cultured at 25°C with shaking for 16 hr using a Sakaguchi flask. The obtained culture broth was hereinafter used as an AAD culture broth.

### Example 2: Expression of 4-hydroxymandelate synthase (HmaS)

### (1) Construction of expression plasmids for quartet mutant HmaS

By using the plasmid pPC-hmaS At-His A199V/I217V/K337Q(SDatc), which contains a triple mutant HmaS (HmaS At, A199V/I217V/K337Q) gene derived from *Actinoplanes teichomyceticus* disclosed in the method for producing benzaldehyde (WO2017/122747, Example 8), as the template, a DNA fragment containing the triple mutant hmaS At gene was PCR-amplified. For amplification of the 1st half, primers RV (5'-CAGGAAACAGCTATGAC-3'; SEQ ID NO: 33) and Q206R-R (5'-GGCACTacgAACCACCTGGGAATCCAT-3'; SEQ ID NO: 34) were used. For amplification of the 2nd half, primers Q206R-F (5'-GTGGTTcgtAGTGCCGGTGGGGCTGTG-3'; SEQ ID NO: 35) and M4 (5'-GTTTTCCCAGTCACGAC-3'; SEQ ID NO: 36) were used. PCR was carried out by using KOD-plus-ver.2 (TOYOBO) under the following conditions:

| | |
|---|---|
| 1 cycle | 94°C, 2 min |
| 25 cycles | 98°C, 10 sec |
| | 60°C, 10 sec |
| | 68°C, 60 sec |
| 1 cycle | 68°C, 60 sec |
| | 4°C |

By PCR using the obtained two DNA fragments as the templates and primers of RV and M4, a DNA fragment containing the quartet mutant hmaS At gene in full length was amplified. PCR was carried out by using KOD-plus-ver.2 (TOYOBO) under the aforementioned conditions.

The obtained DNA fragment of about 1100 bp was treated with restriction enzymes *Nde*I and *Xho*I, and ligated with pPC-hmaS At-His (SDatc) (WO2017/122747, Example 8) similarly treated with *Nde*I and *Xho*I. *E. coli* JM109 was transformed with this ligation mixture, an objective plasmid was extracted from an ampicillin resistant strain. The obtained plasmid was designated as pPC-hmaS At-His A199V/Q206R/I217V/K337Q (SDatc). This plasmid expresses the quartet mutant HmaS At (A199V/Q206R/I217V/K337Q) added with His-tag at the C-terminus. The nucleotide sequence of the quartet mutant hmaS At-His gene is shown as SEQ ID NO: 1, and the amino acid sequence of HMAS encoded by this gene is shown as SEQ ID NO: 2.

### (2) Expression of quartet mutant HmaS

*E. coli* JM109 was introduced with the plasmid pPC-hmaS At-His A199V/Q206R/I217V/K337Q (SDatc), which contains the quartet mutant HmaS gene derived from *Actinoplanes teichomyceticus,* and a transformant strain harboring this plasmid was obtained from ampicillin resistant strains. The transformant strain was cultured overnight at 25°C on LB-amp (100 mg/L) plate. The obtained cells were inoculated into 100 mL of TB-amp (100 mg/L), and cultured at 37°C with shaking for 16 hr using a Sakaguchi flask. The obtained culture broth was hereinafter used as a HmaS At culture broth.

### Example 3: Expression of (S)-mandelate dehydrogenase (MdlB, SMDH)

*E. coli* JM109 (DE3) was introduced with the plasmid pET22-mdlB, which contains mdlB gene derived from *Pseudomonas putida* disclosed in the method for producing benzaldehyde (WO2017/122747, Example 3), and a transformant strain harboring this plasmid was obtained from ampicillin resistant strains. The transformant strain was cultured overnight at 25°C on LB-amp (100 mg/L) plate. The obtained cells were inoculated into 100 mL of Overnight Express Instant TB Medium (Novagen) containing 100 mg/L of ampicillin contained, and cultured at 37°C with shaking for 16 hr using a Sakaguchi flask. The obtained culture broth was hereinafter used as a MdlB culture broth.

### Example 4: Expression of benzoylformate decarboxylase (MdlC, BFDC)

*E. coli* BL21 (DE3) /pET22-mdlC, which is a strain expressing MdlC derived from *Pseudomonas putida* disclosed in the method for producing benzaldehyde (WO2017/122747, Example 4), was cultured overnight at 25°C on LB-amp (100 mg/L) plate. The obtained cells were inoculated into 100 mL of Overnight Express Instant TB Medium (Novagen) containing 100 mg/L of ampicillin contained, and cultured at 37°C with shaking for 16 hr using a Sakaguchi flask. The obtained culture broth was hereinafter used as a MdlC culture broth.

### Example 5: Synthesis of benzaldehyde from L-Phe with addition of catalase

### (1) Analysis conditions

Benzaldehyde was quantified by HPLC analysis. The analysis conditions were as follows.
Mobile phase A: 10 mM KH₂PO₄/10 mM K₂HPO₄
Mobile phase B: acetonitrile
Flow rate: 1.0 mL/min
Column temperature: 40°C
Detection: UV 210 nm
Column: CAPCELL PAK MGII, 4.6 x 150 mm, 3 µm (Shiseido)
Gradient: 0-2 min (B: 2%), 2-16 min (B: 2-50%), 16.1-20 min (B: 2%)

### (2) Preparation of concentrates

A 10 mL aliquot of the AAD culture broth obtained in Example 1 was centrifuged, 8 mL of the supernatant was removed, and the cells were suspended in the remaining culture supernatant, to obtain a 5-fold concentrated culture broth. This was used for the following reactions as an AAD concentrate. Similarly, 5-fold concentrates were prepared from the HmaS At culture broths, MdlB culture broth, and MdlC culture broth obtained in Examples 2 to 4, and used for the following reactions as a HmaS At concentrates, MdlB concentrate, and MdlC concentrate.

### (3) Benzaldehyde synthesis reaction

A reaction mixture (1 mL) containing 50 mM of L-Phe, 0.01 mM of iron sulfate, 10 mM of trisodium citrate, 30 mM of sodium ascorbate, 10 mM of thiamine pyrophosphate chloride, 1 mM of magnesium sulfate, 100 mM of potassium phosphate buffer (pH 7.0), 0.02 mL of the AAD concentrate, 0.1 mL of the HmaS At concentrate, 0.02 mL of the MdlB concentrate, and 20 µL of catalase or water was put into a test tube, and shaken at 25°C. After 20 hours, the reaction mixture was centrifuged after 20 hr, a 0.49 mL aliquot of the supernatant was mixed with 0.01 mL of the MdlC concentrate, and the resultant mixture was put into a test tube and shaken at 25°C. After 4 hours, a 0.1 mL aliquot of the reaction mixture was mixed with 1 mL of a reaction stop solution (1% phosphoric acid, 50% ethanol), and a centrifugal supernatant thereof was subjected to HPLC analysis. Catalases added and the generation amount of benzaldehyde are shown in Table 1. An improvement in the generation amount of benzaldehyde was observed by addition of any of catalases.

**Table 1**

| Catalases added (manufacturer) | Benzaldehyde (mM) |
|---|---|
| Catalase, derived from *Corynebacterium glutamicum* (Sigma-Aldrich) | 11.5 |
| Catalase, derived from *Micrococcus lysodeikticus* (Sigma-Aldrich) | 11.5 |
| Ask Super 25 (MGC Advance) | 11.3 |
| Ask Super G (MGC Advance) | 11.6 |
| ENZYNASE ROG-50 (Rakuto Kasei Industrial) | 12.0 |
| ENZYLON OL-50S (Rakuto Kasei Industrial) | 12.2 |
| Catalase U5L (HBI) | 11.4 |
| Water | 8.1 |

### Example 6: Expression of catalase (Cat)

### (1) Construction of strain expressing Cat HPI derived from E. coli (Cat 1)

Synthesis of phoC promoter derived from *Enterobacter aerogenes* and a gene encoding Cat HPI derived from *E. coli* (GenBank Accession Number: NP_418377) was outsourced to Eurofins Genomics, and a DNA fragment containing the gene ligated downstream of the phoC promoter (SEQ ID NO: 3, wherein the codon usage frequency has been optimized for expression in *E. coli*) was obtained. The DNA fragment was inserted into EcoRI-HindIII site of pMW218 (Takara Bio), to thereby construct an expression plasmid for Cat 1. The constructed plasmid was designated as pMW-cat1. The constructed plasmid was introduced into *E. coli* JM109, a transformant harboring this plasmid was obtained from kanamycin resistant strains, and used as a Cat 1-expressing strain. The amino acid sequence of Cat HPI derived from *E. coli* (Cat 1) is shown as SEQ ID NO: 4.

### (2) Construction of strain expressing Cat HPII derived from E. coli (Cat 2)

Synthesis of phoC promoter derived from *Enterobacter aerogenes* and a gene encoding Cat HPII derived from *E. coli* (GenBank Accession Number: AML01688) was outsourced to Eurofins Genomics, and a DNA fragment containing the gene ligated downstream of the phoC promoter (SEQ ID NO: 5, wherein the codon usage frequency has been optimized for expression in *E. coli)* was obtained. Then, a Cat 2-expressing strain was obtained in the same manner as (1). The amino acid sequence of Cat HPII derived from *E. coli* (Cat 2) is shown as SEQ ID NO: 6.

### (3) Construction of strain expressing Cat derived from Thermus thermophilus (Cat 3)

Synthesis of phoC promoter derived from *Enterobacter aerogenes* and a gene encoding Cat derived from *Thermus thermophilus* (GenBank Accession Number: AAS82214) was outsourced to Eurofins Genomics, and a DNA fragment containing the gene ligated downstream of the phoC promoter (SEQ ID NO: 7, wherein the codon usage frequency has been optimized for expression in *E. coli*) was obtained. Then, a Cat 3-expressing strain was obtained in the same manner as (1). The amino acid sequence of Cat derived from *Thermus thermophilus* (Cat 3) is shown as SEQ ID NO: 8.

### (4) Construction of strain expressing Cat derived from Rhodothermus marinus (Cat 4)

Synthesis of phoC promoter derived from *Enterobacter aerogenes* and a gene encoding Cat derived from *Rhodothermus marinus* (GenBank Accession Number: ACY49648) was outsourced to Eurofins Genomics, and a DNA fragment containing the gene ligated downstream of the phoC promoter (SEQ ID NO: 9, wherein the codon usage frequency has been optimized for expression in *E. coli*) was obtained. Then, a Cat 4-expressing strain was obtained in the same manner as (1). The amino acid sequence of Cat derived from *Rhodothermus marinus* (Cat 4) is shown as SEQ ID NO: 10.

### (5) Construction of strain expressing Cat derived from Corynebacterium glutamicum (Cat 5)

Synthesis of phoC promoter derived from *Enterobacter aerogenes* and a gene encoding Cat derived from *Corynebacterium glutamicum* (GenBank Accession Number: BAV22054) was outsourced to Eurofins Genomics, and a DNA fragment containing the gene ligated downstream of the phoC promoter (SEQ ID NO: 11, wherein the codon usage frequency has been optimized for expression in *E. coli*) was obtained. Then, a Cat 5-expressing strain was obtained in the same manner as (1). The amino acid sequence of Cat derived from *Corynebacterium glutamicum* (Cat 5) is shown as SEQ ID NO: 12.

### (6) Construction of strain expressing Cat derived from Bacillus subtilis (Cat 6)

Synthesis of phoC promoter derived from *Enterobacter aerogenes* and a gene encoding Cat derived from *Bacillus subtilis* (GenBank Accession Number: NP_388762) was outsourced to Eurofins Genomics, and a DNA fragment containing the gene ligated downstream of the phoC promoter (SEQ ID NO: 13, wherein the codon usage frequency has been optimized for expression in *E. coli*) was obtained. Then, a Cat 6-expressing strain was obtained in the same manner as (1). The amino acid sequence of Cat derived from *Bacillus subtilis* (Cat 6) is shown as SEQ ID NO: 14.

### (7) Construction of strain expressing Cat derived from Pseudomonas syringae (Cat 7)

Synthesis of phoC promoter derived from *Enterobacter aerogenes* and a gene encoding Cat derived from *Pseudomonas syringae* (GenBank Accession Number: AAC61659) was outsourced to Eurofins Genomics, and a DNA fragment containing the gene ligated downstream of the phoC promoter (SEQ ID NO: 15, wherein the codon usage frequency has been optimized for expression in *E. coli*) was obtained. Then, a Cat 7-expressing strain was obtained in the same manner as (1). The amino acid sequence of Cat derived from *Pseudomonas syringae* (Cat 7) is shown as SEQ ID NO: 16.

### (8) Construction of strain expressing Cat derived from Pseudomonas putida (Cat 8)

Synthesis of phoC promoter derived from *Enterobacter aerogenes* and a gene encoding Cat derived from *Pseudomonas putida* (GenBank Accession Number: WP_012313921) was outsourced to Eurofins Genomics, and a DNA fragment containing the gene ligated downstream of the phoC promoter (SEQ ID NO: 17, wherein the codon usage frequency has been optimized for expression in *E. coli*) was obtained. Then, a Cat 8-expressing strain was obtained in the same manner as (1). The amino acid sequence of Cat derived from *Pseudomonas putida* (Cat 8) is shown as SEQ ID NO: 18.

### (9) Construction of strain expressing PelB signal sequence derived from E. coli and Cat derived from Pseudomonas putida (Cat 9)

Synthesis of phoC promoter derived from *Enterobacter aerogenes* and a gene encoding PelB signal sequence derived from *E. coli* and Cat derived from *Pseudomonas putida* whose signal sequence was removed was outsourced to Eurofins Genomics, and a DNA fragment containing the gene ligated downstream of the phoC promoter (SEQ ID NO: 19, wherein the codon usage frequency has been optimized for expression in *E*. *coli*) was obtained. Then, a Cat 9-expressing strain was obtained in the same manner as (1). The amino acid sequence of Cat derived from *Pseudomonas putida* added with PelB signal sequence derived from *E. coli* (Cat 9) is shown as SEQ ID NO: 20.

### (10) Construction of strain expressing Cat derived from Shewanella oneidensis (Cat 10)

Synthesis of phoC promoter derived from *Enterobacter aerogenes* and a gene encoding Cat derived from *Shewanella oneidensis* (GenBank Accession Number: NP_716358) was outsourced to Eurofins Genomics, and a DNA fragment containing the gene ligated downstream of the phoC promoter (SEQ ID NO: 21, wherein the codon usage frequency has been optimized for expression in *E. coli*) was obtained. Then, a Cat 10-expressing strain was obtained in the same manner as (1). The amino acid sequence of Cat derived from *Shewanella oneidensis* (Cat 10) is shown as SEQ ID NO: 22.

(11) Construction of strain expressing Cat derived from *Pseudomonas entomophila* (Cat 11)

Synthesis of a gene encoding Cat derived from *Pseudomonas entomophila* (GenBank Accession Number: WP_011533980) was outsourced to Eurofins Genomics, and a plasmid containing the gene was obtained. This DNA fragment was treated with NdeI and SacI, to thereby obtain a DNA fragment containing the cat 11 gene (SEQ ID NO: 23, wherein the codon usage frequency has been optimized for expression in *E*. *coli*).

By PCR using pMW218-cat3 obtained in (3) as the template and primers pMW218_delNde I (5'-ggaattcattaATgcacagatgaaaacggtg-3') and RV (5'-CAGGAAACAGCTATGAC-3'), a DNA fragment containing the cat 3 gene, the phoC promoter, and an upstream region thereof was amplified. PCR was carried out by using KOD-plus-ver.2 (TOYOBO) under the following conditions:

| | |
|---|---|
| 1 cycle | 94°C, 2 min |
| 25 cycles | 98°C, 10 sec |
| | 55°C, 10 sec |
| | 68°C, 90 sec |
| 1 cycle | 68°C, 90 sec |
| | 4°C |

The obtained DNA fragment of about 1500 bp was treated with restriction enzymes *Vsp*I and *Eco*RI, and ligated with pMW218 treated with *Nde*I and *Eco*RI*. E. coli* JM109 was transformed with this ligation mixture, an objective plasmid was extracted from a kanamycin resistant strain. The obtained plasmid was designated as pMW-delNdeI-cat3.

The cat 11 gene was treated with NdeI and SacI, and inserted into NdeI-SacI site of pMW218-delNdeI-cat3, to thereby construct an expression plasmid for Cat 11 containing the cat 11 gene ligated downstream of the phoC promoter derived from *Enterobacter aerogenes.* The constructed plasmid was designated as pMW-cat11. The constructed plasmid was introduced into *E. coli* JM109, a transformant harboring this plasmid was obtained from kanamycin resistant strains, and used as a Cat 11-expressing strain. The amino acid sequence of Cat derived from *Pseudomonas entomophila* (Cat 11) is shown as SEQ ID NO: 24.

(12) Construction of strain expressing Cat derived from *Pseudomonas parafulva* (Cat 12)

Synthesis of a gene encoding Cat derived from *Pseudomonas parafulva* (GenBank Accession Number: WP_039579465) was outsourced to Eurofins Genomics, and a plasmid containing the gene and a DNA fragment containing the gene (SEQ ID NO: 25, wherein the codon usage frequency has been optimized for expression in *E*. *coli*) was obtained. Then, a Cat 12-expressing strain was obtained in the same manner as (11). The amino acid sequence of Cat derived from *Pseudomonas parafulva* (Cat 12) is shown as SEQ ID NO: 26.

(13) Construction of strain expressing Cat derived from *Pseudomonas protegens* (Cat 13)

Synthesis of a gene encoding Cat derived from *Pseudomonas protegens* (GenBank Accession Number: WP_053153995) was outsourced to Eurofins Genomics, and a plasmid containing the gene and a DNA fragment containing the gene (SEQ ID NO: 27, wherein the codon usage frequency has been optimized for expression in *E*. *coli*) was obtained. Then, a Cat 13-expressing strain was obtained in the same manner as (11). The amino acid sequence of Cat derived from *Pseudomonas protegens* (Cat 13) is shown as SEQ ID NO: 28.

### (14) Construction of strain expressing Cat derived from Erwinia mallotivora (Cat 14)

Synthesis of a gene encoding Cat derived from *Erwinia mallotivora* (GenBank Accession Number: WP_034939749) was outsourced to Eurofins Genomics, and a plasmid containing the gene and a DNA fragment containing the gene (SEQ ID NO: 29, wherein the codon usage frequency has been optimized for expression in *E. coli*) was obtained. Then, a Cat 14-expressing strain was obtained in the same manner as (11). The amino acid sequence of Cat derived from *Erwinia mallotivora* (Cat 14) is shown as SEQ ID NO: 30.

### (15) Construction of strain expressing Cat derived from Erwinia tracheiphila (Cat 15)

Synthesis of a gene encoding Cat derived from *Erwinia tracheiphila* (GenBank Accession Number: KKF37821) was outsourced to Eurofins Genomics, and a plasmid containing the gene and a DNA fragment containing the gene (SEQ ID NO: 31, wherein the codon usage frequency has been optimized for expression in *E. coli*) was obtained. Then, a Cat 15-expressing strain was obtained in the same manner as (11). The amino acid sequence of Cat derived from *Erwinia tracheiphila* (Cat 15) is shown as SEQ ID NO: 32.

### (16) Construction of pMW218(Ctrl) strain

Plasmid pMW218 (Takara Bio) was introduced into *E. coli* JM109, and a transformant harboring pMW218 was obtained from kanamycin resistant strains, and used as a Ctrl strain not expressing catalase.

### (17) Preparation of culture broths of Cat-expressing strains

The Cat 1-expressing strain was cultured overnight at 25°C on LB-Km (25 mg/L) plate. The obtained cells were inoculated into 4 mL of TB-Km (25 mg/L), i.e. TB medium containing 25 mg/L of kanamycin, and cultured at 30°C with shaking for 16 hr using a test tube. The obtained culture broth was hereinafter used as a Cat 1 culture broth. Similarly, Cat 2 to 15 culture broths and a Ctrl culture broth were obtained.

### Example 7: Synthesis of benzaldehyde from L-Phe with addition of culture broth of catalase-expressing strain

### (1) Analysis conditions

Analysis was carried out in the same manner as Example 5.

### (2) Preparation of concentrates

A 10 mL aliquot of the AAD culture broth obtained in Example 1 was centrifuged, 8 mL of the supernatant was removed, and the cells were suspended in the remaining culture supernatant, to obtain a 5-fold concentrated culture broth. This was used for the following reactions as an AAD concentrate. Similarly, 5-fold concentrates were prepared from the HmaS At culture broths, MdlB culture broth, and MdlC culture broth obtained in Examples 2 to 4, and used for the following reactions as a HmaS At concentrates, MdlB concentrate, and MdlC concentrate. A 1 mL aliquot of the Cat 1 culture broth obtained in Example 6 was centrifuged, 0.8 mL of the supernatant was removed, and the cells were suspended in the remaining culture supernatant, to obtain a 5-fold concentrated culture broth. This was used for the following reactions as a Cat 1 concentrate. Similarly, 5-fold concentrates were prepared from the Cat 2 to 15 culture broths and the Ctrl culture broth, and used for the following reactions as Cat 2 to 15 concentrates and a Ctrl concentrate.

### (3) Benzaldehyde synthesis reaction

A reaction mixture (1 mL) containing 50 mM of L-Phe, 0.01 mM of iron sulfate, 10 mM of trisodium citrate, 30 mM of sodium ascorbate, 1 mM of thiamine pyrophosphate chloride, 1 mM of magnesium sulfate, 100 mM of potassium phosphate buffer (pH 7.0), 0.02 mL of the AAD concentrate, 0.1 mL of the HmaS At concentrate, 0.02 mL of the MdlB concentrate, and 0.1 mL of any of the Cat 1 to 15 concentrates or the Ctrl concentrate was put into a test tube, and shaken at 25°C. After 20 hours, the reaction mixture was centrifuged, a 0.49 mL aliquot of the supernatant was mixed with 0.01 mL of the MdlC concentrate, and the resultant mixture was put into a test tube and shaken at 25°C. After 4 hours, a 0.1 mL aliquot of the reaction mixture was mixed with 1 mL of a reaction stop solution (1% phosphoric acid, 50% ethanol), and a centrifugal supernatant thereof was subjected to HPLC analysis. Cat concentrates (concentrated culture broths of catalase-expressing strains) added and the generation amount of benzaldehyde are shown in Table 2. An improvement in the generation amount of benzaldehyde was observed by addition of any of Cat concentrates. In particular, a significant improvement in the generation amount of benzaldehyde was observed by addition of Cat concentrates other than Cat 7 concentrate.

**Table 2**

| Concentrated culture broths of catalase-expressing strains added (origin of catalases) | Benzaldehyde (mM) |
|---|---|
| Cat 1 (*E. coli*) | 10.8 |
| Cat 2 (*E. coli*) | 10.7 |
| Cat 3 (*Thermus thermophiles*) | 9.5 |
| Cat 4 (*Rhodothermus marinus*) | 10.3 |
| Cat 5 (*Corynebacterium glutamicum*) | 10.5 |
| Cat 6 (*Bacillus subtilis*) | 10.7 |
| Cat 7 (*Pseudomonas syringae*) | 9.2 |
| Cat 8 (*Pseudomonas putida*) | 10.8 |
| Cat 9 (*E. coli* - *P. putida*) | 10.0 |
| Cat 10 (*Shewanella oneidensis*) | 9.6 |
| Cat 11 (*Pseudomonas entomophila*) | 10.4 |
| Cat 12 (*Pseudomonas parafulva*) | 10.4 |
| Cat 13 (*Pseudomonas protegens*) | 9.5 |
| Cat 14 (*Erwinia mallotivora*) | 10.7 |
| Cat 15 (*Erwinia tracheiphila*) | 10.4 |
| Ctrl | 9.1 |

### Example 8: Decrease in HMAS activity by hydrogen peroxide

### (1) Purification of HMAS

Cells were collected from 10 mL of the HmaS At culture broth obtained in Example 2 by centrifugation, suspended in 2 mL of xTractor Buffer (Takara Bio), and allowed to stand at room temperature for 20 minutes, to disrupt the cells. The cell residue was removed from the disruptant by centrifugation, and the obtained supernatant was used as a soluble fraction. The soluble fraction was applied to a His-tag protein purification column HisTALON Superflow Cartridges (Takara Bio, CV=1 mL) equilibrated with a buffer containing 20 mM Tris-HCl (pH 8.0), 300 mM NaCl, and 10 mM Imidazole, to adsorb proteins on the carrier. Proteins not adsorbed on the carrier (non-adsorbed proteins) were washed off with a buffer containing 20 mM Tris-HCl (pH 8.0), 300 mM NaCl, and 10 mM Imidazole, and then a buffer containing 20 mM Tris-HCl (pH 8.0), 300 mM NaCl, and 150 mM Imidazole was flowed at 1.5 mL/min, to elute the adsorbed proteins. The eluted fractions were collected and concentrated using Amicon Ultra-15 30k (Millipore). The concentrate was diluted with 20 mM of Tris-HCl (pH 7.6), and hereinafter used as a HmaS At-His purified enzyme.

### (2) Measurement of HMAS activity

A reaction mixture (0.2 mL) containing 10 mM of sodium phenylpyruvate, 0.01 mM of iron sulfate, 10 mM of trisodium citrate, 10 mM of sodium ascorbate, 100 mM of Tris-HCl (pH 7.0), 0.02 mg/mL of the HmaS At-His purified enzyme, and 0 to 1 mM of hydrogen peroxide was put into a 1.5 mL-volume tube, and a reaction was carried out at 25°C. Similarly, a reaction without addition of HmaS At-His was carried out. After 15 minutes, a 0.05 mL aliquot of the reaction mixture was mixed with 0.2 mL of a reaction stop solution (1% phosphoric acid), and subjected to HPLC analysis. HPLC analysis was carried out in the same manner as Example 5, and mandelate enzymatically-generated was quantified. The amount of hydrogen peroxide added and the generated amount of mandelate are shown in Table 3, wherein the generated amount of mandelate in the case of not adding hydrogen peroxide was taken as 100%. The generated amount of mandelate was decreased when 0.01 to 1 mM of hydrogen peroxide was added. That is, it was revealed that hydrogen peroxide inhibits HMAS activity. Hence, it is considered that benzaldehyde can be efficiently produced by, in particular, using HMAS in the presence of catalase.

**Table 3**

| Hydrogen peroxide added (mM) | Generated amount of mandelate (%) |
|---|---|
| 0 | 100 |
| 0.001 | 103 |
| 0.01 | 94 |
| 0.1 | 39 |
| 1 | 1 |

### Industrial Applicability

According to the present invention, benzaldehyde can be efficiently produced.

### <Explanation of Sequence Listing>

### SEQ ID NOS:

1: Nucleotide sequence of quartet mutant HmaS gene of *Actinoplanes teichomyceticus*
2: Amino acid sequence of quartet mutant HmaS of *Actinoplanes teichomyceticus*
3: Nucleotide sequence of Cat HPI gene of *Escherichia coli*
4: Amino acid sequence of Cat HPI of *Escherichia coli*
5: Nucleotide sequence of Cat HPII gene of *Escherichia coli*
6: Amino acid sequence of Cat HPII of *Escherichia coli*
7: Nucleotide sequence of Cat gene of *Thermus thermophilus*
8: Amino acid sequence of Cat of *Thermus thermophilus*
9: Nucleotide sequence of Cat gene of *Rhodothermus marinus*
10: Amino acid sequence of Cat of *Rhodothermus marinus*
11: Nucleotide sequence of Cat gene of *Corynebacterium glutamicum*
12: Amino acid sequence of Cat of *Corynebacterium glutamicum*
13: Nucleotide sequence of Cat gene of *Bacillus subtilis*
14: Amino acid sequence of Cat of *Bacillus subtilis*
15: Nucleotide sequence of Cat gene of *Pseudomonas syringiae*
16: Amino acid sequence of Cat of *Pseudomonas syringiae*
17: Nucleotide sequence of Cat gene of *Pseudomonas putida*
18: Amino acid sequence of Cat of *Pseudomonas putida*
19: Nucleotide sequence of Cat gene of *Pseudomonas putida*
20: Amino acid sequence of Cat of *Pseudomonas putida*
21: Nucleotide sequence of Cat gene of *Shewanella oneidensis*
22: Amino acid sequence of Cat of *Shewanella oneidensis*
23: Nucleotide sequence of Cat gene of *Pseudomonas entomophila*
24: Amino acid sequence of Cat of *Pseudomonas entomophila*
25: Nucleotide sequence of Cat gene of *Pseudomonas parafulva*
26: Amino acid sequence of Cat of *Pseudomonas parafulva*
27: Nucleotide sequence of Cat gene of *Pseudomonas protegens*
28: Amino acid sequence of Cat of *Pseudomonas protegens*
29: Nucleotide sequence of Cat gene of *Erwinia mallotivora*
30: Amino acid sequence of Cat of *Erwinia mallotivora*
31: Nucleotide sequence of Cat gene of *Erwinia tracheiphila*
32: Amino acid sequence of Cat of *Erwinia tracheiphila*
33 to 36: Primers
37: Nucleotide sequence of AAD gene of *Providencia rettgeri AJ2770*
38: Amino acid sequence of AAD of *Providencia rettgeri AJ2770*
39: HmaS gene of *Actinoplanes teichomyceticus*
40: HmaS of *Actinoplanes teichomyceticus*
41: Nucleotide sequence of SMDH gene of *Pseudomonas putida*
42: Amino acid sequence of SMDH of *Pseudomonas putida*
43: Nucleotide sequence of BFDC gene of *Pseudomonas putida*
44: Amino acid sequence of BFDC of *Pseudomonas putida*

## Claims

1. A method for producing benzaldehyde, the method comprising the step (A) mentioned below:
(A) a step of producing benzaldehyde by using four enzymes: amino acid deaminase (AAD), 4-hydroxymandelate synthase (HMAS), (S)-mandelate dehydrogenase (SMDH), and benzoylformate decarboxylase (BFDC),
wherein at least a part of the step (A) is carried out in the presence of catalase.

2. The method according to claim 1, wherein the part is a part using HMAS.

3. The method according to claim 1 or 2,
wherein the four enzymes are used in the form of at least one microorganism having these enzymes, and
wherein the at least one microorganism is a single microorganism alone having the four enzymes or a combination of a plurality of microorganisms having the four enzymes as a whole.

4. The method according to any one of claims 1 to 3, wherein the AAD is AAD not generating hydrogen peroxide.

5. The method according to any one of claims 1 to 4, wherein benzaldehyde is produced from L-phenylalanine or a carbon source.

6. The method according to any one of claims 1 to 5, wherein the step (A) comprises the steps (A1) and (A2) mentioned below:
(A1) a step of producing benzoylformate by using the AAD, HMAS, and SMDH;
(A2) a step of converting benzoylformate generated in the step (A1) into benzaldehyde by using the BFDC.

7. The method according to claim 6, wherein the step (A1) comprise the step (1a), (1b), or (1c) mentioned below:
(1a) a step of cultivating at least one microorganism in a culture medium containing the carbon source to generate and accumulate benzoylformate in the culture medium, wherein the at least one microorganism is a single microorganism alone having the AAD, HMAS, and SMDH as well as L-phenylalanine-producing ability or a combination of a plurality of microorganisms as a whole having the AAD, HMAS, and SMDH as well as L-phenylalanine-producing ability;
(1b) a step of cultivating at least one microorganism in a culture medium containing L-phenylalanine to generate and accumulate benzoylformate in the culture medium, wherein the at least one microorganism is a single microorganism alone having the AAD, HMAS, and SMDH or a combination of a plurality of microorganisms as a whole having the AAD, HMAS, and SMDH
(1c) a step of allowing the AAD, HMAS, and SMDH to coexist with L-phenylalanine in a reaction mixture to generate and accumulate benzoylformate in the reaction mixture.

8. The method according to claim 7,
wherein the AAD, HMAS, and SMDH in the step (1c) are used in the form of cells of at least one microorganism having the AAD, HMAS, and SMDH, and
wherein the at least one microorganism is a single microorganism alone having the AAD, HMAS, and SMDH or a combination of a plurality of microorganisms having the AAD, HMAS, and SMDH as a whole.

9. The method according to any one of claims 6 to 8, wherein the step (A2) comprises the step (2a) or (2b) mentioned below:
(2a) a step of cultivating a microorganism having the BFDC in a culture medium containing benzoylformate generated in the step (A1) to generate and accumulate benzaldehyde in the culture medium;
(2b) a step of allowing the BFDC to coexist with benzoylformate generated in the step (A1) in a reaction mixture to generate and accumulate benzaldehyde in the reaction mixture.

10. The method according to claim 9, wherein the BFDC in the step (2b) is used in the form of cells of a microorganism having the BFDC.

11. The method according to any one of claims 8 to 10, wherein the cells are used in the form of a culture broth of the at least one microorganism, cells collected from the culture broth, a processed product thereof, or a combination thereof.

12. The method according to any one of claims 1 to 11, wherein the catalase is a protein defined in (a), (b), or (c) mentioned below:
(a) a protein comprising the amino acid sequence of SEQ ID NO: 4, 6, 8, 10, 12, 14, 16, 18, 20, 22, 24, 26, 28, 30, or 32;
(b) a protein comprising the amino acid sequence of SEQ ID NO: 4, 6, 8, 10, 12, 14, 16, 18, 20, 22, 24, 26, 28, 30, or 32 but including substitution, deletion, insertion, and/or addition of 1 to 10 amino acid residues, and having catalase activity;
(c) a protein comprising an amino acid sequence showing an identity of 90% or higher to the amino acid sequence of SEQ ID NO: 4, 6, 8, 10, 12, 14, 16, 18, 20, 22, 24, 26, 28, 30, or 32, and having catalase activity.

13. The method according to any one of claims 3 to 12, wherein the microorganism(s) each is/are a bacterium or yeast.

14. The method according to any one of claims 3 to 13, wherein the microorganism(s) each is/are a bacterium belonging to the family *Enterobacteriaceae* or a coryneform bacterium.

15. The method according to any one of claims 3 to 14, wherein the microorganism(s) each is/are a bacterium belonging to the genus *Escherichia.*

16. The method according to any one of claims 3 to 15, wherein the microorganism(s) each is/are *Escherichia coli.*

17. The method according to any one of claims 1 to 16, the method further comprising collecting benzaldehyde.


### SEQUENCE LISTING

<110> Ajinomoto Co., Inc.
<120> METHOD FOR PRODUCING BENZALDEHYDE
<130> G112-201058
<150> JP2019-195625
   <151> 2019-10-28
<160> 44
<170> PatentIn version 3.5
<210> 1
   <211> 1086
   <212> DNA
   <213> Actinoplanes teichomyceticus
<400> 1
   atgaccatga cgggccactt tcaggacctg acactggatc atgtccgcat ttactgcgca      60
   gacctggacc ccttgattgc acagtttggc tcctatggtc tggatgtccg tgccgaaggt     120
   gtaggcccgg gagcagagca tagcgtggtc ttgggccatg gggacattcg cctggttctg     180
   acacggccgg gtactggcga tcaccctggt ggcatgtata ccgcccaaca tggctacgga     240
   gtttcggaca ttgcattagg cacagcggat gctgcgggcg cttttcacga ggcggtacgt     300
   cgtggggcac gtccgattgc ggcgccagag cgtaccgccg gtgtggttac ggccagtgtt     360
   gcgggttttg gcgatgtgat ccataccttt gtacagcgtg aaccaggagg cccttggtcg     420
   ctcccgggtc tgaatccggt gcatcgcccg ggtactccgg ggattggact gcgcctggtg     480
   gatcactttg ccgtttgtgt cgaagcaggg cgcttatccg aagtggtgga acactacgaa     540
   cgcgttttcg gcttttctgc catcttcacc gaacgcatcg tggttggaga acaagtgatg     600
   gattcccagg tggttcgtag tgccggtggg gctgtgacct taacggtcgt tgcgccggat     660
   accacacgcc gtccgggtca gatcgatacc ttcctgaagg atcatggcgg tcccggtgtc     720
   cagcacattg cgtttgaaac ggatgatgtc acccgttcag ttggcgccat gtctgacgct     780
   ggtattgagt tccttacgac tccagcagcg tactatgagc ggcttcgcga tcgccttcaa     840
   ctcacgcgcc atagcgtgac cgaactgagc cgcctgaacg tattggctga cgaagatcac     900
   gacggccaac tctatcagat tttcacgaaa agcactcatc ctcgcgggac cctgttcttt     960
   gagatcatcg aacgtgtagg tgcccgcact ttcggttcag gcaacatcca agcgctgtat    1020
   gaagcggtgg aactggacca ggctgccgcg gatggccgtc tcgagcacca ccaccaccac    1080
   cactga                                                               1086
<210> 2
   <211> 361
   <212> PRT
   <213> Actinoplanes teichomyceticus
<400> 2
   Met Thr Met Thr Gly His Phe Gln Asp Leu Thr Leu Asp His Val Arg
   1               5                   10                  15
   Ile Tyr Cys Ala Asp Leu Asp Pro Leu Ile Ala Gln Phe Gly Ser Tyr
               20                  25                  30
   Gly Leu Asp Val Arg Ala Glu Gly Val Gly Pro Gly Ala Glu His Ser
           35                  40                  45
   Val Val Leu Gly His Gly Asp Ile Arg Leu Val Leu Thr Arg Pro Gly
       50                  55                  60
   Thr Gly Asp His Pro Gly Gly Met Tyr Thr Ala Gln His Gly Tyr Gly
   65                  70                  75                  80
   Val Ser Asp Ile Ala Leu Gly Thr Ala Asp Ala Ala Gly Ala Phe His
                   85                  90                  95
   Glu Ala Val Arg Arg Gly Ala Arg Pro Ile Ala Ala Pro Glu Arg Thr
               100                 105                 110
   Ala Gly Val Val Thr Ala Ser Val Ala Gly Phe Gly Asp Val Ile His
                   115                 120                 125
   Thr Phe Val Gln Arg Glu Pro Gly Gly Pro Trp Ser Leu Pro Gly Leu
       130                 135                 140
   Asn Pro Val His Arg Pro Gly Thr Pro Gly Ile Gly Leu Arg Leu Val
   145                 150                 155                 160
   Asp His Phe Ala Val Cys Val Glu Ala Gly Arg Leu Ser Glu Val Val
                   165                 170                 175
   Glu His Tyr Glu Arg Val Phe Gly Phe Ser Ala Ile Phe Thr Glu Arg
               180                 185                 190
   Ile Val Val Gly Glu Gln Val Met Asp Ser Gln Val Val Arg Ser Ala
           195                 200                 205
   Gly Gly Ala Val Thr Leu Thr Val Val Ala Pro Asp Thr Thr Arg Arg
       210                 215                 220
   Pro Gly Gln Ile Asp Thr Phe Leu Lys Asp His Gly Gly Pro Gly Val
   225                 230                 235                 240
   Gln His Ile Ala Phe Glu Thr Asp Asp Val Thr Arg Ser Val Gly Ala
                   245                 250                 255
   Met Ser Asp Ala Gly Ile Glu Phe Leu Thr Thr Pro Ala Ala Tyr Tyr
               260                 265                 270
   Glu Arg Leu Arg Asp Arg Leu Gln Leu Thr Arg His Ser Val Thr Glu
           275                 280                 285
   Leu Ser Arg Leu Asn Val Leu Ala Asp Glu Asp His Asp Gly Gln Leu
       290                 295                 300
   Tyr Gln Ile Phe Thr Lys Ser Thr His Pro Arg Gly Thr Leu Phe Phe
   305                 310                 315                 320
   Glu Ile Ile Glu Arg Val Gly Ala Arg Thr Phe Gly Ser Gly Asn Ile
                   325                 330                 335
   Gln Ala Leu Tyr Glu Ala Val Glu Leu Asp Gln Ala Ala Ala Asp Gly
               340                 345                 350
   Arg Leu Glu His His His His His His
           355                 360
<210> 3
   <211> 2306
   <212> DNA
   <213> Escherichia coli
<400> 3
   gaattcctcg agattttttc aatgtgattt taacttttac ttccagatga ctataatgtg     60
   actaaaaaca aaaccattgt tctggacata taacaccgta aggaggaatc catatgtcaa    120
   cgagcgacga tatccacaac acaactgcga caggcaaatg ccctttccat caagggggtc   180
   acgaccaatc cgccggagct ggcaccacaa cgcgcgattg gtggcccaac cagcttcgtg    240
   tggaccttct gaaccaacac tcgaatcggt ctaatccgct gggtgaagac ttcgactatc    300
   gcaaagaatt tagcaagctg gattactatg gcctaaaaaa ggatctgaaa gcactcctga    360
   ccgaatcaca accgtggtgg cccgcggatt ggggctccta tgcgggactc ttcatccgca    420
   tggcttggca cggtgccgga acgtatcgca gtattgacgg cagaggcggc gcaggtcgtg    480
   ggcaacagcg ttttgctccc ttgaatagct ggcccgataa tgtctcgctc gataaggcgc    540
   gtcgcctgtt atggccgata aaacagaaat atggtcagaa aatcagctgg gcggacctgt    600
   ttatcctggc ggggaacgta gcgctggaaa acagcggctt tcggaccttt ggctttgggg    660
   cgggtcgtga agatgtgtgg gagccagact tggacgtaaa ctggggcgat gaaaaagcct    720
   ggctcacgca ccgtcatccg gaagcgttgg ctaaagcccc gcttggtgca accgagatgg    780
   gtcttattta tgtaaacccg gaaggtccag accatagcgg tgaaccactg agcgcggcag    840
   ccgctattcg cgcaaccttc ggtaatatgg gcatgaacga tgaagagacc gtggctttga    900
   ttgccggggg ccatacgtta ggcaaaacac atggggctgg ccctacctcg aatgttggcc    960
   cagatcctga agcagcaccg attgaggaac agggcctggg ttgggcctct acttatggga   1020
   gtggcgttgg cgctgatgcc atcacgtctg gcctggaagt ggtgtggacc cagactccga   1080
   cccagtggtc aaactacttc ttcgagaacc tgttcaaata cgaatgggtc cagactcgtt   1140
   ctccggcggg cgcgatacag tttgaggcag ttgatgcccc ggagattatc ccagatccgt   1200
   ttgatccgtc caaaaaacgc aaaccgacca tgttggtgac ggatctgaca ttacgctttg   1260
   acccggagtt tgagaagatt agtcgccgct tcctgaatga tccacaggcg tttaacgaag   1320
   catttgccag ggcgtggttc aaactgaccc atcgagatat gggtcctaaa tcccgttaca   1380
   ttggcccgga agttccgaaa gaagacctga tttggcaaga tccgttgccc caaccaatct   1440
   acaatccgac cgaacaggat atcatcgacc tgaaattcgc gattgcagat tcgggtttat   1500
   ccgtcagcga actcgttagc gtggcctggg ctagtgcgtc cacctttcgt ggtggggaca   1560
   agcgcggtgg cgcgaatggt gctcggttag ccctgatgcc tcagcgcgac tgggacgtga   1620
   atgcggccgc ggtccgtgcc ttacctgtac ttgagaaaat tcagaaagaa tcaggaaaag   1680
   cgagcttggc ggatattatc gtgttagcgg gagtggttgg cgtggaaaaa gccgcaagcg   1740
   ccgcaggctt gtcaattcac gtaccgtttg caccgggacg ggttgatgcg cgccaggacc   1800
   agaccgatat cgaaatgttt gaactccttg aaccaattgc cgatggtttt cgtaactatc   1860
   gcgcacgact ggatgtgagt accacggaga gtctgctaat cgacaaggcg cagcaactga   1920
   ctctgactgc tccggaaatg acggcactgg tgggagggat gcgagtcctg ggtgccaact   1980
   tcgatggttc gaaaaatggt gtctttaccg atcgtgtagg cgtattaagt aacgactttt   2040
   ttgttaacct gctggatatg cgctacgaat ggaaggcaac tgatgagtcg aaagagctgt   2100
   tcgaaggtcg tgatcgtgaa accggcgaag ttaagttcac ggcctcccgt gcggatctcg   2160
   tgtttgggag caattcggtc ctgcgtgcgg ttgccgaagt ttatgcgtct tctgatgcgc   2220
   atgagaaatt cgtcaaagac tttgtggccg cttgggtgaa agtcatgaat ctggatcgct   2280
   tcgatctgct ttaagagctc aagctt                                        2306
<210> 4
   <211> 726
   <212> PRT
   <213> Escherichia coli
<400> 4
   Met Ser Thr Ser Asp Asp Ile His Asn Thr Thr Ala Thr Gly Lys Cys
   1               5                   10                  15
   Pro Phe His Gln Gly Gly His Asp Gln Ser Ala Gly Ala Gly Thr Thr
               20                  25                  30
   Thr Arg Asp Trp Trp Pro Asn Gln Leu Arg Val Asp Leu Leu Asn Gln
           35                  40                  45
   His Ser Asn Arg Ser Asn Pro Leu Gly Glu Asp Phe Asp Tyr Arg Lys
       50                  55                  60
   Glu Phe Ser Lys Leu Asp Tyr Tyr Gly Leu Lys Lys Asp Leu Lys Ala
   65                  70                  75                  80
   Leu Leu Thr Glu Ser Gln Pro Trp Trp Pro Ala Asp Trp Gly Ser Tyr
                   85                  90                  95
   Ala Gly Leu Phe Ile Arg Met Ala Trp His Gly Ala Gly Thr Tyr Arg
               100                 105                 110
   Ser Ile Asp Gly Arg Gly Gly Ala Gly Arg Gly Gln Gln Arg Phe Ala
           115                 120                 125
   Pro Leu Asn Ser Trp Pro Asp Asn Val Ser Leu Asp Lys Ala Arg Arg
       130                 135                 140
   Leu Leu Trp Pro Ile Lys Gln Lys Tyr Gly Gln Lys Ile Ser Trp Ala
   145                 150                 155                 160
   Asp Leu Phe Ile Leu Ala Gly Asn Val Ala Leu Glu Asn Ser Gly Phe
                   165                 170                 175
   Arg Thr Phe Gly Phe Gly Ala Gly Arg Glu Asp Val Trp Glu Pro Asp
               180                 185                 190
   Leu Asp Val Asn Trp Gly Asp Glu Lys Ala Trp Leu Thr His Arg His
           195                 200                 205
   Pro Glu Ala Leu Ala Lys Ala Pro Leu Gly Ala Thr Glu Met Gly Leu
       210                 215                 220
   Ile Tyr Val Asn Pro Glu Gly Pro Asp His Ser Gly Glu Pro Leu Ser
   225                 230                 235                 240
   Ala Ala Ala Ala Ile Arg Ala Thr Phe Gly Asn Met Gly Met Asn Asp
                   245                 250                 255
   Glu Glu Thr Val Ala Leu Ile Ala Gly Gly His Thr Leu Gly Lys Thr
               260                 265                 270
   His Gly Ala Gly Pro Thr Ser Asn Val Gly Pro Asp Pro Glu Ala Ala
           275                 280                 285
   Pro Ile Glu Glu Gln Gly Leu Gly Trp Ala Ser Thr Tyr Gly Ser Gly
       290                 295                 300
   Val Gly Ala Asp Ala Ile Thr Ser Gly Leu Glu Val Val Trp Thr Gln
   305                 310                 315                 320
   Thr Pro Thr Gln Trp Ser Asn Tyr Phe Phe Glu Asn Leu Phe Lys Tyr
                   325                 330                 335
   Glu Trp Val Gln Thr Arg Ser Pro Ala Gly Ala Ile Gln Phe Glu Ala
               340                 345                 350
   Val Asp Ala Pro Glu Ile Ile Pro Asp Pro Phe Asp Pro Ser Lys Lys
           355                 360                 365
   Arg Lys Pro Thr Met Leu Val Thr Asp Leu Thr Leu Arg Phe Asp Pro
       370                 375                 380
   Glu Phe Glu Lys Ile Ser Arg Arg Phe Leu Asn Asp Pro Gln Ala Phe
   385                 390                 395                 400
   Asn Glu Ala Phe Ala Arg Ala Trp Phe Lys Leu Thr His Arg Asp Met
                   405                 410                 415
   Gly Pro Lys Ser Arg Tyr Ile Gly Pro Glu Val Pro Lys Glu Asp Leu
               420                 425                 430
   Ile Trp Gln Asp Pro Leu Pro Gln Pro Ile Tyr Asn Pro Thr Glu Gln
           435                 440                 445
   Asp Ile Ile Asp Leu Lys Phe Ala Ile Ala Asp Ser Gly Leu Ser Val
       450                 455                 460
   Ser Glu Leu Val Ser Val Ala Trp Ala Ser Ala Ser Thr Phe Arg Gly
   465                 470                 475                 480
   Gly Asp Lys Arg Gly Gly Ala Asn Gly Ala Arg Leu Ala Leu Met Pro
                   485                 490                 495
   Gln Arg Asp Trp Asp Val Asn Ala Ala Ala Val Arg Ala Leu Pro Val
               500                 505                 510
   Leu Glu Lys Ile Gln Lys Glu Ser Gly Lys Ala Ser Leu Ala Asp Ile
           515                 520                 525
   Ile Val Leu Ala Gly Val Val Gly Val Glu Lys Ala Ala Ser Ala Ala
       530                 535                 540
   Gly Leu Ser Ile His Val Pro Phe Ala Pro Gly Arg Val Asp Ala Arg
   545                 550                 555                 560
   Gln Asp Gln Thr Asp Ile Glu Met Phe Glu Leu Leu Glu Pro Ile Ala
                   565                 570                 575
   Asp Gly Phe Arg Asn Tyr Arg Ala Arg Leu Asp Val Ser Thr Thr Glu
               580                 585                 590
   Ser Leu Leu Ile Asp Lys Ala Gln Gln Leu Thr Leu Thr Ala Pro Glu
           595                 600                 605
   Met Thr Ala Leu Val Gly Gly Met Arg Val Leu Gly Ala Asn Phe Asp
       610                 615                 620
   Gly Ser Lys Asn Gly Val Phe Thr Asp Arg Val Gly Val Leu Ser Asn
   625                 630                 635                 640
   Asp Phe Phe Val Asn Leu Leu Asp Met Arg Tyr Glu Trp Lys Ala Thr
                   645                 650                 655
   Asp Glu Ser Lys Glu Leu Phe Glu Gly Arg Asp Arg Glu Thr Gly Glu
               660                 665                 670
   Val Lys Phe Thr Ala Ser Arg Ala Asp Leu Val Phe Gly Ser Asn Ser
           675                 680                 685
   Val Leu Arg Ala Val Ala Glu Val Tyr Ala Ser Ser Asp Ala His Glu
       690                 695                 700
   Lys Phe Val Lys Asp Phe Val Ala Ala Trp Val Lys Val Met Asn Leu
   705                 710                 715                 720
   Asp Arg Phe Asp Leu Leu
                   725
<210> 5
   <211> 2387
   <212> DNA
   <213> Escherichia coli
<400> 5
   gaattcctcg agattttttc aatgtgattt taacttttac ttccagatga ctataatgtg     60
   actaaaaaca aaaccattgt tctggacata taacaccgta aggaggaatc catatgagtc    120
   aacacaacga gaagaatccg catcagcacc agtctccact gcatgactca agcgaagcca    180
   aaccgggtat ggattccttg gctcctgaag atggctctca tcgtccggca gcggaaccaa    240
   ccccgcctgg cgcgcaaccc actgcgcccg gtagtcttaa agcgcctgat acgcgcaatg    300
   aaaagctgaa tagccttgaa gacgtgcgca aaggctccga aaattatgct ctgacaacga    360
   accaaggtgt ccgtatcgcg gacgatcaga acagcctgcg tgctggcagt cggggtccta    420
   cccttctgga agatttcatt ctgcgtgaaa aaatcacgca cttcgatcac gaacgtatcc    480
   cggaacgcat tgttcatgcc cgtgggtcgg ccgcccatgg ctatttccag ccttataaaa    540
   gcctgtctga cattacgaaa gcggactttc tgagtgatcc gaacaagata acccccgtat    600
   tcgtacgctt ctcaacggtc caagggggtg caggctcggc agatacggtc cgtgatatcc    660
   gcggctttgc aaccaagttc tacactgagg aaggtatctt tgatctggtg ggcaacaaca    720
   ccccgatctt cttcatccag gatgcccata aatttccgga ctttgttcac gcagtgaaac    780
   cggaaccgca ttgggcgatt ccacagggtc agagcgcaca tgacaccttt tgggattatg    840
   tctcgctcca gccggaaacc ttacacaatg tgatgtgggc catgtctgat cgtgggattc    900
   cacggagcta tcgcaccatg gaaggtttcg gtatccacac ctttcgctta attaatgcgg    960
   agggcaaagc caccttcgtc cggttccatt ggaaaccgct ggctggaaag gcatcgttag   1020
   tgtgggacga agctcaaaaa ttgacgggac gtgaccctga ttttcaccgg cgtgaactgt   1080
   gggaggcgat cgaagcgggt gacttcccag agtacgaact gggatttcag ctgatccccg   1140
   aagaagatga gtttaagttt gatttcgact tgttagatcc gacaaaattg atacctgaag   1200
   aactcgtacc agttcagcga gtgggcaaaa tggtgctaaa ccgcaatccg gataactttt   1260
   ttgcggaaaa cgagcaagcg gcattccatc cagggcacat agtgccaggc ttagatttta   1320
   cgaacgatcc gttgctgcaa ggccgtctct tttcttatac cgacactcag atttcgcgac   1380
   tgggtggacc gaactttcac gagattccga ttaatagacc gacttgcccg tatcataatt   1440
   tccagcgcga cgggatgcat agaatgggga ttgacaccaa tcccgcgaac tatgaaccga   1500
   atagcatcaa cgataattgg cctagggaaa ctccgcctgg cccgaaacgc ggcggttttg   1560
   agagctatca ggagcgcgtg gaaggcaata aggtccgcga acgtagtccg tcttttggcg   1620
   aatactactc ccatccacgt cttttctggc tgagtcagac cccgtttgaa cagcgccaca   1680
   ttgtcgatgg ctttagcttt gagctgtcga aagtggttcg tccgtacatt cgagaacgcg   1740
   ttgttgacca acttgcgcac attgatctaa cactggcgca agccgttgcc aaaaacctgg   1800
   gaatcgaatt aacggatgat cagttaaaca tcacaccgcc accggacgtt aatgggctga   1860
   agaaagaccc gtccctctca ctgtacgcta ttccagatgg cgatgtgaaa ggtcgtgtag   1920
   tcgcgatttt attgaacgat gaggtccgca gcgccgacct gttggcaatc ctgaaagcgc   1980
   tgaaagccaa aggtgtacat gccaaactgc tgtattcacg tatgggtgaa gttacagcgg   2040
   atgatggtac cgtgctgccc attgcggcga cctttgcagg agcgccgtcc ctgacggttg   2100
   atgccgtgat cgtgccttgt ggcaacatcg ccgacattgc cgacaatggc gacgcaaact   2160
   actacctgat ggaagcctat aaacatctta aaccgattgc gctggcaggc gatgcgcgca   2220
   aattcaaagc cactatcaaa attgcagatc agggtgaaga gggtattgtg gaggctgatt   2280
   cagcagatgg gtcctttatg gatgagttgc tcaccctcat ggctgctcat cgcgtatgga   2340
   gccgtattcc gaaaatcgac aagattcccg cttaagagct caagctt                 2387
<210> 6
   <211> 753
   <212> PRT
   <213> Escherichia coli
<400> 6
   Met Ser Gln His Asn Glu Lys Asn Pro His Gln His Gln Ser Pro Leu
   1               5                   10                  15
   His Asp Ser Ser Glu Ala Lys Pro Gly Met Asp Ser Leu Ala Pro Glu
               20                  25                  30
   Asp Gly Ser His Arg Pro Ala Ala Glu Pro Thr Pro Pro Gly Ala Gln
           35                  40                  45
   Pro Thr Ala Pro Gly Ser Leu Lys Ala Pro Asp Thr Arg Asn Glu Lys
       50                  55                  60
   Leu Asn Ser Leu Glu Asp Val Arg Lys Gly Ser Glu Asn Tyr Ala Leu
   65                  70                  75                  80
   Thr Thr Asn Gln Gly Val Arg Ile Ala Asp Asp Gln Asn Ser Leu Arg
                   85                  90                  95
   Ala Gly Ser Arg Gly Pro Thr Leu Leu Glu Asp Phe Ile Leu Arg Glu
               100                 105                 110
   Lys Ile Thr His Phe Asp His Glu Arg Ile Pro Glu Arg Ile Val His
           115                 120                 125
   Ala Arg Gly Ser Ala Ala His Gly Tyr Phe Gln Pro Tyr Lys Ser Leu
       130                 135                 140
   Ser Asp Ile Thr Lys Ala Asp Phe Leu Ser Asp Pro Asn Lys Ile Thr
   145 150 155 160
   Pro Val Phe Val Arg Phe Ser Thr Val Gln Gly Gly Ala Gly Ser Ala
   165 170 175
   Asp Thr Val Arg Asp Ile Arg Gly Phe Ala Thr Lys Phe Tyr Thr Glu
   180 185 190
   Glu Gly Ile Phe Asp Leu Val Gly Asn Asn Thr Pro Ile Phe Phe Ile
   195 200 205
   Gln Asp Ala His Lys Phe Pro Asp Phe Val His Ala Val Lys Pro Glu
   210 215 220
   Pro His Trp Ala Ile Pro Gln Gly Gln Ser Ala His Asp Thr Phe Trp
   225 230 235 240
   Asp Tyr Val Ser Leu Gln Pro Glu Thr Leu His Asn Val Met Trp Ala
   245 250 255
   Met Ser Asp Arg Gly Ile Pro Arg Ser Tyr Arg Thr Met Glu Gly Phe
   260 265 270
   Gly Ile His Thr Phe Arg Leu Ile Asn Ala Glu Gly Lys Ala Thr Phe
   275 280 285
   Val Arg Phe His Trp Lys Pro Leu Ala Gly Lys Ala Ser Leu Val Trp
   290 295 300
   Asp Glu Ala Gln Lys Leu Thr Gly Arg Asp Pro Asp Phe His Arg Arg
   305 310 315 320
   Glu Leu Trp Glu Ala Ile Glu Ala Gly Asp Phe Pro Glu Tyr Glu Leu
   325 330 335
   Gly Phe Gln Leu Ile Pro Glu Glu Asp Glu Phe Lys Phe Asp Phe Asp
   340 345 350
   Leu Leu Asp Pro Thr Lys Leu Ile Pro Glu Glu Leu Val Pro Val Gln
   355 360 365
   Arg Val Gly Lys Met Val Leu Asn Arg Asn Pro Asp Asn Phe Phe Ala
   370 375 380
   Glu Asn Glu Gln Ala Ala Phe His Pro Gly His Ile Val Pro Gly Leu
   385 390 395 400
   Asp Phe Thr Asn Asp Pro Leu Leu Gln Gly Arg Leu Phe Ser Tyr Thr
   405 410 415
   Asp Thr Gln Ile Ser Arg Leu Gly Gly Pro Asn Phe His Glu Ile Pro
   420 425 430
   Ile Asn Arg Pro Thr Cys Pro Tyr His Asn Phe Gln Arg Asp Gly Met
   435 440 445
   His Arg Met Gly Ile Asp Thr Asn Pro Ala Asn Tyr Glu Pro Asn Ser
   450 455 460
   Ile Asn Asp Asn Trp Pro Arg Glu Thr Pro Pro Gly Pro Lys Arg Gly
   465 470 475 480
   Gly Phe Glu Ser Tyr Gln Glu Arg Val Glu Gly Asn Lys Val Arg Glu
   485 490 495
   Arg Ser Pro Ser Phe Gly Glu Tyr Tyr Ser His Pro Arg Leu Phe Trp
   500 505 510
   Leu Ser Gln Thr Pro Phe Glu Gln Arg His Ile Val Asp Gly Phe Ser
   515 520 525
   Phe Glu Leu Ser Lys Val Val Arg Pro Tyr Ile Arg Glu Arg Val Val
   530 535 540
   Asp Gln Leu Ala His Ile Asp Leu Thr Leu Ala Gln Ala Val Ala Lys
   545 550 555 560
   Asn Leu Gly Ile Glu Leu Thr Asp Asp Gln Leu Asn Ile Thr Pro Pro
   565 570 575
   Pro Asp Val Asn Gly Leu Lys Lys Asp Pro Ser Leu Ser Leu Tyr Ala
   580 585 590
   Ile Pro Asp Gly Asp Val Lys Gly Arg Val Val Ala Ile Leu Leu Asn
   595 600 605
   Asp Glu Val Arg Ser Ala Asp Leu Leu Ala Ile Leu Lys Ala Leu Lys
   610 615 620
   Ala Lys Gly Val His Ala Lys Leu Leu Tyr Ser Arg Met Gly Glu Val
   625 630 635 640
   Thr Ala Asp Asp Gly Thr Val Leu Pro Ile Ala Ala Thr Phe Ala Gly
                   645                 650                 655
   Ala Pro Ser Leu Thr Val Asp Ala Val Ile Val Pro Cys Gly Asn Ile
               660                 665                 670
   Ala Asp Ile Ala Asp Asn Gly Asp Ala Asn Tyr Tyr Leu Met Glu Ala
           675                 680                 685
   Tyr Lys His Leu Lys Pro Ile Ala Leu Ala Gly Asp Ala Arg Lys Phe
       690                 695                 700
   Lys Ala Thr Ile Lys Ile Ala Asp Gln Gly Glu Glu Gly Ile Val Glu
   705                 710                 715                 720
   Ala Asp Ser Ala Asp Gly Ser Phe Met Asp Glu Leu Leu Thr Leu Met
                   725                 730                 735
   Ala Ala His Arg Val Trp Ser Arg Ile Pro Lys Ile Asp Lys Ile Pro
               740                 745                 750
   Ala
<210> 7
   <211> 1034
   <212> DNA
   <213> Thermus thermophilus
<400> 7
   gaattcctcg agattttttc aatgtgattt taacttttac ttccagatga ctataatgtg      60
   actaaaaaca aaaccattgt tctggacata taacaccgta aggaggaatc catatgtttc     120
   tgcgcataga ccgcttacag atcgaactgc cgatgccgaa agaacaggac ccgaatgccg     180
   cggctgcggt tcaagcgctt cttgggggtc gtttcggtga aatgtccacc ctgatgaact     240
   acatgtatca gagctttaac tttcgtggca aaaaagcgct caaaccctat tacgacctga     300
   ttgcgaacat tgcaaccgaa gaattaggcc acattgaact ggttgcggca acgatcaact     360
   cactgttggc caagaatccg ggcaaagatc tggaagaagg ggttgatccg gcgtctacgc     420
   cattgggctt tgcgaaagat gtgcgcaatg ctgcacactt cattgctggt ggtgccaatt     480
   cgttggtcat gggtgccatg ggtgagcatt ggaatggaga gtacgtgttc acaagcggta     540
   atctcatcct ggacttactg cacaacttct tcttagaagt cgctgcgcgt acccacaaac     600
   tgcgtgtgta tgagatgacg gataaccctg ttgctcgcga gatgattggc tacctgctgg     660
   tgcgtggagg ggtacatgcc gccgcctatg gcaaagcact tgaaagcctc actggcgtgg     720
   agatgaccaa gatgttgccc attccgaaaa tcgacaactc gaaaattccg gaagcgaaaa     780
   agtacatgga tctgggcttc catcgcaacc tgtatcggtt tagtccggag gattatcgag     840
   acctgggcct aatctggaaa ggcgcgagtc cggaagatgg taccgaagtg gtagtcgtag     900
   atggtccacc tactgggggt ccagtctttg atgcaggaca tgatgccgca gagtttgcac     960
   cggaatttca tcctggcgaa ctctatgaaa tcgcgaagaa actgtacgaa aaagccaaat    1020
   aagagctcaa gctt                                                       1034
<210> 8
   <211> 302
   <212> PRT
   <213> Thermus thermophilus
<400> 8
   Met Phe Leu Arg Ile Asp Arg Leu Gln Ile Glu Leu Pro Met Pro Lys
   1               5                   10                  15
   Glu Gln Asp Pro Asn Ala Ala Ala Ala Val Gln Ala Leu Leu Gly Gly
               20                  25                  30
   Arg Phe Gly Glu Met Ser Thr Leu Met Asn Tyr Met Tyr Gln Ser Phe
           35                  40                  45
   Asn Phe Arg Gly Lys Lys Ala Leu Lys Pro Tyr Tyr Asp Leu Ile Ala
       50                  55                  60
   Asn Ile Ala Thr Glu Glu Leu Gly His Ile Glu Leu Val Ala Ala Thr
   65                  70                  75                  80
   Ile Asn Ser Leu Leu Ala Lys Asn Pro Gly Lys Asp Leu Glu Glu Gly
                   85                  90                  95
   Val Asp Pro Ala Ser Thr Pro Leu Gly Phe Ala Lys Asp Val Arg Asn
               100                 105                 110
   Ala Ala His Phe Ile Ala Gly Gly Ala Asn Ser Leu Val Met Gly Ala
           115                 120                 125
   Met Gly Glu His Trp Asn Gly Glu Tyr Val Phe Thr Ser Gly Asn Leu
       130                 135                 140
   Ile Leu Asp Leu Leu His Asn Phe Phe Leu Glu Val Ala Ala Arg Thr
   145                 150                 155                 160
   His Lys Leu Arg Val Tyr Glu Met Thr Asp Asn Pro Val Ala Arg Glu
                   165                 170                 175
   Met Ile Gly Tyr Leu Leu Val Arg Gly Gly Val His Ala Ala Ala Tyr
               180                 185                 190
   Gly Lys Ala Leu Glu Ser Leu Thr Gly Val Glu Met Thr Lys Met Leu
           195                 200                 205
   Pro Ile Pro Lys Ile Asp Asn Ser Lys Ile Pro Glu Ala Lys Lys Tyr
       210                 215                 220
   Met Asp Leu Gly Phe His Arg Asn Leu Tyr Arg Phe Ser Pro Glu Asp
   225                 230                 235                 240
   Tyr Arg Asp Leu Gly Leu Ile Trp Lys Gly Ala Ser Pro Glu Asp Gly
                   245                 250                 255
   Thr Glu Val Val Val Val Asp Gly Pro Pro Thr Gly Gly Pro Val Phe
               260                 265                 270
   Asp Ala Gly His Asp Ala Ala Glu Phe Ala Pro Glu Phe His Pro Gly
           275                 280                 285
   Glu Leu Tyr Glu Ile Ala Lys Lys Leu Tyr Glu Lys Ala Lys
       290                 295                 300
<210> 9
   <211> 1622
   <212> DNA
   <213> Rhodothermus marinus
<400> 9
   gaattcctcg agattttttc aatgtgattt taacttttac ttccagatga ctataatgtg      60
   actaaaaaca aaaccattgt tctggacata taacaccgta aggaggaatc catatggaag     120
   cgcgtaaagc tccgcgcttg actactgccg atggccggcc tattggcgat aaccagaacg     180
   ctctgacagc gggtccacgt ggtcctctcc tgatccaaga tgttcagctg ctggagcaga     240
   tccagcactt caaccgtgaa cgcattcctg aacgtgttgt ccatgcgaaa ggttcaggtg     300
   cgtatggcac cttcaccgtc acaaatgatg tgacccgcta caccaaagcg gcgtttctga     360
   gtgaggttgg caaacagacg gaagtattcg tacgcttttc gaccgttgcg ggtgaacgcg     420
   gagccgccga tgctgagcgc gatgtgcggg gttttgccgt taagttctat accgaagagg     480
   gcaactttga tctggtgggc aacaataccc cggttttctt tgtgcgtgat ccgtacaaat     540
   tccaaatgtt catccattcg cagaaacgac accccaaaac caatctgcgt gacccggaca     600
   tgcaatggga cttttggtcc ttatgcccgg aatccctgca ccaggttacg atcctgttta     660
   gcgatcgcgg cattccggcc tcttatcggc acatgaatgg atatggtagc catacgtatt     720
   ccatgtacaa tgatcgtggc gaactgtttt gggtcaagtt tcactttaaa actcagcaag     780
   gcatcaaatg cttgacggat gaggaagccg cacgcttaat tggcgaagat cgcgagacac     840
   accaacgtga cctgtatgaa gctatcgaac gcggtgacta tccgagatgg acgctgtata     900
   tccaggtgat gaccccggaa caggcggaaa actttcgttg gaacccattc gatctgacga     960
   aagtctggcc acatgccgaa tttccgctta ttgaggtcgg cgtactggaa ctcaaccgca    1020
   atccggagaa ctacttcgcg gaagttgagc aagcggcctt taaaccgagt gcatttgtac    1080
   ctggtattgg cccgtcaccg gacaaaatgc tgcaagcccg tttgatgtct tatgccgatg    1140
   cccatcgtta ccggttaggc gtgaattacc agcagcttaa ggtgaatcgc ccgcgttgtc    1200
   ccgtccatca ttatcagcgt gatggtttca tggcgcaaat tgaagggagc ggtcatccga    1260
   actactttcc gaacagcatt ccagggagtc ctcaggacga tcccatttac aaagaacccg    1320
   catggcactt aggggaagtg atcgtggacc gctatgactc gcgcaaagac catgatgact    1380
   acactcaagc tgggaatctc taccgccttt tcgacgaagg ccagaaagat cgcctcgcac    1440
   gcgcaatagc ggcatctctg ggacaggctc gtctggaggt gcagaagagg cagcttggac    1500
   atttctatcg tgccgacgtc gattatggcc gacgagtggc gcgtgcattg ggttttgatc    1560
   cagcagcgat tgaagcggaa ctagggatag atgctagcgt agcagggtaa gagctcaagc    1620
   tt                                                                   1622
<210> 10
   <211> 498
   <212> PRT
   <213> Rhodothermus marinus
<400> 10
   Met Glu Ala Arg Lys Ala Pro Arg Leu Thr Thr Ala Asp Gly Arg Pro
   1               5                   10                  15
   Ile Gly Asp Asn Gln Asn Ala Leu Thr Ala Gly Pro Arg Gly Pro Leu
               20                  25                  30
   Leu Ile Gln Asp Val Gln Leu Leu Glu Gln Ile Gln His Phe Asn Arg
           35                  40                  45
   Glu Arg Ile Pro Glu Arg Val Val His Ala Lys Gly Ser Gly Ala Tyr
       50                  55                  60
   Gly Thr Phe Thr Val Thr Asn Asp Val Thr Arg Tyr Thr Lys Ala Ala
   65                  70                  75                  80
   Phe Leu Ser Glu Val Gly Lys Gln Thr Glu Val Phe Val Arg Phe Ser
                   85                  90                  95
   Thr Val Ala Gly Glu Arg Gly Ala Ala Asp Ala Glu Arg Asp Val Arg
               100                 105                 110
   Gly Phe Ala Val Lys Phe Tyr Thr Glu Glu Gly Asn Phe Asp Leu Val
           115                 120                 125
   Gly Asn Asn Thr Pro Val Phe Phe Val Arg Asp Pro Tyr Lys Phe Gln
       130                 135                 140
   Met Phe Ile His Ser Gln Lys Arg His Pro Lys Thr Asn Leu Arg Asp
   145                 150                 155                 160
   Pro Asp Met Gln Trp Asp Phe Trp Ser Leu Cys Pro Glu Ser Leu His
                   165                 170                 175
   Gln Val Thr Ile Leu Phe Ser Asp Arg Gly Ile Pro Ala Ser Tyr Arg
               180                 185                 190
   His Met Asn Gly Tyr Gly Ser His Thr Tyr Ser Met Tyr Asn Asp Arg
           195                 200                 205
   Gly Glu Leu Phe Trp Val Lys Phe His Phe Lys Thr Gln Gln Gly Ile
       210                 215                 220
   Lys Cys Leu Thr Asp Glu Glu Ala Ala Arg Leu Ile Gly Glu Asp Arg
   225                 230                 235                 240
   Glu Thr His Gln Arg Asp Leu Tyr Glu Ala Ile Glu Arg Gly Asp Tyr
                   245                 250                 255
   Pro Arg Trp Thr Leu Tyr Ile Gln Val Met Thr Pro Glu Gln Ala Glu
               260                 265                 270
   Asn Phe Arg Trp Asn Pro Phe Asp Leu Thr Lys Val Trp Pro His Ala
           275                 280                 285
   Glu Phe Pro Leu Ile Glu Val Gly Val Leu Glu Leu Asn Arg Asn Pro
       290                 295                 300
   Glu Asn Tyr Phe Ala Glu Val Glu Gln Ala Ala Phe Lys Pro Ser Ala
   305                 310                 315                 320
   Phe Val Pro Gly Ile Gly Pro Ser Pro Asp Lys Met Leu Gln Ala Arg
                   325                 330                 335
   Leu Met Ser Tyr Ala Asp Ala His Arg Tyr Arg Leu Gly Val Asn Tyr
               340                 345                 350
   Gln Gln Leu Lys Val Asn Arg Pro Arg Cys Pro Val His His Tyr Gln
           355                 360                 365
   Arg Asp Gly Phe Met Ala Gln Ile Glu Gly Ser Gly His Pro Asn Tyr
       370                 375                 380
   Phe Pro Asn Ser Ile Pro Gly Ser Pro Gln Asp Asp Pro Ile Tyr Lys
   385                 390                 395                 400
   Glu Pro Ala Trp His Leu Gly Glu Val Ile Val Asp Arg Tyr Asp Ser
                   405                 410                 415
   Arg Lys Asp His Asp Asp Tyr Thr Gln Ala Gly Asn Leu Tyr Arg Leu
               420                 425                 430
   Phe Asp Glu Gly Gln Lys Asp Arg Leu Ala Arg Ala Ile Ala Ala Ser
           435                 440                 445
   Leu Gly Gln Ala Arg Leu Glu Val Gln Lys Arg Gln Leu Gly His Phe
       450                 455                 460
   Tyr Arg Ala Asp Val Asp Tyr Gly Arg Arg Val Ala Arg Ala Leu Gly
   465                 470                 475                 480
   Phe Asp Pro Ala Ala Ile Glu Ala Glu Leu Gly Ile Asp Ala Ser Val
                   485                 490                 495
   Ala Gly
<210> 11
   <211> 1676
   <212> DNA
   <213> Corynebacterium glutamicum
<400> 11
   gaattcctcg agattttttc aatgtgattt taacttttac ttccagatga ctataatgtg      60
   actaaaaaca aaaccattgt tctggacata taacaccgta aggaggaatc catatgagcg     120
   aaaaatccgc ggctgaccag atagttgacc gtggtatgcg tccgaaactc tcgggcaata     180
   cgacgcgtca taacggtgcg ccagtaccaa gcgagaacat ttcggcgact gctggacctc     240
   agggacctaa cgttttgaac gatatccacc tgatcgagaa attagcccac tttaatcgcg     300
   aaaatgtgcc ggaacgcatt ccgcacgcaa aagggcatgg ggcgtttggc gaactgcaca     360
   ttacggagga tgtcagcgaa tacaccaaag ccgatctgtt ccagcctggt aaagtcaccc     420
   ccctggctgt gcgattcagt accgtcgctg gtgaacaagg ctcaccggat acatggcgtg     480
   atgttcatgg ttttgcgctg cgtttttaca ccgaggaagg taactatgac attgtgggca     540
   acaacactcc cacctttttc ctgagagatg ggatgaagtt tcccgacttt atccatagcc     600
   agaaacgcct gaataaaaac ggcttacggg atgccgatat gcaatgggat ttctggactc     660
   gtgcgccaga aagcgcacac caggttacgt atttgatggg agatcgtggc accccaaaaa     720
   cctcccgcca tcaggacggt tttggtagcc atacctttca gtggataaat gcagaaggaa     780
   aaccggtatg ggtgaagtac cacttcaaaa cgcgccaagg ttgggattgc tttacagatg     840
   ccgaggcagc caaagtcgca ggcgaaaacg ccgattatca gcgcgaagat ctctacaacg     900
   ctatcgagaa tggcgacttt ccgatctggg acgtgaaagt ccaaattatg ccgtttgaag     960
   atgcggagaa ttatcgctgg aatccgttcg atctgacgaa gacctggtca cagaaagact    1020
   atcccctgat cccggttggt tacttcatcc tgaaccgtaa tccgcggaat ttctttgcgc    1080
   agattgagca gattgcgcta gatccgggca atattgtgcc tggcgttggt ctctctccag    1140
   atcgaatgct tcaggccagg attttcgcct atgcagatca gcagcgctat cgcattggcg    1200
   cgaattatcg tgatttgccg gttaatcgtc cgatcaacga ggtgaatacg tatagtcgtg    1260
   aaggttccat gcagtacatt tttgatgccg aaggggaacc gtcatactcg ccgaaccgct    1320
   atgataaagg cgcaggctat ctggacaatg ggaccgatag ttcgagcaac catacctctt    1380
   atggtcaagc ggacgacatc tacgtcaacc cagatccgca tggcactgac ttagtacgcg    1440
   cagcgtatgt gaaacatcag gatgacgacg atttcatcca accggggatt ctttaccgcg    1500
   aagtgttaga tgaaggcgaa aaagaacggc ttgccgacaa catctctaac gccatgcaag    1560
   gcattagtga agcgacagaa cctcgtgtgt acgattactg gaataacgtg gacgaaaacc    1620
   tgggtgctcg cgtaaaggag ctgtatctgc aaaagaaagc gtaagagctc aagctt        1676
<210> 12
   <211> 516
   <212> PRT
   <213> Corynebacterium glutamicum
<400> 12
   Met Ser Glu Lys Ser Ala Ala Asp Gln Ile Val Asp Arg Gly Met Arg
   1               5                   10                  15
   Pro Lys Leu Ser Gly Asn Thr Thr Arg His Asn Gly Ala Pro Val Pro
               20                  25                  30
   Ser Glu Asn Ile Ser Ala Thr Ala Gly Pro Gln Gly Pro Asn Val Leu
           35                  40                  45
   Asn Asp Ile His Leu Ile Glu Lys Leu Ala His Phe Asn Arg Glu Asn
       50                  55                  60
   Val Pro Glu Arg Ile Pro His Ala Lys Gly His Gly Ala Phe Gly Glu
   65                  70                  75                  80
   Leu His Ile Thr Glu Asp Val Ser Glu Tyr Thr Lys Ala Asp Leu Phe
                   85                  90                  95
   Gln Pro Gly Lys Val Thr Pro Leu Ala Val Arg Phe Ser Thr Val Ala
                   100                 105                 110
   Gly Glu Gln Gly Ser Pro Asp Thr Trp Arg Asp Val His Gly Phe Ala
               115                 120                 125
   Leu Arg Phe Tyr Thr Glu Glu Gly Asn Tyr Asp Ile Val Gly Asn Asn
       130                 135                 140
   Thr Pro Thr Phe Phe Leu Arg Asp Gly Met Lys Phe Pro Asp Phe Ile
   145                 150                 155                 160
   His Ser Gln Lys Arg Leu Asn Lys Asn Gly Leu Arg Asp Ala Asp Met
                   165                 170                 175
   Gln Trp Asp Phe Trp Thr Arg Ala Pro Glu Ser Ala His Gln Val Thr
               180                 185                 190
   Tyr Leu Met Gly Asp Arg Gly Thr Pro Lys Thr Ser Arg His Gln Asp
           195                 200                 205
   Gly Phe Gly Ser His Thr Phe Gln Trp Ile Asn Ala Glu Gly Lys Pro
       210                 215                 220
   Val Trp Val Lys Tyr His Phe Lys Thr Arg Gln Gly Trp Asp Cys Phe
   225                 230                 235                 240
   Thr Asp Ala Glu Ala Ala Lys Val Ala Gly Glu Asn Ala Asp Tyr Gln
                   245                 250                 255
   Arg Glu Asp Leu Tyr Asn Ala Ile Glu Asn Gly Asp Phe Pro Ile Trp
               260                 265                 270
   Asp Val Lys Val Gln Ile Met Pro Phe Glu Asp Ala Glu Asn Tyr Arg
           275                 280                 285
   Trp Asn Pro Phe Asp Leu Thr Lys Thr Trp Ser Gln Lys Asp Tyr Pro
       290                 295                 300
   Leu Ile Pro Val Gly Tyr Phe Ile Leu Asn Arg Asn Pro Arg Asn Phe
   305                 310                 315                 320
   Phe Ala Gln Ile Glu Gln Ile Ala Leu Asp Pro Gly Asn Ile Val Pro
                   325                 330                 335
   Gly Val Gly Leu Ser Pro Asp Arg Met Leu Gln Ala Arg Ile Phe Ala
               340                 345                 350
   Tyr Ala Asp Gln Gln Arg Tyr Arg Ile Gly Ala Asn Tyr Arg Asp Leu
           355                 360                 365
   Pro Val Asn Arg Pro Ile Asn Glu Val Asn Thr Tyr Ser Arg Glu Gly
       370                 375                 380
   Ser Met Gln Tyr Ile Phe Asp Ala Glu Gly Glu Pro Ser Tyr Ser Pro
   385                 390                 395                 400
   Asn Arg Tyr Asp Lys Gly Ala Gly Tyr Leu Asp Asn Gly Thr Asp Ser
                   405                 410                 415
   Ser Ser Asn His Thr Ser Tyr Gly Gln Ala Asp Asp Ile Tyr Val Asn
               420                 425                 430
   Pro Asp Pro His Gly Thr Asp Leu Val Arg Ala Ala Tyr Val Lys His
           435                 440                 445
   Gln Asp Asp Asp Asp Phe Ile Gln Pro Gly Ile Leu Tyr Arg Glu Val
       450                 455                 460
   Leu Asp Glu Gly Glu Lys Glu Arg Leu Ala Asp Asn Ile Ser Asn Ala
   465                 470                 475                 480
   Met Gln Gly Ile Ser Glu Ala Thr Glu Pro Arg Val Tyr Asp Tyr Trp
                   485                 490                 495
   Asn Asn Val Asp Glu Asn Leu Gly Ala Arg Val Lys Glu Leu Tyr Leu
               500                 505                 510
   Gln Lys Lys Ala
           515
<210> 13
   <211> 1577
   <212> DNA
   <213> Bacillus subtilis
<400> 13
   gaattcctcg agattttttc aatgtgattt taacttttac ttccagatga ctataatgtg      60
   actaaaaaca aaaccattgt tctggacata taacaccgta aggaggaatc catatgtcgt     120
   cgaacaaact gaccacgtct tggggtgctc cggtgggcga taatcagaac tcaatgacgg     180
   ctggctccag agggccaacg cttattcagg atgttcatct gctggaaaaa ctggcacact     240
   ttaaccgcga gcgtgtaccg gaacgtgttg ttcacgcgaa aggtgcgggt gctcatggct     300
   atttcgaggt gaccaatgat gtaacgaagt acaccaaagc cgcgtttctg agtgaagtgg     360
   ggaaacgtac tccactcttc attcgcttta gcactgtggc cggagaactg ggctccgccg     420
   atacagttcg tgatccacgt gggtttgcag tcaaattcta taccgaagaa ggcaattacg     480
   acatcgtcgg gaataacact ccggtattct ttatacgcga tgcgatcaag ttccctgact     540
   tcattcacac acagaagcgc gaccctaaaa cccacttgaa aaatccgacc gctgtctggg     600
   acttttggag tttgagcccg gaaagtctgc atcaggtgac catcctgatg agcgatcgcg      660
   gtattccggc gactttacgt cacatgcatg gatttggctc acatacgttc aagtggacga     720
   atgccgaagg cgaaggcgtt tggatcaaat accacttcaa aactgaacaa ggtgtgaaga     780
   acctggacgt aaatacagcg gccaaaattg ctggcgaaaa ccccgattat cataccgagg     840
   acctctttaa tgcgattgag aatggtgatt acccagcgtg gaaactgtat gtccagataa     900
   tgccgttgga agacgccaac acctatcgtt tcgatccgtt tgacgtgacc aaagtgtgga     960
   gccagaaaga ctacccgcta atcgaagtgg gcaggatggt gctggatcgt aatccggaaa    1020
   actactttgc agaagtggaa caagctacct ttagccccgg aaccttagta ccgggcattg    1080
   atgtctcacc cgacaaaatg ctgcaagggc ggttatttgc gtatcacgat gcacatcggt    1140
   atcgcgttgg cgcaaatcat caggcgcttc cgatcaatcg cgcgcgcaac aaggtcaaca    1200
   actatcagcg agatggtcaa atgcgctttg atgataacgg tggaggcagc gtctactatg    1260
   agcctaacag ttttggtggt ccgaaagaat ctccagaaga caaacaggcg gcatatcctg    1320
   ttcagggtat tgccgattcc gtgtcgtacg atcactatga tcattacacg caagcgggtg    1380
   atctttatcg actcatgtcg gaagacgagc gtacacgtct ggttgaaaac attgtgaacg    1440
   ccatgaaacc cgtcgagaag gaggagatta aactgcgcca aatcgagcat ttctacaaag    1500
   ccgatccgga atatggcaaa cgcgttgcag aaggcttagg gttgccgatc aaaaaagact    1560
   cttaagagct caagctt                                                   1577
<210> 14
   <211> 483
   <212> PRT
   <213> Bacillus subtilis
<400> 14
   Met Ser Ser Asn Lys Leu Thr Thr Ser Trp Gly Ala Pro Val Gly Asp
   1               5                   10                  15
   Asn Gln Asn Ser Met Thr Ala Gly Ser Arg Gly Pro Thr Leu Ile Gln
               20                  25                  30
   Asp Val His Leu Leu Glu Lys Leu Ala His Phe Asn Arg Glu Arg Val
           35                  40                  45
   Pro Glu Arg Val Val His Ala Lys Gly Ala Gly Ala His Gly Tyr Phe
       50                  55                  60
   Glu Val Thr Asn Asp Val Thr Lys Tyr Thr Lys Ala Ala Phe Leu Ser
   65                  70                  75                  80
   Glu Val Gly Lys Arg Thr Pro Leu Phe Ile Arg Phe Ser Thr Val Ala
                   85                  90                  95
   Gly Glu Leu Gly Ser Ala Asp Thr Val Arg Asp Pro Arg Gly Phe Ala
               100                 105                 110
   Val Lys Phe Tyr Thr Glu Glu Gly Asn Tyr Asp Ile Val Gly Asn Asn
           115                 120                 125
   Thr Pro Val Phe Phe Ile Arg Asp Ala Ile Lys Phe Pro Asp Phe Ile
       130                 135                 140
   His Thr Gln Lys Arg Asp Pro Lys Thr His Leu Lys Asn Pro Thr Ala
   145                 150                 155                 160
   Val Trp Asp Phe Trp Ser Leu Ser Pro Glu Ser Leu His Gln Val Thr
                   165                 170                 175
   Ile Leu Met Ser Asp Arg Gly Ile Pro Ala Thr Leu Arg His Met His
               180                 185                 190
   Gly Phe Gly Ser His Thr Phe Lys Trp Thr Asn Ala Glu Gly Glu Gly
           195                 200                 205
   Val Trp Ile Lys Tyr His Phe Lys Thr Glu Gln Gly Val Lys Asn Leu
       210                 215                 220
   Asp Val Asn Thr Ala Ala Lys Ile Ala Gly Glu Asn Pro Asp Tyr His
   225                 230                 235                 240
   Thr Glu Asp Leu Phe Asn Ala Ile Glu Asn Gly Asp Tyr Pro Ala Trp
                   245                 250                 255
   Lys Leu Tyr Val Gln Ile Met Pro Leu Glu Asp Ala Asn Thr Tyr Arg
               260                 265                 270
   Phe Asp Pro Phe Asp Val Thr Lys Val Trp Ser Gln Lys Asp Tyr Pro
           275                 280                 285
   Leu Ile Glu Val Gly Arg Met Val Leu Asp Arg Asn Pro Glu Asn Tyr
       290                 295                 300
   Phe Ala Glu Val Glu Gln Ala Thr Phe Ser Pro Gly Thr Leu Val Pro
   305                 310                 315                 320
   Gly Ile Asp Val Ser Pro Asp Lys Met Leu Gln Gly Arg Leu Phe Ala
                   325                 330                 335
   Tyr His Asp Ala His Arg Tyr Arg Val Gly Ala Asn His Gln Ala Leu
               340                 345                 350
   Pro Ile Asn Arg Ala Arg Asn Lys Val Asn Asn Tyr Gln Arg Asp Gly
           355                 360                 365
   Gln Met Arg Phe Asp Asp Asn Gly Gly Gly Ser Val Tyr Tyr Glu Pro
       370                 375                 380
   Asn Ser Phe Gly Gly Pro Lys Glu Ser Pro Glu Asp Lys Gln Ala Ala
   385                 390                 395                 400
   Tyr Pro Val Gln Gly Ile Ala Asp Ser Val Ser Tyr Asp His Tyr Asp
                   405                 410                 415
   His Tyr Thr Gln Ala Gly Asp Leu Tyr Arg Leu Met Ser Glu Asp Glu
               420                 425                 430
   Arg Thr Arg Leu Val Glu Asn Ile Val Asn Ala Met Lys Pro Val Glu
           435                 440                 445
   Lys Glu Glu Ile Lys Leu Arg Gln Ile Glu His Phe Tyr Lys Ala Asp
       450                 455                 460
   Pro Glu Tyr Gly Lys Arg Val Ala Glu Gly Leu Gly Leu Pro Ile Lys
   465                 470                 475                 480
   Lys Asp Ser
<210> 15
   <211> 1658
   <212> DNA
   <213> Pseudomonas syringiae
<400> 15
   gaattcctcg agattttttc aatgtgattt taacttttac ttccagatga ctataatgtg      60
   actaaaaaca aaaccattgt tctggacata taacaccgta aggaggaatc catatgccct    120
   tactgaattg gtcacgtcac atggtgtgcc tgacagcagc tgggctgatt acagtgccga    180
   cggtttatgc gactgatacg ctgacccgtg ataatggcgc ggttgtgggt gataaccaaa    240
   attcgcaaac tgcgggagca cagggtcctg tccttctaca agatgtccag ttgcttcaga    300
   aattgcagcg ctttgaccgc gaacgcattc cggagcgtgt ggtacatgct cgtggaacag    360
   gcgtcaaagg cgaatttact gcaagtgccg acataagtga tctgagcaag gccaccgtgt    420
   tcaaaagtgg cgaaaaaacg ccggtatttg tgcgctttag ctctgttgtg catgggaatc    480
   acagtccgga aacgttgcgt gatccgcacg gttttgcgac caaattttac accgccgatg    540
   gcaattggga cctcgtgggc aacaactttc cgacgttttt cattcgagat gcgatcaagt    600
   ttcccgacat ggtccatgca ttcaaacctg atccgcgtac caacctggat aacgatagcc    660
   ggaggttcga ttttttcagc catgtcccgg aagcgactcg caccctgacg ttactgtatt    720
   cgaacgaagg taccccagca gggtatcggt ttatggatgg taacggggtt catgcctata    780
   agctggtcaa cgctaaaggc gaggtgcatt acgttaaatt ccactggaag agcctgcaag    840
   gcatcaaaaa cctcgatcca aaagaggttg cccaggtaca gtctaaagat tactcccatc    900
   tgacaaatga cctggtcggt gcgatcaaaa aaggcgactt cccgaaatgg gatctgtatg    960
   tgcaggtgtt gaaaccggaa gaactcgcca aattcgactt tgatccgctg gatgcgacca   1020
   agatttggcc tgatgtaccg gagaaaaaaa ttggccaaat ggttcttaac aagaacgtgg   1080
   acaatttctt ccaggaaacg gaacaagtgg caatggctcc ggctaatctg gttccgggaa   1140
   ttgaaccgag cgaagaccgc cttctacagg gtcgagtgtt cagttatgca gacacccaga   1200
   tgtatcgttt aggcgcgatt ggcctctctc tgcccgttaa tcagcctaaa gtcgccgtta   1260
   acaacggcaa tcaagatggt gccctgaata ccggtcacac cactagcggc gtaaattacg   1320
   agccatcacg tttggaacca agaccagccg atgataaagc tcgttactcg gaattacccc   1380
   tgtccggtac gacccagcag gcgaaaatca ctcgcgaaca gaactttaag caggcgggag   1440
   atctgtttcg ctcgtatagc gccaaagaaa aaaccgactt agttcaacgc tttggtgagt   1500
   cactggcgga cacgcatacc gagtcgaaaa acatcatgct gtcagtgttg tacaaagagg   1560
   accgtcacta tggtacccgc gtagcggaag tcgcgaaagg ggacttatcc aaggtgaaat   1620
   ctctggccgc atccctcaaa gattaagagc tcaagctt                            1658
<210> 16
   <211> 510
   <212> PRT
   <213> Pseudomonas syringiae
<400> 16
   Met Pro Leu Leu Asn Trp Ser Arg His Met Val Cys Leu Thr Ala Ala
   1               5                   10                  15
   Gly Leu Ile Thr Val Pro Thr Val Tyr Ala Thr Asp Thr Leu Thr Arg
               20                  25                  30
   Asp Asn Gly Ala Val Val Gly Asp Asn Gln Asn Ser Gln Thr Ala Gly
           35                  40                  45
   Ala Gln Gly Pro Val Leu Leu Gln Asp Val Gln Leu Leu Gln Lys Leu
       50                  55                  60
   Gln Arg Phe Asp Arg Glu Arg Ile Pro Glu Arg Val Val His Ala Arg
   65                  70                  75                  80
   Gly Thr Gly Val Lys Gly Glu Phe Thr Ala Ser Ala Asp Ile Ser Asp
                   85                  90                  95
   Leu Ser Lys Ala Thr Val Phe Lys Ser Gly Glu Lys Thr Pro Val Phe
               100                 105                 110
   Val Arg Phe Ser Ser Val Val His Gly Asn His Ser Pro Glu Thr Leu
           115                 120                 125
   Arg Asp Pro His Gly Phe Ala Thr Lys Phe Tyr Thr Ala Asp Gly Asn
       130                 135                 140
   Trp Asp Leu Val Gly Asn Asn Phe Pro Thr Phe Phe Ile Arg Asp Ala
   145                 150                 155                 160
   Ile Lys Phe Pro Asp Met Val His Ala Phe Lys Pro Asp Pro Arg Thr
                   165                 170                 175
   Asn Leu Asp Asn Asp Ser Arg Arg Phe Asp Phe Phe Ser His Val Pro
               180                 185                 190
   Glu Ala Thr Arg Thr Leu Thr Leu Leu Tyr Ser Asn Glu Gly Thr Pro
           195                 200                 205
   Ala Gly Tyr Arg Phe Met Asp Gly Asn Gly Val His Ala Tyr Lys Leu
       210                 215                 220
   Val Asn Ala Lys Gly Glu Val His Tyr Val Lys Phe His Trp Lys Ser
   225                 230                 235                 240
   Leu Gln Gly Ile Lys Asn Leu Asp Pro Lys Glu Val Ala Gln Val Gln
                   245                 250                 255
   Ser Lys Asp Tyr Ser His Leu Thr Asn Asp Leu Val Gly Ala Ile Lys
               260                 265                 270
   Lys Gly Asp Phe Pro Lys Trp Asp Leu Tyr Val Gln Val Leu Lys Pro
           275                 280                 285
   Glu Glu Leu Ala Lys Phe Asp Phe Asp Pro Leu Asp Ala Thr Lys Ile
       290                 295                 300
   Trp Pro Asp Val Pro Glu Lys Lys Ile Gly Gln Met Val Leu Asn Lys
   305                 310                 315                 320
   Asn Val Asp Asn Phe Phe Gln Glu Thr Glu Gln Val Ala Met Ala Pro
                   325                 330                 335
   Ala Asn Leu Val Pro Gly Ile Glu Pro Ser Glu Asp Arg Leu Leu Gln
               340                 345                 350
   Gly Arg Val Phe Ser Tyr Ala Asp Thr Gln Met Tyr Arg Leu Gly Ala
           355                 360                 365
   Ile Gly Leu Ser Leu Pro Val Asn Gln Pro Lys Val Ala Val Asn Asn
       370                 375                 380
   Gly Asn Gln Asp Gly Ala Leu Asn Thr Gly His Thr Thr Ser Gly Val
   385                 390                 395                 400
   Asn Tyr Glu Pro Ser Arg Leu Glu Pro Arg Pro Ala Asp Asp Lys Ala
                   405                 410                 415
   Arg Tyr Ser Glu Leu Pro Leu Ser Gly Thr Thr Gln Gln Ala Lys Ile
               420                 425                 430
   Thr Arg Glu Gln Asn Phe Lys Gln Ala Gly Asp Leu Phe Arg Ser Tyr
           435                 440                 445
   Ser Ala Lys Glu Lys Thr Asp Leu Val Gln Arg Phe Gly Glu Ser Leu
       450                 455                 460
   Ala Asp Thr His Thr Glu Ser Lys Asn Ile Met Leu Ser Val Leu Tyr
   465                 470                 475                 480
   Lys Glu Asp Arg His Tyr Gly Thr Arg Val Ala Glu Val Ala Lys Gly
                   485                 490                 495
   Asp Leu Ser Lys Val Lys Ser Leu Ala Ala Ser Leu Lys Asp
               500                 505                 510
<210> 17
   <211> 1667
   <212> DNA
   <213> Pseudomonas putida
<400> 17
   gaattcctcg agattttttc aatgtgattt taacttttac ttccagatga ctataatgtg      60
   actaaaaaca aaaccattgt tctggacata taacaccgta aggaggaatc catatgaccc     120
   cggtgctcaa acacttccag ccaggcaggt tgcttgtcgc tgcgtcgtta gctgcaagcc     180
   tcttgacgat gagcgtgcaa gccgcgacct taacgcgcga ttcaggagca cctgtgggcg     240
   acaaccaaaa ctctcaaacc gcaggtgcaa atggtgcggt actgctacag gatgcaaatc     300
   tgctgcaaaa actccagcgc tttgatcgcg aacggatacc ggaacgtgtg gtccatgcac     360
   gtgggactgg agtgcatggc gaatttacag cctcagccga tatttccgat ctcagcatcg     420
   cgaaagtatt tcgtccgggt gagaaaacgc cggtgtttgt ccgctttagt gccgttgtgc     480
   atggcaatca tagccctgaa acactgcgcg atccgcgagg ttttgccacg aagttctata     540
   ccgccgatgg taattgggac ctggtcggta acaacttccc tacattcttc attcgtgatg     600
   cgatcaagtt tccggacatg gtacatgcgt tcaaaccaga tccgcgtacc aatcttgatg     660
   acgactcacg tcgcttcgac tttttcagcc atgtaccaga agcgactcgt accctgacct     720
   tactgtactc gaacgaaggt acaccggcta gctatcgcga aatggacggg aacagtgttc     780
   acgcatacaa actggttaac tcgaaaggcc aagttcacta cgtgaagttt cattggaagt     840
   cattacaggg ccagaaaaat ctggacccca agcaggtcga acaagtgcag ggccgtgatt     900
   atagtcacat gaccaatgat ctggtgagtg ccatcaaaga tggcaaattt ccgaaatggg     960
   acctgtatat ccaggttctg aaaccggaag atctggcgaa atttgatttt gacccactgg    1020
   atgcaaccaa aatttggccg aatgtccctg aacgtaagat tgggcagatg gtgctgaatc    1080
   ggaacgtgga caatttcttc caggagactg aacaggttgc catggctccg tcgaacctgg    1140
   ttccggggat tgagccttcc gaggatcgtt tgctgcaagg ccgtctattt gcgtatgccg    1200
   atacgcagat gtaccgcatt ggtgcgaatg gtctgggcct cccggtgaac cagccccgct    1260
   tacccgtcaa caacgtcaac caggatggcc aggcaaatgc tggtcacacc accactggcg    1320
   ttaactacca gccctccaga ttacagccac gcgaggaaca ggcgtctgct cgatatgtgc    1380
   cgactccgct ggtaggatct acccaacagg cgaaaattca gcgcgaacaa aacttcaaac    1440
   aaacggggga gttgtttcgg tcgtataaca agaaagaaca gacggatctg atcaatagcc    1500
   ttggccaggc gttggcagtt acggatgaag agagtcgcta tatcatgctg agctttttct     1560
   acaaagcgga ttccgactat ggtgctggac ttgcctctgt tgcgaaagcc gatctgaaac    1620
   gtgtacagca attggccgcg aaactgcaag actaagagct caagctt                  1667
<210> 18
   <211> 513
   <212> PRT
   <213> Pseudomonas putida
<400> 18
   Met Thr Pro Val Leu Lys His Phe Gln Pro Gly Arg Leu Leu Val Ala
   1               5                   10                  15
   Ala Ser Leu Ala Ala Ser Leu Leu Thr Met Ser Val Gln Ala Ala Thr
               20                  25                  30
   Leu Thr Arg Asp Ser Gly Ala Pro Val Gly Asp Asn Gln Asn Ser Gln
           35                  40                  45
   Thr Ala Gly Ala Asn Gly Ala Val Leu Leu Gln Asp Ala Asn Leu Leu
       50                  55                  60
   Gln Lys Leu Gln Arg Phe Asp Arg Glu Arg Ile Pro Glu Arg Val Val
   65                  70                  75                  80
   His Ala Arg Gly Thr Gly Val His Gly Glu Phe Thr Ala Ser Ala Asp
                   85                  90                  95
   Ile Ser Asp Leu Ser Ile Ala Lys Val Phe Arg Pro Gly Glu Lys Thr
               100                 105                 110
   Pro Val Phe Val Arg Phe Ser Ala Val Val His Gly Asn His Ser Pro
           115                 120                 125
   Glu Thr Leu Arg Asp Pro Arg Gly Phe Ala Thr Lys Phe Tyr Thr Ala
       130                 135                 140
   Asp Gly Asn Trp Asp Leu Val Gly Asn Asn Phe Pro Thr Phe Phe Ile
   145                 150                 155                 160
   Arg Asp Ala Ile Lys Phe Pro Asp Met Val His Ala Phe Lys Pro Asp
                   165                 170                 175
   Pro Arg Thr Asn Leu Asp Asp Asp Ser Arg Arg Phe Asp Phe Phe Ser
               180                 185                 190
   His Val Pro Glu Ala Thr Arg Thr Leu Thr Leu Leu Tyr Ser Asn Glu
           195                 200                 205
   Gly Thr Pro Ala Ser Tyr Arg Glu Met Asp Gly Asn Ser Val His Ala
       210                 215                 220
   Tyr Lys Leu Val Asn Ser Lys Gly Gln Val His Tyr Val Lys Phe His
   225                 230                 235                 240
   Trp Lys Ser Leu Gln Gly Gln Lys Asn Leu Asp Pro Lys Gln Val Glu
                   245                 250                 255
   Gln Val Gln Gly Arg Asp Tyr Ser His Met Thr Asn Asp Leu Val Ser
               260                 265                 270
   Ala Ile Lys Asp Gly Lys Phe Pro Lys Trp Asp Leu Tyr Ile Gln Val
           275                 280                 285
   Leu Lys Pro Glu Asp Leu Ala Lys Phe Asp Phe Asp Pro Leu Asp Ala
       290                 295                 300
   Thr Lys Ile Trp Pro Asn Val Pro Glu Arg Lys Ile Gly Gln Met Val
   305                 310                 315                 320
   Leu Asn Arg Asn Val Asp Asn Phe Phe Gln Glu Thr Glu Gln Val Ala
                   325                 330                 335
   Met Ala Pro Ser Asn Leu Val Pro Gly Ile Glu Pro Ser Glu Asp Arg
               340                 345                 350
   Leu Leu Gln Gly Arg Leu Phe Ala Tyr Ala Asp Thr Gln Met Tyr Arg
           355                 360                 365
   Ile Gly Ala Asn Gly Leu Gly Leu Pro Val Asn Gln Pro Arg Leu Pro
       370                 375                 380
   Val Asn Asn Val Asn Gln Asp Gly Gln Ala Asn Ala Gly His Thr Thr
   385                 390                 395                 400
   Thr Gly Val Asn Tyr Gln Pro Ser Arg Leu Gln Pro Arg Glu Glu Gln
                   405                 410                 415
   Ala Ser Ala Arg Tyr Val Pro Thr Pro Leu Val Gly Ser Thr Gln Gln
               420                 425                 430
   Ala Lys Ile Gln Arg Glu Gln Asn Phe Lys Gln Thr Gly Glu Leu Phe
           435                 440                 445
   Arg Ser Tyr Asn Lys Lys Glu Gln Thr Asp Leu Ile Asn Ser Leu Gly
       450                 455                 460
   Gln Ala Leu Ala Val Thr Asp Glu Glu Ser Arg Tyr Ile Met Leu Ser
   465                 470                 475                 480
   Phe Phe Tyr Lys Ala Asp Ser Asp Tyr Gly Ala Gly Leu Ala Ser Val
                   485                 490                 495
   Ala Lys Ala Asp Leu Lys Arg Val Gln Gln Leu Ala Ala Lys Leu Gln
               500                 505                 510
   Asp
<210> 19
   <211> 1643
   <212> DNA
   <213> Pseudomonas putida
<400> 19
   gaattcctcg agattttttc aatgtgattt taacttttac ttccagatga ctataatgtg      60
   actaaaaaca aaaccattgt tctggacata taacaccgta aggaggaatc catatgaaat     120
   acttgctgcc aacggcagcg gcagggttgc tcttgttggc ggcgcaaccg gctatggcgg     180
   cgaccctgac tcgcgattca ggtgcgccag tgggggataa ccagaacagt caaaccgccg     240
   gagccaatgg cgcggtgctg ttacaggatg cgaatctgct acagaaactt cagcgctttg     300
   atcgggaacg cattccagaa cgtgtggtac atgccagagg tacaggcgtc catggcgagt     360
   ttaccgcaag tgcggatatt agcgatctga gcattgccaa agtgtttcgg cccggtgaga     420
   aaacccctgt gtttgtccgc ttttcggcag ttgtgcacgg caaccactcg ccggaaaccc     480
   tacgtgaccc gcgcggtttt gccaccaagt tttacactgc tgacgggaat tgggatctcg     540
   ttgggaacaa ctttccgacg tttttcatcc gtgacgccat taagtttccg gatatggtcc     600
   atgcgttcaa acccgatccg cgtacgaacc tggacgatga ctctcgtcgc ttcgattttt     660
   tctcgcatgt tccggaagcg acgcgcacct taaccctctt gtattctaac gagggtacac     720
   ccgcctcata tcgcgaaatg gacggaaata gtgtacatgc ctacaaactt gttaattcga     780
   aaggtcaggt tcactacgtg aagttccact ggaagtccct tcagggtcag aaaaacctgg     840
   acccgaaaca ggtggaacag gttcaaggcc gtgattatag ccacatgaca aacgatctgg     900
   ttagcgcgat taaagacggc aaatttccga aatgggacct gtatatccaa gtgctgaaac      960
   cggaagacct ggccaaattc gattttgatc cgctggacgc aaccaaaatc tggccgaatg    1020
   tcccggaacg caagattggc cagatggtgc tcaatcgtaa cgtcgacaat ttcttccagg    1080
   aaactgaaca ggtcgcaatg gcccctagca atcttgtccc gggtattgaa ccaagcgagg    1140
   atcgtctgtt acaagggcga ctgttcgcgt atgctgatac ccaaatgtac cgtataggcg    1200
   ccaatggact gggtctgcct gtaaaccaac cgaggttacc cgtgaacaac gtgaatcaag    1260
   atggtcaggc gaatgctggc catactacca ctggcgtcaa ctaccaacca agtcggctgc    1320
   aacctcgcga agaacaggct tcagcgcgat atgttcctac cccgttagtg ggctctacac    1380
   aacaggccaa gatccagcgt gagcagaatt ttaaacagac gggcgagttg tttcgctcct    1440
   acaacaaaaa agaacagacg gacttaatca actctctggg acaggcgctg gcagttacgg    1500
   atgaagagtc ccgctatatc atgctgagct tcttctataa ggctgatagc gattatggtg    1560
   caggcctggc atccgtagca aaagccgatc tgaaacgtgt acagcagctc gctgcgaaac    1620
   tgcaagatta agagctcaag ctt                                            1643
<210> 20
   <211> 505
   <212> PRT
   <213> Pseudomonas putida
<400> 20
   Met Lys Tyr Leu Leu Pro Thr Ala Ala Ala Gly Leu Leu Leu Leu Ala
   1               5                   10                  15
   Ala Gln Pro Ala Met Ala Ala Thr Leu Thr Arg Asp Ser Gly Ala Pro
               20                  25                  30
   Val Gly Asp Asn Gln Asn Ser Gln Thr Ala Gly Ala Asn Gly Ala Val
           35                  40                  45
   Leu Leu Gln Asp Ala Asn Leu Leu Gln Lys Leu Gln Arg Phe Asp Arg
       50                  55                  60
   Glu Arg Ile Pro Glu Arg Val Val His Ala Arg Gly Thr Gly Val His
   65                  70                  75                  80
   Gly Glu Phe Thr Ala Ser Ala Asp Ile Ser Asp Leu Ser Ile Ala Lys
                   85                  90                  95
   Val Phe Arg Pro Gly Glu Lys Thr Pro Val Phe Val Arg Phe Ser Ala
               100                 105                 110
   Val Val His Gly Asn His Ser Pro Glu Thr Leu Arg Asp Pro Arg Gly
           115                 120                 125
   Phe Ala Thr Lys Phe Tyr Thr Ala Asp Gly Asn Trp Asp Leu Val Gly
       130                 135                 140
   Asn Asn Phe Pro Thr Phe Phe Ile Arg Asp Ala Ile Lys Phe Pro Asp
   145                 150                 155                 160
   Met Val His Ala Phe Lys Pro Asp Pro Arg Thr Asn Leu Asp Asp Asp
                   165                 170                 175
   Ser Arg Arg Phe Asp Phe Phe Ser His Val Pro Glu Ala Thr Arg Thr
               180                 185                 190
   Leu Thr Leu Leu Tyr Ser Asn Glu Gly Thr Pro Ala Ser Tyr Arg Glu
           195                 200                 205
   Met Asp Gly Asn Ser Val His Ala Tyr Lys Leu Val Asn Ser Lys Gly
       210                 215                 220
   Gln Val His Tyr Val Lys Phe His Trp Lys Ser Leu Gln Gly Gln Lys
   225                 230                 235                 240
   Asn Leu Asp Pro Lys Gln Val Glu Gln Val Gln Gly Arg Asp Tyr Ser
                   245                 250                 255
   His Met Thr Asn Asp Leu Val Ser Ala Ile Lys Asp Gly Lys Phe Pro
               260                 265                 270
   Lys Trp Asp Leu Tyr Ile Gln Val Leu Lys Pro Glu Asp Leu Ala Lys
           275                 280                 285
   Phe Asp Phe Asp Pro Leu Asp Ala Thr Lys Ile Trp Pro Asn Val Pro
       290                 295                 300
   Glu Arg Lys Ile Gly Gln Met Val Leu Asn Arg Asn Val Asp Asn Phe
   305                 310                 315                 320
   Phe Gln Glu Thr Glu Gln Val Ala Met Ala Pro Ser Asn Leu Val Pro
                   325                 330                 335
   Gly Ile Glu Pro Ser Glu Asp Arg Leu Leu Gln Gly Arg Leu Phe Ala
               340                 345                 350
   Tyr Ala Asp Thr Gln Met Tyr Arg Ile Gly Ala Asn Gly Leu Gly Leu
           355                 360                 365
   Pro Val Asn Gln Pro Arg Leu Pro Val Asn Asn Val Asn Gln Asp Gly
       370                 375                 380
   Gln Ala Asn Ala Gly His Thr Thr Thr Gly Val Asn Tyr Gln Pro Ser
   385                 390                 395                 400
   Arg Leu Gln Pro Arg Glu Glu Gln Ala Ser Ala Arg Tyr Val Pro Thr
                   405                 410                 415
   Pro Leu Val Gly Ser Thr Gln Gln Ala Lys Ile Gln Arg Glu Gln Asn
               420                 425                 430
   Phe Lys Gln Thr Gly Glu Leu Phe Arg Ser Tyr Asn Lys Lys Glu Gln
           435                 440                 445
   Thr Asp Leu Ile Asn Ser Leu Gly Gln Ala Leu Ala Val Thr Asp Glu
       450                 455                 460
   Glu Ser Arg Tyr Ile Met Leu Ser Phe Phe Tyr Lys Ala Asp Ser Asp
   465                 470                 475                 480
   Tyr Gly Ala Gly Leu Ala Ser Val Ala Lys Ala Asp Leu Lys Arg Val
                   485                 490                 495
   Gln Gln Leu Ala Ala Lys Leu Gln Asp
               500                 505
<210> 21
   <211> 2351
   <212> DNA
   <213> Shewanella oneidensis
<400> 21
   gaattcctcg agattttttc aatgtgattt taacttttac ttccagatga ctataatgtg     60
   actaaaaaca aaaccattgt tctggacata taacaccgta aggaggaatc catatgaaaa    120
   tcaacacgct cccgactttg tcagcgctca cgcttgcgat gtctctggcc ttaggcgctg    180
   gcatggcaac cgcccaagaa caagcaaccg gaaatcagtt ttggtggccg gagaaactca    240
   atctgtcccc attgcgtcag aacgcaattg agagcaatcc ttatggctcg gattaccggt    300
   atgcggaagc gtttaacacg ctggatcttg atgccgtcaa gaaggacata aaagccctga    360
   tgaccgagag tcaagactgg tggcccgccg actacggcca ttacggcccg tttttcatcc    420
   gcatggcttg gcatagcgcc ggggtttatc gcatttttga tggccgcggt ggggctgcgg    480
   gcggtcaaca gcgctttgaa ccgcttaatt cttggccagc ggataacgtg tcgctggata    540
   aagccaggcg tctgctgtgg ccaattaaac agaaatatgg tagcaaactg tcgtggggtg    600
   acctgatggt actgacggga aacgttgccc tggaatcaat gggctttaaa acgttcggct    660
   ttggcggtgg tcgtgtagat gattgggaag cggaaatggt caattggggc agtgaaaaag    720
   catggctgga caacaaacgc cacaacggta aaggtgagtt agcaaaaccg atgggcgcga    780
   cccagatggg tctgatttac gtgaatccgg aagggcctaa tggcgtgccg gaccccttag    840
   cgagcgcgaa agaaatccgc gatacatttg gtcgtatggc gatgaacgat gaggaaaccg    900
   tggcgttgat tgccggtggt cacacatttg gcaaagctca cggtgcgcat gatccggcga    960
   aatgtgtagg ggcagatccg gctgccgcag gtattgaaga acagggtctt gggtggaaaa   1020
   acaagtgcgg caaaggacat agcgaggaca cagtcacctc tggcttagaa ggcgcatggt   1080
   cctctaaccc tactaaatgg accatggaat atctgacctg gctgtatacc ttcgactggg   1140
   tgcagaccaa gtcgccagca ggtcacatcc aatggacccc cgctgatggc aaagcagcga   1200
   atctggttcc agatgcccat cttccggata aacgtcatgc gccgatcatg ttcacctccg   1260
   acattgcgct gaaagctgat ccgatttacc gggaaatcac tactcgtttc ctcaaaaacc   1320
   ctcaggaatt tgagttagcg tttgccaaag cctggttcaa gttgactcat cgcgacttag   1380
   gcccgaaagc ccgctattta ggcgcggacg ttcctgccga ggcccttatt tggcaagacc   1440
   ccattccagc attagaccac cccctgatcg ataatgcgga cattaaggcg ttgggcaaca   1500
   aaattctggc ttcgggtttg actgtaccgg aattggtgcg cacagcatgg gcttcagcat   1560
   cttcctttcg tgggaccgat atgagaggcg gtgcaaatgg ggctcgtatt cgactcgaac   1620
   cgatgatgaa ctggcaggcg aataatccga aggagctggc taaagtgttg gcgaagctag   1680
   agaaagtgca gaaagatttc aatggaagcc tgaaagggag taaaaaagtg agtctggcag   1740
   atgttatagt actcggtggt agcgtggctg tggaaaaagc cgccaaagag gcgggtgtgg   1800
   tcatctccgt tccgttcacc cctgggcgaa tggatgcaac ccaggtacag acggatgtta   1860
   acagctttgc ggttctggaa ccggcagcgg atggttttcg gaactactat agcaaggatt   1920
   cttccctgag tccggccgaa atgctcatcg aacgtgcgaa tatgctgaac ctgacggtcc   1980
   cggaaatgac ggttcttgtt ggagggttac gcgcactgga cgccaactca gccggtgtca   2040
   aacatggcgt cttcaccgat aaaccgggcg ttctatcgaa cgatttcttc gtgaacctgc   2100
   tggacatgag tacgaaatgg cgcaaaagcg ataagcagga aggcatctac gaaggacagg   2160
   atcgtaactc aggcaaactg aaatggacgg cgacaccagt cgatctgatc tttggcagcc   2220
   actcggagct gcgtgcggtc agcgaagtgt atggcgctca agatggacaa gaccgtttta   2280
   tccaggattt cgtgaaggcg tggaataagg tgatgaatgc cgaccgcttt gacatttaag   2340
   agctcaagct t                                                        2351
<210> 22
   <211> 741
   <212> PRT
   <213> Shewanella oneidensis
<400> 22
   Met Lys Ile Asn Thr Leu Pro Thr Leu Ser Ala Leu Thr Leu Ala Met
   1               5                   10                  15
   Ser Leu Ala Leu Gly Ala Gly Met Ala Thr Ala Gln Glu Gln Ala Thr
               20                  25                  30
   Gly Asn Gln Phe Trp Trp Pro Glu Lys Leu Asn Leu Ser Pro Leu Arg
           35                  40                  45
   Gln Asn Ala Ile Glu Ser Asn Pro Tyr Gly Ser Asp Tyr Arg Tyr Ala
       50                  55                  60
   Glu Ala Phe Asn Thr Leu Asp Leu Asp Ala Val Lys Lys Asp Ile Lys
   65                  70                  75                  80
   Ala Leu Met Thr Glu Ser Gln Asp Trp Trp Pro Ala Asp Tyr Gly His
                   85                  90                  95
   Tyr Gly Pro Phe Phe Ile Arg Met Ala Trp His Ser Ala Gly Val Tyr
               100                 105                 110
   Arg Ile Phe Asp Gly Arg Gly Gly Ala Ala Gly Gly Gln Gln Arg Phe
           115                 120                 125
   Glu Pro Leu Asn Ser Trp Pro Ala Asp Asn Val Ser Leu Asp Lys Ala
       130                 135                 140
   Arg Arg Leu Leu Trp Pro Ile Lys Gln Lys Tyr Gly Ser Lys Leu Ser
   145                 150                 155                 160
   Trp Gly Asp Leu Met Val Leu Thr Gly Asn Val Ala Leu Glu Ser Met
                   165                 170                 175
   Gly Phe Lys Thr Phe Gly Phe Gly Gly Gly Arg Val Asp Asp Trp Glu
               180                 185                 190
   Ala Glu Met Val Asn Trp Gly Ser Glu Lys Ala Trp Leu Asp Asn Lys
           195                 200                 205
   Arg His Asn Gly Lys Gly Glu Leu Ala Lys Pro Met Gly Ala Thr Gln
       210                 215                 220
   Met Gly Leu Ile Tyr Val Asn Pro Glu Gly Pro Asn Gly Val Pro Asp
   225                 230                 235                 240
   Pro Leu Ala Ser Ala Lys Glu Ile Arg Asp Thr Phe Gly Arg Met Ala
                   245                 250                 255
   Met Asn Asp Glu Glu Thr Val Ala Leu Ile Ala Gly Gly His Thr Phe
               260                 265                 270
   Gly Lys Ala His Gly Ala His Asp Pro Ala Lys Cys Val Gly Ala Asp
           275                 280                 285
   Pro Ala Ala Ala Gly Ile Glu Glu Gln Gly Leu Gly Trp Lys Asn Lys
       290                 295                 300
   Cys Gly Lys Gly His Ser Glu Asp Thr Val Thr Ser Gly Leu Glu Gly
   305                 310                 315                 320
   Ala Trp Ser Ser Asn Pro Thr Lys Trp Thr Met Glu Tyr Leu Thr Trp
                   325                 330                 335
   Leu Tyr Thr Phe Asp Trp Val Gln Thr Lys Ser Pro Ala Gly His Ile
               340                 345                 350
   Gln Trp Thr Pro Ala Asp Gly Lys Ala Ala Asn Leu Val Pro Asp Ala
           355                 360                 365
   His Leu Pro Asp Lys Arg His Ala Pro Ile Met Phe Thr Ser Asp Ile
       370                 375                 380
   Ala Leu Lys Ala Asp Pro Ile Tyr Arg Glu Ile Thr Thr Arg Phe Leu
   385                 390                 395                 400
   Lys Asn Pro Gln Glu Phe Glu Leu Ala Phe Ala Lys Ala Trp Phe Lys
                   405                 410                 415
   Leu Thr His Arg Asp Leu Gly Pro Lys Ala Arg Tyr Leu Gly Ala Asp
               420                 425                 430
   Val Pro Ala Glu Ala Leu Ile Trp Gln Asp Pro Ile Pro Ala Leu Asp
           435                 440                 445
   His Pro Leu Ile Asp Asn Ala Asp Ile Lys Ala Leu Gly Asn Lys Ile
       450                 455                 460
   Leu Ala Ser Gly Leu Thr Val Pro Glu Leu Val Arg Thr Ala Trp Ala
   465                 470                 475                 480
   Ser Ala Ser Ser Phe Arg Gly Thr Asp Met Arg Gly Gly Ala Asn Gly
                   485                 490                 495
   Ala Arg Ile Arg Leu Glu Pro Met Met Asn Trp Gln Ala Asn Asn Pro
               500                 505                 510
   Lys Glu Leu Ala Lys Val Leu Ala Lys Leu Glu Lys Val Gln Lys Asp
           515                 520                 525
   Phe Asn Gly Ser Leu Lys Gly Ser Lys Lys Val Ser Leu Ala Asp Val
       530                 535                 540
   Ile Val Leu Gly Gly Ser Val Ala Val Glu Lys Ala Ala Lys Glu Ala
   545                 550                 555                 560
   Gly Val Val Ile Ser Val Pro Phe Thr Pro Gly Arg Met Asp Ala Thr
                   565                 570                 575
   Gln Val Gln Thr Asp Val Asn Ser Phe Ala Val Leu Glu Pro Ala Ala
               580                 585                 590
   Asp Gly Phe Arg Asn Tyr Tyr Ser Lys Asp Ser Ser Leu Ser Pro Ala
           595                 600                 605
   Glu Met Leu Ile Glu Arg Ala Asn Met Leu Asn Leu Thr Val Pro Glu
       610                 615                 620
   Met Thr Val Leu Val Gly Gly Leu Arg Ala Leu Asp Ala Asn Ser Ala
   625                 630                 635                 640
   Gly Val Lys His Gly Val Phe Thr Asp Lys Pro Gly Val Leu Ser Asn
                   645                 650                 655
   Asp Phe Phe Val Asn Leu Leu Asp Met Ser Thr Lys Trp Arg Lys Ser
               660                 665                 670
   Asp Lys Gln Glu Gly Ile Tyr Glu Gly Gln Asp Arg Asn Ser Gly Lys
           675                 680                 685
   Leu Lys Trp Thr Ala Thr Pro Val Asp Leu Ile Phe Gly Ser His Ser
       690                 695                 700
   Glu Leu Arg Ala Val Ser Glu Val Tyr Gly Ala Gln Asp Gly Gln Asp
   705                 710                 715                 720
   Arg Phe Ile Gln Asp Phe Val Lys Ala Trp Asn Lys Val Met Asn Ala
                   725                 730                 735
   Asp Arg Phe Asp Ile
               740
<210> 23
   <211> 1551
   <212> DNA
   <213> Pseudomonas entomophila
<400> 23
   catatgattc ctagcctcaa actgttccaa ccaggtcgtc tgttagtggc ggctagcttg      60
   gcggcatcat tgctgtctat gtctgtacaa gcagcgacgc tcacacgtga taatggcgcg     120
   ccggtgggtg acaaccagaa ctcgcagacc gcgggcccta acggtgcagt gctgctgcaa     180
   gacgttcaat tacttcagaa gctgcaacgc tttgatcgtg aacgcatccc ggaacgcgtc     240
   gttcatgctc gtggtactgg cgcacatggt caattcacgg cgagcgccga tatctccgat     300
   ctgtccatgg cgaaagtgtt tcgccctggt gaaaagactc cggtctttgt gcgcttcagc     360
   tcggtggtac acggcaatca ttcgccggaa acgctgcgtg atccgcgtgg atttgccacg     420
   aagttttata ccgcggatgg gaattgggat ctggttggta acaactttcc caccttcttc     480
   attcgcgatg ctatcaaatt tccggacatg gttcatgcct ttaaaccgga cccgcgctcg     540
   aacttagacg atgattctcg tcgctttgac ttcttcagtc atgttcctga agccacccgc     600
   accctgaccc tcctgtactc caatgagggg acgccagcat catacgggga gatggacggc     660
   aatagcgtcc atgcctataa actggtgaac gcgaaaggcg aaacccacta tgtaaaattc     720
   cactggaaat cccttcaggg tcagaagaat ttagacccga aacaggtcga acaggttcag     780
   gggcgtgact attcgcacat gacgcacgat cttgtgagtg cgattggaaa gggcaacttt     840
   ccgaaatggg acctgtacat ccaggtcctg aaaccggaag agttggccaa attcgatttt     900
   gatccgttag atgcgacgaa aatttggcct ggtattcccg agcggaaaat tggtcaaatg     960
   gttctggatc ggaatgtaga taacttcttt caggaaactg aacaagtcgc aatggcgcca    1020
   agtaatctcg ttccgggcat tgagcccagt gaggaccgtc tgcttcaggg ccgcctgttt    1080
   gcttacgccg atacccagat gtatcgtatc ggggctaacg gcctgagtct gccagtcaat    1140
   cgcccacgtg ctgaagtgaa cagcattaac caagatggcg caatgaacgc cggtcatacc    1200
   gattcgggcg tgaattatca gccctctcgt cgccagccgc gtgacgaaca ggcatccgcc    1260
   cgctacgtga gtaccccgtt gtctggtagc acacagcagg ccaaaattca gcgcgaacag    1320
   aactttaaac aaactggaga gctgttccgc agctatggga agaaagatca gaccgactta    1380
   atcaattcac tgggagcagc gctggcaaca gctgatgaag aatcacgcta catcatgttg    1440
   agcttctttt ataaagcgga tagcgattat ggcacaggcc tggcgaaggt agccaaagcg    1500
   gacttgaaac gcgtgcagca gctcgccgcc aaactgcaag attaagagct c             1551
<210> 24
   <211> 513
   <212> PRT
   <213> Pseudomonas entomophila
<400> 24
   Met Ile Pro Ser Leu Lys Leu Phe Gln Pro Gly Arg Leu Leu Val Ala
   1               5                   10                  15
   Ala Ser Leu Ala Ala Ser Leu Leu Ser Met Ser Val Gln Ala Ala Thr
               20                  25                  30
   Leu Thr Arg Asp Asn Gly Ala Pro Val Gly Asp Asn Gln Asn Ser Gln
           35                  40                  45
   Thr Ala Gly Pro Asn Gly Ala Val Leu Leu Gln Asp Val Gln Leu Leu
       50                  55                  60
   Gln Lys Leu Gln Arg Phe Asp Arg Glu Arg Ile Pro Glu Arg Val Val
   65                  70                  75                  80
   His Ala Arg Gly Thr Gly Ala His Gly Gln Phe Thr Ala Ser Ala Asp
                   85                  90                  95
   Ile Ser Asp Leu Ser Met Ala Lys Val Phe Arg Pro Gly Glu Lys Thr
               100                 105                 110
   Pro Val Phe Val Arg Phe Ser Ser Val Val His Gly Asn His Ser Pro
           115                 120                 125
   Glu Thr Leu Arg Asp Pro Arg Gly Phe Ala Thr Lys Phe Tyr Thr Ala
   130 135 140
   Asp Gly Asn Trp Asp Leu Val Gly Asn Asn Phe Pro Thr Phe Phe Ile
   145 150 155 160
   Arg Asp Ala Ile Lys Phe Pro Asp Met Val His Ala Phe Lys Pro Asp
   165 170 175
   Pro Arg Ser Asn Leu Asp Asp Asp Ser Arg Arg Phe Asp Phe Phe Ser
   180 185 190
   His Val Pro Glu Ala Thr Arg Thr Leu Thr Leu Leu Tyr Ser Asn Glu
   195 200 205
   Gly Thr Pro Ala Ser Tyr Arg Glu Met Asp Gly Asn Ser Val His Ala
   210 215 220
   Tyr Lys Leu Val Asn Ala Lys Gly Glu Thr His Tyr Val Lys Phe His
   225 230 235 240
   Trp Lys Ser Leu Gln Gly Gln Lys Asn Leu Asp Pro Lys Gln Val Glu
   245 250 255
   Gln Val Gln Gly Arg Asp Tyr Ser His Met Thr His Asp Leu Val Ser
   260 265 270
   Ala Ile Gly Lys Gly Asn Phe Pro Lys Trp Asp Leu Tyr Ile Gln Val
   275 280 285
   Leu Lys Pro Glu Glu Leu Ala Lys Phe Asp Phe Asp Pro Leu Asp Ala
   290 295 300
   Thr Lys Ile Trp Pro Gly Ile Pro Glu Arg Lys Ile Gly Gln Met Val
   305 310 315 320
   Leu Asp Arg Asn Val Asp Asn Phe Phe Gln Glu Thr Glu Gln Val Ala
   325 330 335
   Met Ala Pro Ser Asn Leu Val Pro Gly Ile Glu Pro Ser Glu Asp Arg
   340 345 350
   Leu Leu Gln Gly Arg Leu Phe Ala Tyr Ala Asp Thr Gln Met Tyr Arg
   355 360 365
   Ile Gly Ala Asn Gly Leu Ser Leu Pro Val Asn Arg Pro Arg Ala Glu
   370 375 380
   Val Asn Ser Ile Asn Gln Asp Gly Ala Met Asn Ala Gly His Thr Asp
   385 390 395 400
   Ser Gly Val Asn Tyr Gln Pro Ser Arg Arg Gln Pro Arg Asp Glu Gln
   405 410 415
   Ala Ser Ala Arg Tyr Val Ser Thr Pro Leu Ser Gly Ser Thr Gln Gln
   420 425 430
   Ala Lys Ile Gln Arg Glu Gln Asn Phe Lys Gln Thr Gly Glu Leu Phe
   435 440 445
   Arg Ser Tyr Gly Lys Lys Asp Gln Thr Asp Leu Ile Asn Ser Leu Gly
   450 455 460
   Ala Ala Leu Ala Thr Ala Asp Glu Glu Ser Arg Tyr Ile Met Leu Ser
   465 470 475 480
   Phe Phe Tyr Lys Ala Asp Ser Asp Tyr Gly Thr Gly Leu Ala Lys Val
   485 490 495
   Ala Lys Ala Asp Leu Lys Arg Val Gln Gln Leu Ala Ala Lys Leu Gln
   500 505 510
   Asp
<210> 25
   <211> 1551
   <212> DNA
   <213> Pseudomonas parafulva
<400> 25
   catatgaaac ctgtgacgtc cccttatccg gcgagtcggc ttctgctgac cgcgagcttc      60
   tgtgcgagct tattttgcag cgccgcgcaa gccaccccgc tgactcgcga taacggcgct     120
   ccagttggcg acaaccagaa ctctcagacc gccggtgcaa atggacccgt tttgctgcaa     180
   gacgttcagc tgttgcagaa attacagcgc tttgaccgcg aacgtatccc ggaacgtgtc     240
   gtacatgcgc gtggcacagg tgcacacggc gaatttaccg cttcggcaga catttcggat     300
   ctgacatccg cgaaagtatt ccgtcaaggc gaacataccc cggtgtttgt tcgctttagc     360
   agtgtcgtcc atggcaatca tagtccggaa actttgcgcg atccacgcgg tttcgcaacg     420
   aaattctata cggccgatgg taactgggac ctggtaggga acaatttccc cacgttcttc     480
   attcgcgatg ccatcaaatt tccggatatg gtgcacgcat ttaaaccaga tccccgtacc     540
   aacctggatg atgatagccg ccgctttgat ttctttagcc acgtgccgga ggcgacacgt     600
   acgctgaccc tgttgtattc caatgaaggc acaccggcct cctatcgcga aatggacggc     660
   aatagcgttc acgcctataa actggtaaac gcgaaaggtg acgttcatta cgtgaagttc     720
   cactggaagt cattacaggg acagaagaac ctggacccgc gccaagtggc tgaaatccaa     780
   gctcgtgact attcgcacat gacccatgat ctggttgccg ccattggaaa aggcaattac     840
   ccgaaatggg atctgtatat ccaggtcctc aaaccggaag atttagcgaa attcgacttc     900
   gatccgctgg atgcgaccaa aatctggccg gatgtccctg aacgcaaagt ggggcagatg     960
   gtgctgaatc gcaacgtgga aaacttcttt caggaaacgg agcaagtagc tatggcaccc    1020
   tccaacttag ttccgggtat tgaaccttcg gaggatcgct tgcttcaagg ccgtctgttt    1080
   gcgtatgccg atactcagat gtatcgtatt ggtgcaaacg gcctcagctt accagtgaac    1140
   cgcccacgtt cggatgtcaa cacggtcaat caggacggtg caatgaatgc cggtcatacc    1200
   cagtctgggg tgaactatca accgtcacgc cttcagccgc gtgatgagca agcgaatgcc    1260
   cgggctgtgc agatgcctct taatgggagc acccagcagg cgaaaatcca acgtgagcag    1320
   aattttaaac agactgggga actctttcgc agttactcta agaaggatcg ggaggacctg    1380
   attcagtcac tgggcgaagc gctggcgatt accgacgagc agtctcgcta cattatgctg    1440
   agttactttt acaaggcaga ctcagattac ggcactggtc tggctaaagt cgccaaagca    1500
   gacgtgcagc gggttcgtga tctggcggcg caactcaaag attaagagct c             1551
<210> 26
   <211> 513
   <212> PRT
   <213> Pseudomonas parafulva
<400> 26
   Met Lys Pro Val Thr Ser Pro Tyr Pro Ala Ser Arg Leu Leu Leu Thr
   1               5                   10                  15
   Ala Ser Phe Cys Ala Ser Leu Phe Cys Ser Ala Ala Gln Ala Thr Pro
               20                  25                  30
   Leu Thr Arg Asp Asn Gly Ala Pro Val Gly Asp Asn Gln Asn Ser Gln
           35                  40                  45
   Thr Ala Gly Ala Asn Gly Pro Val Leu Leu Gln Asp Val Gln Leu Leu
       50                  55                  60
   Gln Lys Leu Gln Arg Phe Asp Arg Glu Arg Ile Pro Glu Arg Val Val
   65                  70                  75                  80
   His Ala Arg Gly Thr Gly Ala His Gly Glu Phe Thr Ala Ser Ala Asp
                   85                  90                  95
   Ile Ser Asp Leu Thr Ser Ala Lys Val Phe Arg Gln Gly Glu His Thr
               100                 105                 110
   Pro Val Phe Val Arg Phe Ser Ser Val Val His Gly Asn His Ser Pro
           115                 120                 125
   Glu Thr Leu Arg Asp Pro Arg Gly Phe Ala Thr Lys Phe Tyr Thr Ala
       130                 135                 140
   Asp Gly Asn Trp Asp Leu Val Gly Asn Asn Phe Pro Thr Phe Phe Ile
   145                 150                 155                 160
   Arg Asp Ala Ile Lys Phe Pro Asp Met Val His Ala Phe Lys Pro Asp
                   165                 170                 175
   Pro Arg Thr Asn Leu Asp Asp Asp Ser Arg Arg Phe Asp Phe Phe Ser
               180                 185                 190
   His Val Pro Glu Ala Thr Arg Thr Leu Thr Leu Leu Tyr Ser Asn Glu
           195                 200                 205
   Gly Thr Pro Ala Ser Tyr Arg Glu Met Asp Gly Asn Ser Val His Ala
       210                 215                 220
   Tyr Lys Leu Val Asn Ala Lys Gly Asp Val His Tyr Val Lys Phe His
   225                 230                 235                 240
   Trp Lys Ser Leu Gln Gly Gln Lys Asn Leu Asp Pro Arg Gln Val Ala
                   245                 250                 255
   Glu Ile Gln Ala Arg Asp Tyr Ser His Met Thr His Asp Leu Val Ala
               260                 265                 270
   Ala Ile Gly Lys Gly Asn Tyr Pro Lys Trp Asp Leu Tyr Ile Gln Val
           275                 280                 285
   Leu Lys Pro Glu Asp Leu Ala Lys Phe Asp Phe Asp Pro Leu Asp Ala
       290                 295                 300
   Thr Lys Ile Trp Pro Asp Val Pro Glu Arg Lys Val Gly Gln Met Val
   305                 310                 315                 320
   Leu Asn Arg Asn Val Glu Asn Phe Phe Gln Glu Thr Glu Gln Val Ala
                   325                 330                 335
   Met Ala Pro Ser Asn Leu Val Pro Gly Ile Glu Pro Ser Glu Asp Arg
               340                 345                 350
   Leu Leu Gln Gly Arg Leu Phe Ala Tyr Ala Asp Thr Gln Met Tyr Arg
           355                 360                 365
   Ile Gly Ala Asn Gly Leu Ser Leu Pro Val Asn Arg Pro Arg Ser Asp
       370                 375                 380
   Val Asn Thr Val Asn Gln Asp Gly Ala Met Asn Ala Gly His Thr Gln
   385                 390                 395                 400
   Ser Gly Val Asn Tyr Gln Pro Ser Arg Leu Gln Pro Arg Asp Glu Gln
                   405                 410                 415
   Ala Asn Ala Arg Ala Val Gln Met Pro Leu Asn Gly Ser Thr Gln Gln
               420                 425                 430
   Ala Lys Ile Gln Arg Glu Gln Asn Phe Lys Gln Thr Gly Glu Leu Phe
           435                 440                 445
   Arg Ser Tyr Ser Lys Lys Asp Arg Glu Asp Leu Ile Gln Ser Leu Gly
       450                 455                 460
   Glu Ala Leu Ala Ile Thr Asp Glu Gln Ser Arg Tyr Ile Met Leu Ser
   465                 470                 475                 480
   Tyr Phe Tyr Lys Ala Asp Ser Asp Tyr Gly Thr Gly Leu Ala Lys Val
                   485                 490                 495
   Ala Lys Ala Asp Val Gln Arg Val Arg Asp Leu Ala Ala Gln Leu Lys
               500                 505                 510
   Asp
<210> 27
   <211> 1551
   <212> DNA
   <213> Pseudomonas protegens
<400> 27
   catatgaccg catcactcgg actcggctcg ttatcgcaac gccgcgtcct gggtgtctta      60
   gccgcgtcca tgttgtcatt gagtgttcag gcagcgacgc tgactcgcga taatggcgcc     120
   gcggttggtg ataaccagaa ctcgcagacc gcgggtgcga ccggacctgt gctgttgcaa     180
   gacgttcaac tgattcagaa actccaacgc ttcgatcgcg aacgtatccc agagcgtgtg     240
   gtccatgctc gtggcacagg tgctcatggg acctttactg tgactgataa tctgacagat     300
   ttaacgcgtg caaaagtatt tgccgcgggt gaagtgaccc cggtatttgt gcgcttttca     360
   gcggtcgttc atgggaacca cagccctgaa acccttcgtg acccacgtgg ttttgcaacg     420
   aaattttata cggccgatgg caattgggat ctggttggca acaattttcc gaccttcttc     480
   atccgtgacg cgatcaaatt ccccgacatg gtgcacgctt tcaaaccgga cccgcgcact     540
   aatctcgatg acgatagccg ccgttttgat ttctttagcc atgtgcccga agcaactcgc     600
   accctgaccg aactttactc caactcaggc acgcctgcat cgtatcgcga gatggacggc     660
   aacggcgtac atgcgtacaa actgattaac gccaaaggtg aagtccacta tgtcaaattc     720
   cactggaaaa gccttcaagg tctgaagaac ctggacccga aacaggtggt ggaagtgcag     780
   gggcgtgatt actctcacat gacaaacgat ctggtgacgc atattaataa gggcgacttc     840
   ccgaagtggg atctgtacgt tcaggttctg aaaccggaag atttggcgaa attcgatttt     900
   gacccgcttg acgccaccaa aatttggccg ggcgtcccgg agcgcaaagt tggtcaaatg     960
   gtgttaaacc gcaatccggc gaacttcttt caggaaaccg aacaagtggc gatggctcca    1020
   gcgaatctgg tgccgggcat cgaaccctcc gaagatcgcc tgctgcaagg tcgtgtattt    1080
   tcttacgccg atacccagat gtatcggatt ggcgcaaatg cactgcaatt accgattaac    1140
   gcaccgaaga atccggtaaa caacggtaat caggatggtg ccatgaattt ggggcattcc    1200
   agtacgggcg tcaattacca accatctcgc ctgatgcccc gcgaagagcc acagaccgct    1260
   cgctatagcc agatggcgct ggctgggtct acccagcagg ccaaaattca gcgtgaacag    1320
   aactttaaac aggctgggga tctctatcgg agttttagca agaaagagcg gcaggatttg    1380
   attgacagct tcggaggcag tctggccact acagatgacg agagtaaaca catcatgctg    1440
   agctttctgt ataaagccga tcctgagtat ggtacgggag taacgaaagt tgcgaagggc    1500
   gatctggcgc gtgttaaggc cctggcagcc aaattatcgg actaagagct c             1551
<210> 28
   <211> 513
   <212> PRT
   <213> Pseudomonas protegens
<400> 28
   Met Thr Ala Ser Leu Gly Leu Gly Ser Leu Ser Gln Arg Arg Val Leu
   1               5                   10                  15
   Gly Val Leu Ala Ala Ser Met Leu Ser Leu Ser Val Gln Ala Ala Thr
               20                  25                  30
   Leu Thr Arg Asp Asn Gly Ala Ala Val Gly Asp Asn Gln Asn Ser Gln
           35                  40                  45
   Thr Ala Gly Ala Thr Gly Pro Val Leu Leu Gln Asp Val Gln Leu Ile
       50                  55                  60
   Gln Lys Leu Gln Arg Phe Asp Arg Glu Arg Ile Pro Glu Arg Val Val
   65                  70                  75                  80
   His Ala Arg Gly Thr Gly Ala His Gly Thr Phe Thr Val Thr Asp Asn
                   85                  90                  95
   Leu Thr Asp Leu Thr Arg Ala Lys Val Phe Ala Ala Gly Glu Val Thr
               100                 105                 110
   Pro Val Phe Val Arg Phe Ser Ala Val Val His Gly Asn His Ser Pro
           115                 120                 125
   Glu Thr Leu Arg Asp Pro Arg Gly Phe Ala Thr Lys Phe Tyr Thr Ala
       130                 135                 140
   Asp Gly Asn Trp Asp Leu Val Gly Asn Asn Phe Pro Thr Phe Phe Ile
   145                 150                 155                 160
   Arg Asp Ala Ile Lys Phe Pro Asp Met Val His Ala Phe Lys Pro Asp
                   165                 170                 175
   Pro Arg Thr Asn Leu Asp Asp Asp Ser Arg Arg Phe Asp Phe Phe Ser
               180                 185                 190
   His Val Pro Glu Ala Thr Arg Thr Leu Thr Glu Leu Tyr Ser Asn Ser
           195                 200                 205
   Gly Thr Pro Ala Ser Tyr Arg Glu Met Asp Gly Asn Gly Val His Ala
       210                 215                 220
   Tyr Lys Leu Ile Asn Ala Lys Gly Glu Val His Tyr Val Lys Phe His
   225                 230                 235                 240
   Trp Lys Ser Leu Gln Gly Leu Lys Asn Leu Asp Pro Lys Gln Val Val
                   245                 250                 255
   Glu Val Gln Gly Arg Asp Tyr Ser His Met Thr Asn Asp Leu Val Thr
               260                 265                 270
   His Ile Asn Lys Gly Asp Phe Pro Lys Trp Asp Leu Tyr Val Gln Val
           275                 280                 285
   Leu Lys Pro Glu Asp Leu Ala Lys Phe Asp Phe Asp Pro Leu Asp Ala
       290                 295                 300
   Thr Lys Ile Trp Pro Gly Val Pro Glu Arg Lys Val Gly Gln Met Val
   305                 310                 315                 320
   Leu Asn Arg Asn Pro Ala Asn Phe Phe Gln Glu Thr Glu Gln Val Ala
                   325                 330                 335
   Met Ala Pro Ala Asn Leu Val Pro Gly Ile Glu Pro Ser Glu Asp Arg
               340                 345                 350
   Leu Leu Gln Gly Arg Val Phe Ser Tyr Ala Asp Thr Gln Met Tyr Arg
           355                 360                 365
   Ile Gly Ala Asn Ala Leu Gln Leu Pro Ile Asn Ala Pro Lys Asn Pro
       370                 375                 380
   Val Asn Asn Gly Asn Gln Asp Gly Ala Met Asn Leu Gly His Ser Ser
   385                 390                 395                 400
   Thr Gly Val Asn Tyr Gln Pro Ser Arg Leu Met Pro Arg Glu Glu Pro
                   405                 410                 415
   Gln Thr Ala Arg Tyr Ser Gln Met Ala Leu Ala Gly Ser Thr Gln Gln
                   420                 425                 430
   Ala Lys Ile Gln Arg Glu Gln Asn Phe Lys Gln Ala Gly Asp Leu Tyr
               435                 440                 445
   Arg Ser Phe Ser Lys Lys Glu Arg Gln Asp Leu Ile Asp Ser Phe Gly
       450                 455                 460
   Gly Ser Leu Ala Thr Thr Asp Asp Glu Ser Lys His Ile Met Leu Ser
   465                 470                 475                 480
   Phe Leu Tyr Lys Ala Asp Pro Glu Tyr Gly Thr Gly Val Thr Lys Val
                   485                 490                 495
   Ala Lys Gly Asp Leu Ala Arg Val Lys Ala Leu Ala Ala Lys Leu Ser
               500                 505                 510
   Asp
<210> 29
   <211> 1557
   <212> DNA
   <213> Erwinia mallotivora
<400> 29
   catatgtcga atgaccacat aactggtagt ggcgtatccc gcaaaaaact gctgattgtc     60
   ctgatgagtt tggcgatctc tccgattgtt caggcagata ccctcacccg tgacaatggt    120
   gcccccgttg gcgataatca gaacagccaa accgccggtg ccaacggtcc ggtgctgtta    180
   caggatgtgc agttattgca gaaactccag cgctttgacc gagaacgcat tcctgaacgt    240
   gttgttcatg cgcgaggaac cggtgcgcat ggcgaattta cggcgaccgc ggatatctct    300
   gatctgacca ttgcgaaagt gttcactacc ggctctaaaa ctccggtgtt tgttcgcttt    360
   tcaagcgtgg ttcatgggaa ccattcaccg gaaacactgc gtgatcctcg cggctttgcg    420
   acaaagttct acacgacaga cggcaattgg gatctcgtgg gtaacaattt tccgacgttc    480
   ttcattcgtg atgcgatcaa attcccggac atggtacatg cgttcaaacc tgatccccgt    540
   acgaatctcg ataacgatag cagacgtttt gactttttct cacatgttcc tgaatccacg    600
   cgcactctta ccctgctgta ctcgaatgaa ggcacaccag ccagctatcg taacatggat    660
   ggcaatgggg tgcacgcgta taagctggtc aacagcaaag gcgaagtaca ctacgtgaaa    720
   ttccactgga aaacccttca aggagtcaaa aatcttgacc cccagcaggt ggaacaggtg    780
   caaggcaaag actattcgca catgacccat gacttggttg ctgctatcaa tcgtggtgat    840
   tacccgaaat gggatctgta cattcaggta ctgaaaccgg aggacctgaa gaagttcgac    900
   tttgatccct tggatgccac caaaatttgg ccaggggtcc cggaacgtaa gattgggcag    960
   atggtcctga ataagaaccc agacaacgtc tttcaggaga ccgaacaggt tgcaatggca   1020
   ccgagtaatt tagtaccagg tatcgaaccg tccgaagata aactgctaca gggccggctg   1080
   tttgcctatg ccgatacgca actgtatcgc attggtgcta atggtttgtc gctgccgatt   1140
   aacaaaccgc gcaatgaggt gaacaacggt aaccaagatg gctcgctgaa cagtggccat   1200
   acccaagaca aaggggtgaa ctatcagccg agccgcctct atccgaggga agaactggct   1260
   tctgcacggt attctcacac gccattagaa ggaaccaccc ttcaaagcaa aatccaacgc   1320
   gagcagaact ttaaacagac cggagagttg tatcgctcct acagcaaaaa agagcaagat   1380
   gatctgatca actcactagg gacgagtctt gcggatacgg acacagagag caagaacatc   1440
   atgttatcct acttttataa agccgataaa gattacggca ctcgtctgac gggtgtggca   1500
   aaaggcgatt tagcaactgt ccagaaactg gctgacaagc tgagcgatta agagctc      1557
<210> 30
   <211> 515
   <212> PRT
   <213> Erwinia mallotivora
<400> 30
   Met Ser Asn Asp His Ile Thr Gly Ser Gly Val Ser Arg Lys Lys Leu
   1               5                   10                  15
   Leu Ile Val Leu Met Ser Leu Ala Ile Ser Pro Ile Val Gln Ala Asp
               20                  25                  30
   Thr Leu Thr Arg Asp Asn Gly Ala Pro Val Gly Asp Asn Gln Asn Ser
           35                  40                  45
   Gln Thr Ala Gly Ala Asn Gly Pro Val Leu Leu Gln Asp Val Gln Leu
       50                  55                  60
   Leu Gln Lys Leu Gln Arg Phe Asp Arg Glu Arg Ile Pro Glu Arg Val
   65 70 75 80
   Val His Ala Arg Gly Thr Gly Ala His Gly Glu Phe Thr Ala Thr Ala
   85 90 95
   Asp Ile Ser Asp Leu Thr Ile Ala Lys Val Phe Thr Thr Gly Ser Lys
   100 105 110
   Thr Pro Val Phe Val Arg Phe Ser Ser Val Val His Gly Asn His Ser
   115 120 125
   Pro Glu Thr Leu Arg Asp Pro Arg Gly Phe Ala Thr Lys Phe Tyr Thr
   130 135 140
   Thr Asp Gly Asn Trp Asp Leu Val Gly Asn Asn Phe Pro Thr Phe Phe
   145 150 155 160
   Ile Arg Asp Ala Ile Lys Phe Pro Asp Met Val His Ala Phe Lys Pro
   165 170 175
   Asp Pro Arg Thr Asn Leu Asp Asn Asp Ser Arg Arg Phe Asp Phe Phe
   180 185 190
   Ser His Val Pro Glu Ser Thr Arg Thr Leu Thr Leu Leu Tyr Ser Asn
   195 200 205
   Glu Gly Thr Pro Ala Ser Tyr Arg Asn Met Asp Gly Asn Gly Val His
   210 215 220
   Ala Tyr Lys Leu Val Asn Ser Lys Gly Glu Val His Tyr Val Lys Phe
   225 230 235 240
   His Trp Lys Thr Leu Gln Gly Val Lys Asn Leu Asp Pro Gln Gln Val
   245 250 255
   Glu Gln Val Gln Gly Lys Asp Tyr Ser His Met Thr His Asp Leu Val
   260 265 270
   Ala Ala Ile Asn Arg Gly Asp Tyr Pro Lys Trp Asp Leu Tyr Ile Gln
   275 280 285
   Val Leu Lys Pro Glu Asp Leu Lys Lys Phe Asp Phe Asp Pro Leu Asp
   290 295 300
   Ala Thr Lys Ile Trp Pro Gly Val Pro Glu Arg Lys Ile Gly Gln Met
   305 310 315 320
   Val Leu Asn Lys Asn Pro Asp Asn Val Phe Gln Glu Thr Glu Gln Val
   325 330 335
   Ala Met Ala Pro Ser Asn Leu Val Pro Gly Ile Glu Pro Ser Glu Asp
   340 345 350
   Lys Leu Leu Gln Gly Arg Leu Phe Ala Tyr Ala Asp Thr Gln Leu Tyr
   355 360 365
   Arg Ile Gly Ala Asn Gly Leu Ser Leu Pro Ile Asn Lys Pro Arg Asn
   370 375 380
   Glu Val Asn Asn Gly Asn Gln Asp Gly Ser Leu Asn Ser Gly His Thr
   385 390 395 400
   Gln Asp Lys Gly Val Asn Tyr Gln Pro Ser Arg Leu Tyr Pro Arg Glu
   405 410 415
   Glu Leu Ala Ser Ala Arg Tyr Ser His Thr Pro Leu Glu Gly Thr Thr
   420 425 430
   Leu Gln Ser Lys Ile Gln Arg Glu Gln Asn Phe Lys Gln Thr Gly Glu
   435 440 445
   Leu Tyr Arg Ser Tyr Ser Lys Lys Glu Gln Asp Asp Leu Ile Asn Ser
   450 455 460
   Leu Gly Thr Ser Leu Ala Asp Thr Asp Thr Glu Ser Lys Asn Ile Met
   465 470 475 480
   Leu Ser Tyr Phe Tyr Lys Ala Asp Lys Asp Tyr Gly Thr Arg Leu Thr
   485 490 495
   Gly Val Ala Lys Gly Asp Leu Ala Thr Val Gln Lys Leu Ala Asp Lys
   500 505 510
   Leu Ser Asp
   515
<210> 31
   <211> 1542
   <212> DNA
   <213> Erwinia tracheiphila
<400> 31
   catatggctg gtggcttcaa caatcgcaag aaactgctca tcgtgttaac gtctctggcc      60
   attagccaga tggcgatggc ggaaaccctg actcgtgaca atggagcgcc ggtaggcgac     120
   aaccagaata gtcagaccgc cggtgcgaat ggtccagtat tgctccagga tgtccagctc     180
   ttgcaaaagt tacagcgctt tgatcgagag cgtattccag aacgcgttgt ccatgcaaga     240
   ggcactggtg cacatgggga gtttacggct actgccgata tttccgatct gacaaccgcg     300
   aaagtgtttt ctgtcggctc taaaacgccg gtatttgttc gtttttcctc agtcgtgcat     360
   gggaaccatt cccccgaaac cctgcgtgat ccacgtggct ttgcgacccg cttttacacg     420
   actgaaggta actgggattt agtggggaac aactttccga ccttcttcat ccgggatgcg     480
   atcaagttcc ctgacatggt tcacgcattc aaacccgatc cacgcaccaa cttagacaac     540
   gactcacgcc gtttcgactt tttctcgcat ctaccggaaa gcacccgcac gctcaccctg     600
   ttgtactcta atgaaggcac acccgcctct taccgcaata tggatggtaa ttcggttcac     660
   gcctacaaat tcgtgaacag caaaggtgaa gtgcactatg tgaagtttca ttggaaaact     720
   cttcagggta tcaaaaatct ggacccgaag caagttgagc aagttcaagg caaagattac     780
   agccacatga cacacgatct ggtgacagcg attaaccgag gggattatcc caaatgggat      840
   ctgtatatcc aggtgctgaa accggaagat ctgaaaaagt ttgacttcga tccgttagat     900
   gctaccaaaa tttggccggg tgtaccggag cgcaaaattg gccaaatggt cctgaataaa     960
   aacccggaca atgtatttca ggagacggaa caagtggcta tggcaccgtc caaccttgtc    1020
   ccgggaattg aaccgtccga agacaaactg cttcaaggcc gcctgtttgc ctatgcggat    1080
   acccagttgt atcgtattgg agcgaatggc ctgagcctgc cgatcaatcg tccacacagt    1140
   ccggtgaaca atggcaatca ggacgggagc ctgaacagcg gtaatacgca ggataaaggc    1200
   gttaactatc agccttcgcg tctttggcct cgtgaagaga tggcttcagc ccggtatagc    1260
   catacccctt tggaaggtac gacacaacag tcgaaaattc agagggaaca aaacttcaaa    1320
   cagactggcg aactatatcg gagttatagc aaaaaagatc aggacgattt gatcaacagt    1380
   ctgggaacca gtctggtgga taccgatacc gagagcaaga acataatgct gtcgtacttt    1440
   tacaaagccg acaaagacta tggtacgcgc ttaaccaccg ttgcaaaagg cgatctggcg    1500
   acggtcaaaa agctggcaga caaactctca gattaagagc tc                       1542
<210> 32
   <211> 510
   <212> PRT
   <213> Erwinia tracheiphila
<400> 32
   Met Ala Gly Gly Phe Asn Asn Arg Lys Lys Leu Leu Ile Val Leu Thr
   1               5                   10                  15
   Ser Leu Ala Ile Ser Gln Met Ala Met Ala Glu Thr Leu Thr Arg Asp
               20                  25                  30
   Asn Gly Ala Pro Val Gly Asp Asn Gln Asn Ser Gln Thr Ala Gly Ala
           35                  40                  45
   Asn Gly Pro Val Leu Leu Gln Asp Val Gln Leu Leu Gln Lys Leu Gln
       50                  55                  60
   Arg Phe Asp Arg Glu Arg Ile Pro Glu Arg Val Val His Ala Arg Gly
   65                  70                  75                  80
   Thr Gly Ala His Gly Glu Phe Thr Ala Thr Ala Asp Ile Ser Asp Leu
                   85                  90                  95
   Thr Thr Ala Lys Val Phe Ser Val Gly Ser Lys Thr Pro Val Phe Val
               100                 105                 110
   Arg Phe Ser Ser Val Val His Gly Asn His Ser Pro Glu Thr Leu Arg
           115                 120                 125
   Asp Pro Arg Gly Phe Ala Thr Arg Phe Tyr Thr Thr Glu Gly Asn Trp
       130                 135                 140
   Asp Leu Val Gly Asn Asn Phe Pro Thr Phe Phe Ile Arg Asp Ala Ile
   145                 150                 155                 160
   Lys Phe Pro Asp Met Val His Ala Phe Lys Pro Asp Pro Arg Thr Asn
                   165                 170                 175
   Leu Asp Asn Asp Ser Arg Arg Phe Asp Phe Phe Ser His Leu Pro Glu
               180                 185                 190
   Ser Thr Arg Thr Leu Thr Leu Leu Tyr Ser Asn Glu Gly Thr Pro Ala
           195                 200                 205
   Ser Tyr Arg Asn Met Asp Gly Asn Ser Val His Ala Tyr Lys Phe Val
       210                 215                 220
   Asn Ser Lys Gly Glu Val His Tyr Val Lys Phe His Trp Lys Thr Leu
   225                 230                 235                 240
   Gln Gly Ile Lys Asn Leu Asp Pro Lys Gln Val Glu Gln Val Gln Gly
                   245                 250                 255
   Lys Asp Tyr Ser His Met Thr His Asp Leu Val Thr Ala Ile Asn Arg
               260                 265                 270
   Gly Asp Tyr Pro Lys Trp Asp Leu Tyr Ile Gln Val Leu Lys Pro Glu
           275                 280                 285
   Asp Leu Lys Lys Phe Asp Phe Asp Pro Leu Asp Ala Thr Lys Ile Trp
       290                 295                 300
   Pro Gly Val Pro Glu Arg Lys Ile Gly Gln Met Val Leu Asn Lys Asn
   305                 310                 315                 320
   Pro Asp Asn Val Phe Gln Glu Thr Glu Gln Val Ala Met Ala Pro Ser
                   325                 330                 335
   Asn Leu Val Pro Gly Ile Glu Pro Ser Glu Asp Lys Leu Leu Gln Gly
               340                 345                 350
   Arg Leu Phe Ala Tyr Ala Asp Thr Gln Leu Tyr Arg Ile Gly Ala Asn
           355                 360                 365
   Gly Leu Ser Leu Pro Ile Asn Arg Pro His Ser Pro Val Asn Asn Gly
       370                 375                 380
   Asn Gln Asp Gly Ser Leu Asn Ser Gly Asn Thr Gln Asp Lys Gly Val
   385                 390                 395                 400
   Asn Tyr Gln Pro Ser Arg Leu Trp Pro Arg Glu Glu Met Ala Ser Ala
                   405                 410                 415
   Arg Tyr Ser His Thr Pro Leu Glu Gly Thr Thr Gln Gln Ser Lys Ile
               420                 425                 430
   Gln Arg Glu Gln Asn Phe Lys Gln Thr Gly Glu Leu Tyr Arg Ser Tyr
           435                 440                 445
   Ser Lys Lys Asp Gln Asp Asp Leu Ile Asn Ser Leu Gly Thr Ser Leu
       450                 455                 460
   Val Asp Thr Asp Thr Glu Ser Lys Asn Ile Met Leu Ser Tyr Phe Tyr
   465                 470                 475                 480
   Lys Ala Asp Lys Asp Tyr Gly Thr Arg Leu Thr Thr Val Ala Lys Gly
                   485                 490                 495
   Asp Leu Ala Thr Val Lys Lys Leu Ala Asp Lys Leu Ser Asp
               500                 505                 510
<210> 33
   <211> 17
   <212> DNA
   <213> Artificial Sequence
<220>
   <223> primer
<400> 33
   caggaaacag ctatgac 17
<210> 34
   <211> 27
   <212> DNA
   <213> Artificial Sequence
<220>
   <223> primer
<400> 34
   ggcactacga accacctggg aatccat 27
<210> 35
   <211> 27
   <212> DNA
   <213> Artificial Sequence
<220>
   <223> primer
<400> 35
   gtggttcgta gtgccggtgg ggctgtg 27
<210> 36
   <211> 17
   <212> DNA
   <213> Artificial Sequence
<220>
   <223> primer
<400> 36
   gttttcccag tcacgac 17
<210> 37
   <211> 1419
   <212> DNA
   <213> Providencia rettgeri
<400> 37
   atgaaaatct cgcgtcgtaa gctgttatta ggggttggtg ctgctggtgt tttagcaggg     60
   ggtgctgcgg ttgttcctat gatcaatcgt gaaggtcgtt ttgaatcgac taaatcacgt    120
   gtaccagctg ttgctggcac agaaggcaaa ttaccagagt ctgcagatgc agtcatcatc    180
   ggtgccggcc ttcaagggat catgactgca attaaccttg ctgaaaaagg tcttaatgtt    240
   gttatctgtg aaaaaggtgt tgtcggtggt gagcaatcag gccgtgcata tagccaaatt    300
   atcagttata agacttcccc agctattttc cctttacacc attacggaaa aattcaatgg    360
   cttggcatga acgaaaaaat cggtgctgat accagctacc gtgttcaagg ccgtgttgaa    420
   gtaccttcaa gcgaagaaga tttagaaatt tcaagagcct ggattaaatc tgcatctgaa    480
   aacccaggtt tcgatacacc tttacgtacc cgtatgattg aaggaactga actggcgaat    540
   cgtctggttg atgcacaaac tccatggaaa atcggtggat ttgaagaaga ctcaggtagc    600
   cttgaccctg aagttgtcac accaaccatg gcaaactacg caaaatcaat cggtattcgc    660
   atctacacca attgcgcagt acgtggtatt gaaacggcgg gcggcaaaat ttctgatgtt    720
   gtcacagaaa aaggtgcaat caaaacttct cgtgttgttc tgacgggcgg tatttggtcg    780
   cgtctgttca tgggtaactt aggcattgat gttccaacac tgaacgttta cctatcacaa    840
   cagcgtatta ctggcgtacc aggcgcacca aaaggtaacg tccacttacc taacggtatt    900
   cacttccgtg aacaagctga tggtacctac gccgttgcgc cacgtatctt tactagctct    960
   atcgtaaaag acagcttcct gttaggacca agattcctac acgtattagg cggcggggaa   1020
   ttaccattag agttctctct tggtaaagat ttattcaact ccttcatgat ggcaacgtct   1080
   tggaacttag acgagaaaac accttttgaa gagttccgta ccgcaactaa tacaccaaac   1140
   aacgaacact tagatggcgt tctggaaaga ctgagaaaag aattcccagt atttaaagag   1200
   tctaaagtgg ttgaacgttg ggtggtaccc gttgcaccaa cggatgatga aattccaatt   1260
   atttcaacaa tcgagcagta tccaggacta gtcatcaaca ccgccacagg ctggggtatg   1320
   acggaaagcc ctgcatctgg tcgattaacg gcagaattgt taatgggcga aacaccattt   1380
   attgatccta cgccgtataa actttcccgt tttagctaa                           1419
<210> 38
   <211> 472
   <212> PRT
   <213> Providencia rettgeri
<400> 38
   Met Lys Ile Ser Arg Arg Lys Leu Leu Leu Gly Val Gly Ala Ala Gly
   1               5                   10                  15
   Val Leu Ala Gly Gly Ala Ala Val Val Pro Met Ile Asn Arg Glu Gly
               20                  25                  30
   Arg Phe Glu Ser Thr Lys Ser Arg Val Pro Ala Val Ala Gly Thr Glu
           35                  40                  45
   Gly Lys Leu Pro Glu Ser Ala Asp Ala Val Ile Ile Gly Ala Gly Leu
       50                  55                  60
   Gln Gly Ile Met Thr Ala Ile Asn Leu Ala Glu Lys Gly Leu Asn Val
   65                  70                  75                  80
   Val Ile Cys Glu Lys Gly Val Val Gly Gly Glu Gln Ser Gly Arg Ala
                   85                  90                  95
   Tyr Ser Gln Ile Ile Ser Tyr Lys Thr Ser Pro Ala Ile Phe Pro Leu
               100                 105                 110
   His His Tyr Gly Lys Ile Gln Trp Leu Gly Met Asn Glu Lys Ile Gly
           115                 120                 125
   Ala Asp Thr Ser Tyr Arg Val Gln Gly Arg Val Glu Val Pro Ser Ser
       130                 135                 140
   Glu Glu Asp Leu Glu Ile Ser Arg Ala Trp Ile Lys Ser Ala Ser Glu
   145                 150                 155                 160
   Asn Pro Gly Phe Asp Thr Pro Leu Arg Thr Arg Met Ile Glu Gly Thr
                   165                 170                 175
   Glu Leu Ala Asn Arg Leu Val Asp Ala Gln Thr Pro Trp Lys Ile Gly
               180                 185                 190
   Gly Phe Glu Glu Asp Ser Gly Ser Leu Asp Pro Glu Val Val Thr Pro
           195                 200                 205
   Thr Met Ala Asn Tyr Ala Lys Ser Ile Gly Ile Arg Ile Tyr Thr Asn
       210                 215                 220
   Cys Ala Val Arg Gly Ile Glu Thr Ala Gly Gly Lys Ile Ser Asp Val
   225                 230                 235                 240
   Val Thr Glu Lys Gly Ala Ile Lys Thr Ser Arg Val Val Leu Thr Gly
                   245                 250                 255
   Gly Ile Trp Ser Arg Leu Phe Met Gly Asn Leu Gly Ile Asp Val Pro
               260                 265                 270
   Thr Leu Asn Val Tyr Leu Ser Gln Gln Arg Ile Thr Gly Val Pro Gly
           275                 280                 285
   Ala Pro Lys Gly Asn Val His Leu Pro Asn Gly Ile His Phe Arg Glu
       290                 295                 300
   Gln Ala Asp Gly Thr Tyr Ala Val Ala Pro Arg Ile Phe Thr Ser Ser
   305                 310                 315                 320
   Ile Val Lys Asp Ser Phe Leu Leu Gly Pro Arg Phe Leu His Val Leu
                   325                 330                 335
   Gly Gly Gly Glu Leu Pro Leu Glu Phe Ser Leu Gly Lys Asp Leu Phe
               340                 345                 350
   Asn Ser Phe Met Met Ala Thr Ser Trp Asn Leu Asp Glu Lys Thr Pro
           355                 360                 365
   Phe Glu Glu Phe Arg Thr Ala Thr Asn Thr Pro Asn Asn Glu His Leu
       370                 375                 380
   Asp Gly Val Leu Glu Arg Leu Arg Lys Glu Phe Pro Val Phe Lys Glu
   385                 390                 395                 400
   Ser Lys Val Val Glu Arg Trp Gly Gly Thr Val Ala Pro Thr Asp Asp
                   405                 410                 415
   Glu Ile Pro Ile Ile Ser Thr Ile Glu Gln Tyr Pro Gly Leu Val Ile
               420                 425                 430
   Asn Thr Ala Thr Gly Trp Gly Met Thr Glu Ser Pro Ala Ser Gly Arg
           435                 440                 445
   Leu Thr Ala Glu Leu Leu Met Gly Glu Thr Pro Phe Ile Asp Pro Thr
       450                 455                 460
   Pro Tyr Lys Leu Ser Arg Phe Ser
   465                 470
<210> 39
   <211> 1062
   <212> DNA
   <213> Actinoplanes teichomyceticus
<400> 39
   atgaccatga cgggccactt tcaggacctg acagtggatc atgtccgcat ttactgcgca     60
   gacctggacc ccttgattgc acagtttggc tcctatggtc tggatgtccg tgccgaaggt    120
   gtaggcccgg gagcagagca tagcgtggtc ttgggccatg gggacattcg cctggttctg    180
   acacggccgg gtactggcga tcaccctggt ggcatgtata ccgcccaaca tggctacgga    240
   gtttcggaca ttgcattagg cacagcggat gctgcgggcg cttttcacga ggcggtacgt    300
   cgtggggcac gtccgattgc ggcgccagag cgtaccgccg gtgtggttac ggccagtgtt    360
   gcgggttttg gcgatgtgat ccataccttt gtacagcgtg aaccaggagg cccttggtcg    420
   ctcccgggtc tgaatccggt gcatcgcccg ggtactccgg ggattggact gcgcctggtg    480
   gatcactttg ccgtttgtgt cgaagcaggg cgcttaaccg aagtggtgga acactacgaa    540
   cgcgttttcg gcttttctgc catcttcacc gaacgcatcg tggttggaga acaagcgatg    600
   gattcccagg tggttcaaag tgccggtggg gctgtgacct taacggtcat tgcgccggat    660
   accacacgcc gtccgggtca gatcgatacc ttcctgaagg atcatggcgg tcccggtgtc    720
   cagcacattg cgtttgaaac ggatgatgtc acccgttcag ttggcgccat gtctgacgct    780
   ggtattgagt tccttacgac tccagcagcg tactatgagc ggcttcgcga tcgccttcaa    840
   ctcacgcgcc atagcgtgac cgaactgagc cgcctgaacg tattggctga cgaagatcac    900
   gacggccaac tctatcagat tttcacgaaa agcactcatc ctcgcgggac cctgttcttt    960
   gagatcatcg aacgtgtagg tgcccgcact ttcggttcag gcaacatcaa agcgctgtat   1020
   gaagcggtgg aactggacca ggctgccgcg gatggccgtt aa                      1062
<210> 40
   <211> 353
   <212> PRT
   <213> Actinoplanes teichomyceticus
<400> 40
   Met Thr Met Thr Gly His Phe Gln Asp Leu Thr Val Asp His Val Arg
   1               5                   10                  15
   Ile Tyr Cys Ala Asp Leu Asp Pro Leu Ile Ala Gln Phe Gly Ser Tyr
               20                  25                  30
   Gly Leu Asp Val Arg Ala Glu Gly Val Gly Pro Gly Ala Glu His Ser
           35                  40                  45
   Val Val Leu Gly His Gly Asp Ile Arg Leu Val Leu Thr Arg Pro Gly
       50                  55                  60
   Thr Gly Asp His Pro Gly Gly Met Tyr Thr Ala Gln His Gly Tyr Gly
   65                  70                  75                  80
   Val Ser Asp Ile Ala Leu Gly Thr Ala Asp Ala Ala Gly Ala Phe His
                   85                  90                  95
   Glu Ala Val Arg Arg Gly Ala Arg Pro Ile Ala Ala Pro Glu Arg Thr
               100                 105                 110
   Ala Gly Val Val Thr Ala Ser Val Ala Gly Phe Gly Asp Val Ile His
           115                 120                 125
   Thr Phe Val Gln Arg Glu Pro Gly Gly Pro Trp Ser Leu Pro Gly Leu
       130                 135                 140
   Asn Pro Val His Arg Pro Gly Thr Pro Gly Ile Gly Leu Arg Leu Val
   145                 150                 155                 160
   Asp His Phe Ala Val Cys Val Glu Ala Gly Arg Leu Thr Glu Val Val
                   165                 170                 175
   Glu His Tyr Glu Arg Val Phe Gly Phe Ser Ala Ile Phe Thr Glu Arg
               180                 185                 190
   Ile Val Val Gly Glu Gln Ala Met Asp Ser Gln Val Val Gln Ser Ala
           195                 200                 205
   Gly Gly Ala Val Thr Leu Thr Val Ile Ala Pro Asp Thr Thr Arg Arg
       210                 215                 220
   Pro Gly Gln Ile Asp Thr Phe Leu Lys Asp His Gly Gly Pro Gly Val
   225                 230                 235                 240
   Gln His Ile Ala Phe Glu Thr Asp Asp Val Thr Arg Ser Val Gly Ala
                   245                 250                 255
   Met Ser Asp Ala Gly Ile Glu Phe Leu Thr Thr Pro Ala Ala Tyr Tyr
               260                 265                 270
   Glu Arg Leu Arg Asp Arg Leu Gln Leu Thr Arg His Ser Val Thr Glu
           275                 280                 285
   Leu Ser Arg Leu Asn Val Leu Ala Asp Glu Asp His Asp Gly Gln Leu
       290                 295                 300
   Tyr Gln Ile Phe Thr Lys Ser Thr His Pro Arg Gly Thr Leu Phe Phe
   305                 310                 315                 320
   Glu Ile Ile Glu Arg Val Gly Ala Arg Thr Phe Gly Ser Gly Asn Ile
                   325                 330                 335
   Lys Ala Leu Tyr Glu Ala Val Glu Leu Asp Gln Ala Ala Ala Asp Gly
               340                 345                 350
   Arg
<210> 41
   <211> 1182
   <212> DNA
   <213> Pseudomonas putida
<400> 41
   atgagccgca acctgtttaa cgttgaagat tatcgtaaac tggcacagaa acgtctgccg     60
   aaaatggttt atgattatct ggaaggtggt gccgaagatg aatatggtgt taaacataac    120
   cgtgatgtgt ttcagcagtg gcgttttaaa ccgaaacgcc tggttgatgt tagccgtcgt    180
   agtctgcagg cagaagttct gggtaaacgt cagagcatgc cgctgctgat tggtccgacc    240
   ggtctgaatg gtgcactgtg gccgaaaggt gatctggcac tggcccaggc agcaaccaaa    300
   gcaggtattc cgtttgttct gagcaccgca agcaatatga gcattgagga cctggcacgt    360
   cagtgtgatg gtgatctgtg gtttcagctg tatgttattc atcgtgaaat tgcccagggt    420
   atggttctga aagcactgca tagcggttat accaccctgg ttctgaccac cgatgttgca    480
   gttaatggtt atcgtgaacg tgatctgcat aaccgcttta aaatgccgat gagctatacc    540
   ccgaaagtta tgctggatgg ttgtctgcat ccgcgttgga gcctggatct ggttcgtcat    600
   ggtatgccgc agctggcaaa ttttgttagc agccagacca gcagcctgga aatgcaggca    660
   gcactgatga gccgtcagat ggatgcaagc tttaattggg aagcactgcg ttggctgcgc    720
   gatctgtggc ctcataaact gctggttaaa ggtctgctga gcgcagaaga tgcagatcgt    780
   tgtattgccg aaggtgccga tggtgttatt ctgagcaatc atggtggtcg tcagctggat    840
   tgtgcagtta gcccgatgga agtgctggcc cagagcgttg caaaaaccgg taaaccggtt    900
   ctgattgata gcggttttcg tcgtggtagc gatattgtta aagcactggc gctgggtgca    960
   gaagcagttc tgctgggtcg tgcaaccctg tatggtctgg cagcacgtgg tgaaaccggt   1020
   gttggtgaag ttctgaccct gctgaaagca gatattgatc gtaccctggc gcagattggt   1080
   tgtccggata ttaccagcct gagtccggat tatctgcaga gcgaaggtgt taccaatacc   1140
   gcaccggttg atcatctgat tggtaaaggc acccatgcat ga                       1182
<210> 42
   <211> 393
   <212> PRT
   <213> Pseudomonas putida
<400> 42
   Met Ser Arg Asn Leu Phe Asn Val Glu Asp Tyr Arg Lys Leu Ala Gln
   1               5                   10                  15
   Lys Arg Leu Pro Lys Met Val Tyr Asp Tyr Leu Glu Gly Gly Ala Glu
               20                  25                  30
   Asp Glu Tyr Gly Val Lys His Asn Arg Asp Val Phe Gln Gln Trp Arg
           35                  40                  45
   Phe Lys Pro Lys Arg Leu Val Asp Val Ser Arg Arg Ser Leu Gln Ala
       50                  55                  60
   Glu Val Leu Gly Lys Arg Gln Ser Met Pro Leu Leu Ile Gly Pro Thr
   65                  70                  75                  80
   Gly Leu Asn Gly Ala Leu Trp Pro Lys Gly Asp Leu Ala Leu Ala Gln
                   85                  90                  95
   Ala Ala Thr Lys Ala Gly Ile Pro Phe Val Leu Ser Thr Ala Ser Asn
                   100                 105                 110
   Met Ser Ile Glu Asp Leu Ala Arg Gln Cys Asp Gly Asp Leu Trp Phe
               115                 120                 125
   Gln Leu Tyr Val Ile His Arg Glu Ile Ala Gln Gly Met Val Leu Lys
       130                 135                 140
   Ala Leu His Ser Gly Tyr Thr Thr Leu Val Leu Thr Thr Asp Val Ala
   145                 150                 155                 160
   Val Asn Gly Tyr Arg Glu Arg Asp Leu His Asn Arg Phe Lys Met Pro
                   165                 170                 175
   Met Ser Tyr Thr Pro Lys Val Met Leu Asp Gly Cys Leu His Pro Arg
               180                 185                 190
   Trp Ser Leu Asp Leu Val Arg His Gly Met Pro Gln Leu Ala Asn Phe
           195                 200                 205
   Val Ser Ser Gln Thr Ser Ser Leu Glu Met Gln Ala Ala Leu Met Ser
       210                 215                 220
   Arg Gln Met Asp Ala Ser Phe Asn Trp Glu Ala Leu Arg Trp Leu Arg
   225                 230                 235                 240
   Asp Leu Trp Pro His Lys Leu Leu Val Lys Gly Leu Leu Ser Ala Glu
                   245                 250                 255
   Asp Ala Asp Arg Cys Ile Ala Glu Gly Ala Asp Gly Val Ile Leu Ser
               260                 265                 270
   Asn His Gly Gly Arg Gln Leu Asp Cys Ala Val Ser Pro Met Glu Val
           275                 280                 285
   Leu Ala Gln Ser Val Ala Lys Thr Gly Lys Pro Val Leu Ile Asp Ser
       290                 295                 300
   Gly Phe Arg Arg Gly Ser Asp Ile Val Lys Ala Leu Ala Leu Gly Ala
   305                 310                 315                 320
   Glu Ala Val Leu Leu Gly Arg Ala Thr Leu Tyr Gly Leu Ala Ala Arg
                   325                 330                 335
   Gly Glu Thr Gly Val Gly Glu Val Leu Thr Leu Leu Lys Ala Asp Ile
               340                 345                 350
   Asp Arg Thr Leu Ala Gln Ile Gly Cys Pro Asp Ile Thr Ser Leu Ser
           355                 360                 365
   Pro Asp Tyr Leu Gln Ser Glu Gly Val Thr Asn Thr Ala Pro Val Asp
       370                 375                 380
   His Leu Ile Gly Lys Gly Thr His Ala
   385                 390
<210> 43
   <211> 1587
   <212> DNA
   <213> Pseudomonas putida
<400> 43
   atggcaagcg ttcatggcac cacctatgaa ctgctgcgtc gtcagggtat tgataccgtt     60
   tttggtaatc cgggtagcaa tgaactgccg tttctgaaag attttccgga agattttcgt    120
   tatattctgg cactgcaaga agcatgcgtt gttggtattg cagatggtta tgcacaggca    180
   agccgtaaac cggcatttat caatctgcat agcgcagcag gcaccggtaa tgcaatgggt    240
   gcactgagca atgcatggaa tagccatagt ccgctgattg ttaccgcagg tcagcagacc    300
   cgtgcaatga ttggtgttga agcactgctg accaatgttg atgcagcaaa tctgcctcgt    360
   ccgctggtta aatggtcata tgaaccggca agcgcagccg aagttccgca tgcaatgagc    420
   cgtgcaattc atatggcaag catggcaccg cagggtccgg tttatctgag cgttccgtat    480
   gatgattggg ataaagatgc agatccgcag agccatcacc tgtttgatcg tcatgttagc    540
   agcgcagttc gtctgaatga tcaggatctg gaaattctgg ttaaagcact gaatagcgca    600
   agcaatccgg caattgttct gggtccggat gtggatgcag ccaatgcaaa tgccgattgt    660
   gttcagctgg cagaacgtct gaaagcaccg gtttgggttg caccgagcgc accgcgttgt    720
   ccgtttccga cccgtcatcc gtgttttcgt ggtctgatgc ctgcaggtat tgccgcaatt    780
   agccagctgc tggaaggtca tgatctggtt ctggttattg gtgcaccggt gtttcgttat    840
   catcagtatg atccgggtca gtatctgaaa ccgggtacac gtctgattag cgttacctgt    900
   gatccgctgg aagcagcacg tgcaccgatg ggtgatgcaa ttgttgcaga tatcggtgca    960
   atggccagcg cactggcaaa tagcgttgaa gaatgtagcc gtccgctgcc gaccgcagca   1020
   ccggaacctg caaaagtgga tcaggatgca ggtcgtctgc atccggaaac cgtgtttgat   1080
   accctgaatg atatggcacc ggaaaatgcc atttatctga atgaaagtac cagcaccacc   1140
   gcacagatgt ggcagcgtct gaatatgcgt aatcctggta gttattactt ttgtgcagcc   1200
   ggtggtctgg gttttgcact gcctgcagca attggtgtgc agctggccga gccggaacgt   1260
   caggttattg ccgtgattgg tgatggtagc gcaaattata gcattagcgc actgtggacc   1320
   gcagcccagt ataacattcc gaccattttt gtgattatga acaatggcac ctatggtgca   1380
   ctgcgttggt ttgccggtgt tctggaagcc gaaaacgttc cgggtctgga tgttccgggt   1440
   atcgattttt gtgcactggc caaaggttat ggtgttcagg cactgaaagc aaataatctg   1500
   gaacagctga aggtagcctt gcaagaggca ctgagcgcaa aaggtccggt tctgattgaa   1560
   gttagcaccg ttagtccggt taaatga                                        1587
<210> 44
   <211> 528
   <212> PRT
   <213> Pseudomonas putida
<400> 44
   Met Ala Ser Val His Gly Thr Thr Tyr Glu Leu Leu Arg Arg Gln Gly
   1               5                   10                  15
   Ile Asp Thr Val Phe Gly Asn Pro Gly Ser Asn Glu Leu Pro Phe Leu
               20                  25                  30
   Lys Asp Phe Pro Glu Asp Phe Arg Tyr Ile Leu Ala Leu Gln Glu Ala
           35                  40                  45
   Cys Val Val Gly Ile Ala Asp Gly Tyr Ala Gln Ala Ser Arg Lys Pro
       50                  55                  60
   Ala Phe Ile Asn Leu His Ser Ala Ala Gly Thr Gly Asn Ala Met Gly
   65                  70                  75                  80
   Ala Leu Ser Asn Ala Trp Asn Ser His Ser Pro Leu Ile Val Thr Ala
                   85                  90                  95
   Gly Gln Gln Thr Arg Ala Met Ile Gly Val Glu Ala Leu Leu Thr Asn
               100                 105                 110
   Val Asp Ala Ala Asn Leu Pro Arg Pro Leu Val Lys Trp Ser Tyr Glu
           115                 120                 125
   Pro Ala Ser Ala Ala Glu Val Pro His Ala Met Ser Arg Ala Ile His
       130                 135                 140
   Met Ala Ser Met Ala Pro Gln Gly Pro Val Tyr Leu Ser Val Pro Tyr
   145                 150                 155                 160
   Asp Asp Trp Asp Lys Asp Ala Asp Pro Gln Ser His His Leu Phe Asp
                   165                 170                 175
   Arg His Val Ser Ser Ala Val Arg Leu Asn Asp Gln Asp Leu Glu Ile
               180                 185                 190
   Leu Val Lys Ala Leu Asn Ser Ala Ser Asn Pro Ala Ile Val Leu Gly
           195                 200                 205
   Pro Asp Val Asp Ala Ala Asn Ala Asn Ala Asp Cys Val Gln Leu Ala
       210                 215                 220
   Glu Arg Leu Lys Ala Pro Val Trp Val Ala Pro Ser Ala Pro Arg Cys
   225                 230                 235                 240
   Pro Phe Pro Thr Arg His Pro Cys Phe Arg Gly Leu Met Pro Ala Gly
                   245                 250                 255
   Ile Ala Ala Ile Ser Gln Leu Leu Glu Gly His Asp Leu Val Leu Val
               260                 265                 270
   Ile Gly Ala Pro Val Phe Arg Tyr His Gln Tyr Asp Pro Gly Gln Tyr
           275                 280                 285
   Leu Lys Pro Gly Thr Arg Leu Ile Ser Val Thr Cys Asp Pro Leu Glu
       290                 295                 300
   Ala Ala Arg Ala Pro Met Gly Asp Ala Ile Val Ala Asp Ile Gly Ala
   305                 310                 315                 320
   Met Ala Ser Ala Leu Ala Asn Ser Val Glu Glu Cys Ser Arg Pro Leu
                   325                 330                 335
   Pro Thr Ala Ala Pro Glu Pro Ala Lys Val Asp Gln Asp Ala Gly Arg
               340                 345                 350
   Leu His Pro Glu Thr Val Phe Asp Thr Leu Asn Asp Met Ala Pro Glu
           355                 360                 365
   Asn Ala Ile Tyr Leu Asn Glu Ser Thr Ser Thr Thr Ala Gln Met Trp
       370                 375                 380
   Gln Arg Leu Asn Met Arg Asn Pro Gly Ser Tyr Tyr Phe Cys Ala Ala
   385                 390                 395                 400
   Gly Gly Leu Gly Phe Ala Leu Pro Ala Ala Ile Gly Val Gln Leu Ala
                   405                 410                 415
   Glu Pro Glu Arg Gln Val Ile Ala Val Ile Gly Asp Gly Ser Ala Asn
               420                 425                 430
   Tyr Ser Ile Ser Ala Leu Trp Thr Ala Ala Gln Tyr Asn Ile Pro Thr
           435                 440                 445
   Ile Phe Val Ile Met Asn Asn Gly Thr Tyr Gly Ala Leu Arg Trp Phe
       450                 455                 460
   Ala Gly Val Leu Glu Ala Glu Asn Val Pro Gly Leu Asp Val Pro Gly
   465                 470                 475                 480
   Ile Asp Phe Cys Ala Leu Ala Lys Gly Tyr Gly Val Gln Ala Leu Lys
                   485                 490                 495
   Ala Asn Asn Leu Glu Gln Leu Lys Gly Ser Leu Gln Glu Ala Leu Ser
               500                 505                 510
   Ala Lys Gly Pro Val Leu Ile Glu Val Ser Thr Val Ser Pro Val Lys
           515                 520                 525